# EUROPEAN PATENT APPLICATION

(11) **EP 3 799 882 A1**
(43) Date of publication of application: **07.04.2021**
(21) Application number: 20201771.1
(22) Date of filing: 01.11.2017
(51) Int. Cl.: A61K 39/00, A61P 3/10

(54) **TOLEROGENIC DNA VACCINE**

(30) Priority: 01.11.2016 US 201662415717 P; 02.01.2017 EP 17150037; 02.05.2017 EP 17169019; 22.06.2017 EP 17177289; 24.10.2017 EP 17198041
(62) Divisional of application: 17801363.7
(71) Applicant: Novo Nordisk A/S, 2880 Bagsværd (DK)
(72) Inventor: CHAPLIN, Jay, Seattle, Washington 98109 (US); WIJARANAKULA, Michael, Seattle, Washington 98109 (US)

(57) **Abstract**

The present invention relates to plasmids useful for prevention and/or delay of e.g. type 1 diabetes.

SEQUENCE LISTING

<110>  Novo Nordisk A/S

<120>  Tolerogenic DNA vaccine

<130>  150042EP05

<150>  US 62/415717
<151>  2016-11-01

<150>  EP 17150037.4
<151>  2017-01-02

<150>  EP 17169019.1
<151>  2017-05-02

<150>  EP 17177289.0
<151>  2017-06-22

<150>  EP 17198041.0
<151>  2017-10-24

<160>  29

<170>  PatentIn version 3.5

<210>  1
<211>  86
<212>  PRT
<213>  mus musculus

<400>  1

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210>  2
<211>  86
<212>  PRT
<213>  homo sapiens

<400>  2

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

Glu Ala Glu Asp Leu Gln Val Gly Gln Val Glu Leu Gly Gly Gly Pro
        35                  40                  45

Gly Ala Gly Ser Leu Gln Pro Leu Ala Leu Glu Gly Ser Leu Gln Lys
    50                  55                  60

Arg Gly Ile Val Glu Gln Cys Cys Thr Ser Ile Cys Ser Leu Tyr Gln
65                  70                  75                  80

Leu Glu Asn Tyr Cys Asn
                85

<210>  3
<211>  110
<212>  PRT
<213>  synthetic

<400>  3

Met Ala Leu Trp Met Arg Leu Leu Pro Leu Leu Ala Leu Leu Ala Leu
1               5                   10                  15

Trp Gly Pro Asp Pro Glu Gln Glu Phe Val Asn Gln His Leu Cys Gly
            20                  25                  30

Ser His Leu Val Glu Ala Leu Tyr Leu Val Cys Gly Glu Arg Gly Phe
        35                  40                  45

Phe Tyr Thr Pro Lys Thr Arg Arg Glu Ala Glu Asp Leu Gln Val Gly
    50                  55                  60

Gln Val Glu Leu Gly Gly Gly Pro Gly Ala Gly Ser Leu Gln Pro Leu
65                  70                  75                  80

Ala Leu Glu Gly Ser Leu Gln Lys Arg Gly Ile Val Glu Gln Cys Cys
                85                  90                  95

Thr Ser Ile Cys Ser Leu Tyr Gln Leu Glu Asn Tyr Cys Asn
            100                 105                 110

<210>  4
<211>  110

<212>   PRT
<213>   synthetic

<400>   4

Met Ala Leu Trp Met Arg Leu Leu Pro Leu Leu Ala Leu Leu Ala Leu
1               5                   10                  15

Trp Gly Pro Asp Pro Glu Gln Glu Phe Val Asn Gln His Leu Cys Gly
            20                  25                  30

Ser His Leu Val Glu Ala Leu Tyr Leu Val Cys Gly Glu Arg Gly Phe
        35                  40                  45

Phe Tyr Thr Pro Lys Thr Arg Arg Glu Ala Glu Asp Leu Gln Val Gly
    50                  55                  60

Gln Val Glu Leu Gly Gly Gly Pro Gly Ala Gly Ser Leu Gln Pro Leu
65                  70                  75                  80

Ala Leu Glu Gly Ser Leu Gln Lys Arg Gly Ile Val Glu Gln Cys Cys
                85                  90                  95

Thr Ser Ile Cys Ser Leu Tyr Gln Leu Glu Asn Tyr Cys Asn
            100                 105                 110


<210>   5
<211>   109
<212>   PRT
<213>   mus musculus

<400>   5

Ala Leu Trp Met Arg Leu Leu Pro Leu Leu Ala Leu Leu Ala Leu Trp
1               5                   10                  15

Gly Pro Asp Pro Ala Gln Ala Phe Val Asn Gln His Leu Cys Gly Ser
            20                  25                  30

His Leu Val Glu Ala Leu Tyr Leu Val Cys Gly Glu Arg Gly Phe Phe
        35                  40                  45

Tyr Thr Pro Lys Thr Arg Arg Glu Ala Glu Asp Leu Gln Val Gly Gln
    50                  55                  60

Val Glu Leu Gly Gly Gly Pro Gly Ala Gly Ser Leu Gln Pro Leu Ala
65                  70                  75                  80

Leu Glu Gly Ser Leu Gln Lys Arg Gly Ile Val Glu Gln Cys Cys Thr
                85                  90                  95

Ser Ile Cys Ser Leu Tyr Gln Leu Glu Asn Tyr Cys Asn
            100                 105

<210> 6
<211> 110
<212> PRT
<213> homo sapiens

<400> 6

Met Ala Leu Trp Met Arg Leu Leu Pro Leu Leu Ala Leu Leu Ala Leu
1               5                   10                  15

Trp Gly Pro Asp Pro Ala Gln Ala Phe Val Asn Gln His Leu Cys Gly
            20                  25                  30

Ser His Leu Val Glu Ala Leu Tyr Leu Val Cys Gly Glu Arg Gly Phe
        35                  40                  45

Phe Tyr Thr Pro Lys Thr Arg Arg Glu Ala Glu Asp Leu Gln Val Gly
    50                  55                  60

Gln Val Glu Leu Gly Gly Gly Pro Gly Ala Gly Ser Leu Gln Pro Leu
65                  70                  75                  80

Ala Leu Glu Gly Ser Leu Gln Lys Arg Gly Ile Val Glu Gln Cys Cys
                85                  90                  95

Thr Ser Ile Cys Ser Leu Tyr Gln Leu Glu Asn Tyr Cys Asn
            100                 105                 110

<210> 7
<211> 15
<212> PRT
<213> homo sapiens

<400> 7

Ser His Leu Val Glu Ala Leu Tyr Leu Val Cys Gly Glu Arg Gly
1               5                   10                  15

<210> 8
<211> 15
<212> PRT
<213> synthetic

<400> 8

Ser His Leu Val Glu Ala Leu Tyr Leu Val Cys Gly Glu Glu Gly
1               5                   10                  15

<210> 9

<211>  81
<212>  PRT
<213>  mus musculus

<400>  9

Met Asp Asp Gln Arg Asp Leu Ile Ser Asn His Glu Gln Leu Pro Ile
1               5                   10                  15

Leu Gly Asn Arg Pro Arg Glu Pro Glu Arg Cys Ser Arg Gly Ala Leu
            20                  25                  30

Tyr Thr Gly Val Ser Val Leu Val Ala Leu Leu Leu Ala Gly Gln Ala
        35                  40                  45

Thr Thr Ala Tyr Phe Leu Tyr Gln Gln Gln Gly Arg Leu Asp Lys Leu
    50                  55                  60

Thr Ile Thr Ser Gln Asn Leu Gln Leu Glu Ser Leu Arg Met Lys Leu
65                  70                  75                  80

Pro

<210>  10
<211>  98
<212>  PRT
<213>  homo sapiens

<400>  10

Met His Arg Arg Arg Ser Arg Ser Cys Arg Glu Asp Gln Lys Pro Val
1               5                   10                  15

Met Asp Asp Gln Arg Asp Leu Ile Ser Asn Asn Glu Gln Leu Pro Met
            20                  25                  30

Leu Gly Arg Arg Pro Gly Ala Pro Glu Ser Lys Cys Ser Arg Gly Ala
        35                  40                  45

Leu Tyr Thr Gly Phe Ser Ile Leu Val Thr Leu Leu Leu Ala Gly Gln
    50                  55                  60

Ala Thr Thr Ala Tyr Phe Leu Tyr Gln Gln Gln Gly Arg Leu Asp Lys
65                  70                  75                  80

Leu Thr Ile Thr Ser Gln Asn Leu Gln Leu Glu Ser Leu Arg Met Lys
                85                  90                  95

Leu Pro

<210>  11
<211>  219
<212>  DNA
<213>  E. coli

<400>  11
atggccaaag aagacaatat tgaaatgcaa ggtaccgttc ttgaaacgtt gcctaatacc      60

atgttccgcg tagagttaga aaacggtcac gtggttactg cacacatctc cggtaaaatg      120

cgcaaaaact acatccgcat cctgacgggc gacaaagtga ctgttgaact gaccccgtac      180

gacctgagca aaggccgcat tgtcttccgt agtcgctga                             219


<210>  12
<211>  72
<212>  PRT
<213>  E. coli

<400>  12


<210>  13
<211>  127
<212>  DNA
<213>  L. monocytogenes

<400>  13
tgtaaaaaac atcatttagc gtgactttct ttcaacagct aacaattgtt gttactgcct      60

aatgttttta gggtatttta aaaaagggcg ataaaaaacg attgggggat gagaaatgaa      120

cgctcaa                                                                127


<210>  14
<211>  10
<212>  DNA
<213>  synthetic

<400>  14

gccrccatgg                                                              10

<210>  15
<211>  24
<212>  PRT
<213>  synthetic

<400>  15

Ala Pro Val Lys Gln Thr Leu Asn Phe Asp Leu Leu Lys Leu Ala Gly
1               5                   10                  15

Asp Val Glu Ser Asn Pro Gly Pro
            20

<210>  16
<211>  20
<212>  PRT
<213>  Thosea asigna

<400>  16

Arg Ala Glu Gly Arg Gly Ser Leu Leu Thr Cys Gly Asp Val Glu Glu
1               5                   10                  15

Asn Pro Gly Pro
            20

<210>  17
<211>  19
<212>  PRT
<213>  synthetic

<400>  17

Ala Thr Asn Phe Ser Leu Leu Lys Gln Ala Gly Asp Val Glu Glu Asn
1               5                   10                  15

Pro Gly Pro

<210>  18
<211>  390
<212>  PRT
<213>  homo sapiens

<400>  18

Met Pro Pro Ser Gly Leu Arg Leu Leu Leu Leu Leu Leu Pro Leu Leu
1               5                   10                  15

Trp Leu Leu Val Leu Thr Pro Gly Arg Pro Ala Ala Gly Leu Ser Thr
            20                  25                  30

Cys Lys Thr Ile Asp Met Glu Leu Val Lys Arg Lys Arg Ile Glu Ala
        35                  40                  45

Ile Arg Gly Gln Ile Leu Ser Lys Leu Arg Leu Ala Ser Pro Pro Ser
    50                  55                  60

Gln Gly Glu Val Pro Pro Gly Pro Leu Pro Glu Ala Val Leu Ala Leu
65                  70                  75                  80

Tyr Asn Ser Thr Arg Asp Arg Val Ala Gly Glu Ser Ala Glu Pro Glu
                85                  90                  95

Pro Glu Pro Glu Ala Asp Tyr Tyr Ala Lys Glu Val Thr Arg Val Leu
            100                 105                 110

Met Val Glu Thr His Asn Glu Ile Tyr Asp Lys Phe Lys Gln Ser Thr
        115                 120                 125

His Ser Ile Tyr Met Phe Phe Asn Thr Ser Glu Leu Arg Glu Ala Val
    130                 135                 140

Pro Glu Pro Val Leu Leu Ser Arg Ala Glu Leu Arg Leu Leu Arg Leu
145                 150                 155                 160

Lys Leu Lys Val Glu Gln His Val Glu Leu Tyr Gln Lys Tyr Ser Asn
                165                 170                 175

Asn Ser Trp Arg Tyr Leu Ser Asn Arg Leu Leu Ala Pro Ser Asp Ser
            180                 185                 190

Pro Glu Trp Leu Ser Phe Asp Val Thr Gly Val Val Arg Gln Trp Leu
        195                 200                 205

Ser Arg Gly Gly Glu Ile Glu Gly Phe Arg Leu Ser Ala His Cys Ser
    210                 215                 220

Cys Asp Ser Arg Asp Asn Thr Leu Gln Val Asp Ile Asn Gly Phe Thr
225                 230                 235                 240

Thr Gly Arg Arg Gly Asp Leu Ala Thr Ile His Gly Met Asn Arg Pro
                245                 250                 255

Phe Leu Leu Leu Met Ala Thr Pro Leu Glu Arg Ala Gln His Leu Gln
            260                 265                 270

Ser Ser Arg His Arg Arg Ala Leu Asp Thr Asn Tyr Cys Phe Ser Ser
        275                 280                 285

Thr Glu Lys Asn Cys Cys Val Arg Gln Leu Tyr Ile Asp Phe Arg Lys
    290                 295                 300

Asp Leu Gly Trp Lys Trp Ile His Glu Pro Lys Gly Tyr His Ala Asn
305                 310                 315                 320

Phe Cys Leu Gly Pro Cys Pro Tyr Ile Trp Ser Leu Asp Thr Gln Tyr
                325                 330                 335

Ser Lys Val Leu Ala Leu Tyr Asn Gln His Asn Pro Gly Ala Ser Ala
            340                 345                 350

Ala Pro Cys Cys Val Pro Gln Ala Leu Glu Pro Leu Pro Ile Val Tyr
        355                 360                 365

Tyr Val Gly Arg Lys Pro Lys Val Glu Gln Leu Ser Asn Met Ile Val
    370                 375                 380

Arg Ser Cys Lys Cys Ser
385                 390

<210>  19
<211>  390
<212>  PRT
<213>  synthetic

<400>  19

Met Pro Pro Ser Gly Leu Arg Leu Leu Leu Leu Leu Leu Pro Leu Leu
1               5                   10                  15

Trp Leu Leu Val Leu Thr Pro Gly Arg Pro Ala Ala Gly Leu Ser Thr
            20                  25                  30

Cys Lys Thr Ile Asp Met Glu Leu Val Lys Arg Lys Arg Ile Glu Ala
        35                  40                  45

Ile Arg Gly Gln Ile Leu Ser Lys Leu Arg Leu Ala Ser Pro Pro Ser
    50                  55                  60

Gln Gly Glu Val Pro Pro Gly Pro Leu Pro Glu Ala Val Leu Ala Leu
65                  70                  75                  80

Tyr Asn Ser Thr Arg Asp Arg Val Ala Gly Glu Ser Ala Glu Pro Glu
                85                  90                  95

Pro Glu Pro Glu Ala Asp Tyr Tyr Ala Lys Glu Val Thr Arg Val Leu
            100                 105                 110

Met Val Glu Thr His Asn Glu Ile Tyr Asp Lys Phe Lys Gln Ser Thr
        115                 120                 125

His Ser Ile Tyr Met Phe Phe Asn Thr Ser Glu Leu Arg Glu Ala Val
    130                 135                 140

Pro Glu Pro Val Leu Leu Ser Arg Ala Glu Leu Arg Leu Leu Arg Leu
145                 150                 155                 160

Lys Leu Lys Val Glu Gln His Val Glu Leu Tyr Gln Lys Tyr Ser Asn
                165                 170                 175

Asn Ser Trp Arg Tyr Leu Ser Asn Arg Leu Leu Ala Pro Ser Asp Ser
            180                 185                 190

Pro Glu Trp Leu Ser Phe Asp Val Thr Gly Val Val Arg Gln Trp Leu
        195                 200                 205

Ser Arg Gly Gly Glu Ile Glu Gly Phe Arg Leu Ser Ala His Val Ser
    210                 215                 220

Val Asp Ser Arg Asp Asn Thr Leu Gln Val Asp Ile Asn Gly Phe Thr
225                 230                 235                 240

Thr Gly Arg Arg Gly Asp Leu Ala Thr Ile His Gly Met Asn Arg Pro
                245                 250                 255

Phe Leu Leu Leu Met Ala Thr Pro Leu Glu Arg Ala Gln His Leu Gln
            260                 265                 270

Ser Ser Arg His Arg Arg Ala Leu Asp Thr Asn Tyr Cys Phe Ser Ser
        275                 280                 285

Thr Glu Lys Asn Cys Cys Val Arg Gln Leu Tyr Ile Asp Phe Arg Lys
    290                 295                 300

Asp Leu Gly Trp Lys Trp Ile His Glu Pro Lys Gly Tyr His Ala Asn
305                 310                 315                 320

Phe Cys Leu Gly Pro Cys Pro Tyr Ile Trp Ser Leu Asp Thr Gln Tyr
                325                 330                 335

Ser Lys Val Leu Ala Leu Tyr Asn Gln His Asn Pro Gly Ala Ser Ala
            340                 345                 350

Ala Pro Cys Cys Val Pro Gln Ala Leu Glu Pro Leu Pro Ile Val Tyr
        355                 360                 365

Tyr Val Gly Arg Lys Pro Lys Val Glu Gln Leu Ser Asn Met Ile Val
    370                 375                 380

Arg Ser Cys Lys Cys Ser
385                 390

<210>  20
<211>  228
<212>  DNA
<213>  synthetic

<400>  20
cgactgtgcc ttctagttgc cagccatctg ttgtttgccc ctcccccgtg ccttccttga      60

ccctggaagg tgccactccc actgtccttt cctaataaaa tgaggaaatt gcatcgcatt     120

gtctgagtag gtgtcattct attctggggg gtggggtggg gcaggacagc aagggggagg     180

attgggaaga caatagcagg catgctgggg atgcggtggg ctctatgg                  228

<210>  21
<211>  31
<212>  PRT
<213>  homo sapiens

<400>  21

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  22
<211>  31
<212>  PRT
<213>  synthetic

<400>  22

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Arg Gly
            20                  25                  30

<210>  23
<211>  31
<212>  PRT
<213>  synthetic

<220>
<221>  MISC_FEATURE

<222>   (2)..(2)
<223>   2-aminoisobutyric acid

<400>   23

His Xaa Glu Gly Thr Phe Thr Ser Asp Val Ser Ser Tyr Leu Glu Gly
1               5                   10                  15

Gln Ala Ala Lys Glu Phe Ile Ala Trp Leu Val Arg Gly Arg Gly
            20                  25                  30

<210>   24
<211>   6401
<212>   DNA
<213>   synthetic

<400>   24
gactcttcgc gatgtacggg ccagatatac gcgttgacat tgattattga ctagttatta      60

atagtaatca attacggggt cattagttca tagcccatat atggagttcc gcgttacata     120

acttacggta aatggcccgc ctggctgacc gcccaacgac ccccgcccat tgacgtcaat     180

aatgacgtat gttcccatag taacgccaat agggactttc cattgacgtc aatgggtgga     240

ctatttacgg taaactgccc acttggcagt acatcaagtg tatcatatgc caagtacgcc     300

ccctattgac gtcaatgacg gtaaatggcc cgcctggcat tatgcccagt acatgacctt     360

atgggacttt cctacttggc agtacatcta cgtattagtc atcgctatta ccatggtgat     420

gcggttttgg cagtacatca atgggcgtgg atagcggttt gactcacggg gatttccaag     480

tctccacccc attgacgtca atgggagttt gttttggcac caaaatcaac gggactttcc     540

aaaatgtcgt aacaactccg ccccattgac gcaaatgggc ggtaggcgtg tacggtggga     600

ggtctatata agcagagctc tctggctaac tagagaaccc actgcttact ggcttatcga     660

aattaatacg actcactata gggagaccca agctggctag cgtttaaact taagcttggt     720

accgagctcg gatccactag tccagtgtgg tggaattctg cactgcagct cgcatctctc     780

cttcacgcgc ccgccgccct acctgaggcc gccatccacg ccggttgagt cgcgttctgc     840

cgcctcccgc ctgtggtgcc tcctgaactg cgtccgccgt ctaggtaagt ttaaagctca     900

ggtcgagacc gggcctttgt ccggcgctcc cttggagcct acctagactc agccggctct     960

ccacgctttg cctgaccctg cttgctcaac tctaggtaag ttaatgagac agatagaaac    1020

tggtcttgta gaaacagagt agtcgcctgc ttttctgcca ggtgctgact tctctcccct    1080

gggctttttt ctttttctca ggttgaaaag aagaagacga agaagacgaa gaagacaaac    1140

cgtcgtcgac tgccatgcgc cgctgattaa cgccgccacc atggcccacc gacgcagatc    1200

cagaagctgc cgtgaggacc agaagcccgt gatggatgat cagagggacc ttatctctaa    1260

caatgaacaa ctgccaatgc tcggcagacg gcctggggcc ccggagagca agtgcagcag    1320

aggagccttg tacacggggt tctccatttt agtgactctc cttctcgccg gccaagctac 1380

caccgcctac tttctgtacc aacagcaagg cagactagac aaactgacaa tcacaagcca 1440

gaaccttcag ctggagtctc tgcggatgaa gctgcccgct ttgtggatga gattgcttcc 1500

tctacttgct ctcctggcgc tctggggacc tgaccccgag caagagtttg ttaatcagca 1560

cctgtgtggg agtcatctgg tggaggcact ctatttagtg tgcggagaga ggggcttctt 1620

ctacactcca aagaccagac gggaggccga agaccttcaa gtggggcaag tagaactggg 1680

tggcggaccc ggtgccggga gccttcagcc gctcgccctg gagggctctc ttcagaaacg 1740

cggcatcgtg gagcagtgtt gcacatccat ttgctcactc taccagctgg agaactactg 1800

caacggaagc ggagtgaagc agacgttgaa ttttgatttg ttgaagttgg cgggggatgt 1860

ggagagcaat ccggggccga tgccccctag tggcctcaga cttttgttat tgttattacc 1920

gcttttatgg ctcttggtgc tgacaccggg ccgtccggct gctggcttgt cgacttgtaa 1980

gacaattgat atggaattgg tgaaacgaaa acggattgag gccatccgag gacagatttt 2040

gagcaagctg cggcttgcct cgccaccctc gcaaggggaa gtcccacccg gacctctacc 2100

agaagcagtc ctagcgctgt acaacagtac aagagataga gtggccgggg aatccgcaga 2160

accagagcct gagcctgaag ccgattatta tgcaaaggaa gtgactaggg tcctgatggt 2220

cgagacccat aacgaaatct acgacaaatt caaacaaagt acccactcta tctacatgtt 2280

cttcaacacc agtgagctaa gagaagccgt gcccgaacct gtgcttcttt cccgcgcaga 2340

actccgcctc ttgagactca aattgaaagt tgaacaacac gtagagcttt accagaaata 2400

ctctaataat tcatggcgat atctttctaa tcgtctcctc gccccatctg acagccctga 2460

atggctctcc ttcgacgtta cgggagttgt gcgccagtgg ctcagcagag gcggagagat 2520

agagggcttt cggctgagcg cacatgtatc tgtggactca agggacaaca cattgcaagt 2580

ggatattaac ggttttacaa ctggacggag aggggacctg gcgaccatcc acggcatgaa 2640

tagacctttc ctgctgctga tggctactcc cctggagagg gcacagcact tacagtcttc 2700

cagacaccgg cgcgccctgg atacaaacta ctgcttcagc tccaccgaaa agaactgttg 2760

cgtgcggcag ctgtacattg acttcagaaa ggatctgggc tggaagtgga ttcatgagcc 2820

caaggggtat catgccaact tctgtcttgg gccatgccca tacatctggt cactggatac 2880

ccagtactcc aaagttctgg ccttgtacaa tcaacacaac cctggagctt ccgccgctcc 2940

ttgctgtgtg ccccaagccc tagagcccct gcccatcgtt tattatgtcg gacgcaagcc 3000

caaagtagaa cagctatcaa atatgatcgt gagaagctgc aagtgtagct gataaacgcg 3060

tcgagcatgc atctagggcg gccaattccg cccctctccc ccccacccct ctccctcccc 3120

cccccctaac gttactggcc gaagccgctt ggaataaggc cggtgtgcgt ttgtctatat 3180

gttattttcc accatattgc cgtcttttgg caatgtgagg gcccggaaac ctggccctgt 3240

cttcttgacg agcattccta ggggtctttc ccctctcgcc aaaggaatgc aaggtctgtt 3300

gaatgtcgtg aaggaagcag ttcctctgga agcttcttga agacaaacaa cgtctgtagc 3360

gaccctttgt agacagcgga accccccacc tggcgataga tgcctctgcg gccaaaagcc 3420

acgtgtataa gatacacctg caaaggcggc acaaccccag tgccacgttg tgagttggat 3480

agttgtggaa agagtcaaat ggctctcctc aagcgtattc aacaaggggc tgaaggatgc 3540

ccagaaggta ccccattgta tgggatctga tctggggcct cggtgcacat gctttacatg 3600

tgtttagtcg aggttaaaaa acgtctaggc cccccgaacc acggggacgt ggttttcctt 3660

tgaaaaacac gatgataata tggctgccgc tcattctagt gcccttcttt gctgcctggt 3720

cctgctcacc ggggtgcgag ctagccctgg acaagggaca caatccgaaa actcgtgcac 3780

ccacttcccg ggcaacctcc ctaacatgct gagggacctc cgtgatgcct tcagtagagt 3840

gaagacgttc ttccaaatga aagatcagtt agataacctg ctcctgaagg agtcactctt 3900

agaagacttc aaaggatacc tcggctgcca agcacttagc gagatgattc aattctactt 3960

agaagaagtc atgcctcaag ctgagaatca agaccccgac atcaaagctc atgtgaattc 4020

tttgggagaa aatttgaaga ctttgcggct gcggctgcgg agatgtcacc gctttctgcc 4080

ctgtgagaac aaatcaaaag cggtcgagca agttaagaat gccttcaata agctacaaga 4140

gaagggcatc tacaaagcaa tgagcgagtt tgatatcttt atcaattaca ttgaagccta 4200

catgacaatg aagattagga atgccgcggg gagcggcgct actaacttca gcctgctgaa 4260

gcaggctgga gacgtggagg agaaccctgg acctatgtac agaatgcagc tgctgagctg 4320

catcgccctg agcctggccc tggtgaccaa cagcgcaccc acgtcctcta gcaccaagaa 4380

gacccagtta cagttggagc atctactttt agacctgcaa atgattttga acggcatcaa 4440

caactacaag aatcctaaac ttactcgcat gcttaccttc aaattttaca tgcccaagaa 4500

ggccaccgaa ctgaagcact tgcaatgtct ggaggaagaa ctcaagccgc tggaggaagt 4560

tctcaacctc gcgcagtcca agaatttcca cctccggcca agagacctga tcagtaacat 4620

taatgtgata gtgctggagc tgaagggaag cgagactaca tttatgtgcg agtacgccga 4680

tgaaaccgct acaatcgtcg agttcctgaa tagatggatc acattttgcc agtcaattat 4740

ctctactctg acatgataac tcgaggtcta gagggcccgt ttaaacccgc tgatcagcct 4800

cgactgtgcc ttctagttgc cagccatctg ttgtttgccc ctcccccgtg ccttccttga 4860

ccctggaagg tgccactccc actgtccttt cctaataaaa tgaggaaatt gcatcgcatt 4920

gtctgagtag gtgtcattct attctggggg gtggggtggg gcaggacagc aagggggagg 4980

attgggaaga caatagcagg catgctgggg atgcggtggg ctctatggct tctactgggc 5040

ggttttatgg acagcaagcg aaccggaatt gccagctggg gcgccctctg gtaaggttgg 5100

gaagccctgc aaagtaaact ggatggcttt ctcgccgcca aggatctgat ggcgcagggg 5160

atcaagctct gatcaagaga caggatgagg atcgtttcgc atggccaaag aagacaatat 5220

tgaaatgcaa ggtaccgttc ttgaaacgtt gcctaatacc atgttccgcg tagagttaga 5280

aaacggtcac gtggttactg cacacatctc cggtaaaatg cgcaaaaact acatccgcat 5340

cctgacgggc gacaaagtga ctgttgaact gaccccgtac gacctgagca aaggccgcat 5400

tgtcttccgt agtcgctgat aaattattaa cgcttacaat ttcctgatgc ggtattttct 5460

ccttacgcat ctgtgcggta tttcacaccg catacaggtg gcacttttcg gggaaatgtg 5520

cgcggaaccc ctatttgttt atttttctaa atacattcaa atatgtatcc gctcatgaga 5580

caataaccct gataaatgct tcaataatag cacgtgctaa aacttcattt ttaatttaaa 5640

aggatctagg tgaagatcct ttttgataat ctcatgacca aaatccctta acgtgagttt 5700

tcgttccact gagcgtcaga ccccgtagaa aagatcaaag gatcttcttg agatcctttt 5760

tttctgcgcg taatctgctg cttgcaaaca aaaaaaccac cgctaccagc ggtggtttgt 5820

ttgccggatc aagagctacc aactcttttt ccgaaggtaa ctggcttcag cagagcgcag 5880

ataccaaata ctgttcttct agtgtagccg tagttaggcc accacttcaa gaactctgta 5940

gcaccgccta catacctcgc tctgctaatc ctgttaccag tggctgctgc cagtggcgat 6000

aagtcgtgtc ttaccgggtt ggactcaaga cgatagttac cggataaggc gcagcggtcg 6060

ggctgaacgg ggggttcgtg cacacagccc agcttggagc gaacgaccta caccgaactg 6120

agatacctac agcgtgagct atgagaaagc gccacgcttc ccgaagggag aaaggcggac 6180

aggtatccgg taagcggcag ggtcggaaca ggagagcgca cgagggagct tccaggggga 6240

aacgcctggt atctttatag tcctgtcggg tttcgccacc tctgacttga gcgtcgattt 6300

ttgtgatgct cgtcaggggg gcggagccta tggaaaaacg ccagcaacgc ggccttttta 6360

cggttcctgg ccttttgctg gccttttgct cacatgttct t 6401

<210> 25
<211> 390
<212> PRT
<213> synthetic

<400> 25

Met Pro Pro Ser Gly Leu Arg Leu Leu Leu Leu Leu Leu Pro Leu Leu
1               5                   10                  15

Trp Leu Leu Val Leu Thr Pro Gly Arg Pro Ala Ala Gly Leu Ser Thr
            20                  25                  30

Cys Lys Thr Ile Asp Met Glu Leu Val Lys Arg Lys Arg Ile Glu Ala
        35                  40                  45

Ile Arg Gly Gln Ile Leu Ser Lys Leu Arg Leu Ala Ser Pro Pro Ser
    50                  55                  60

Gln Gly Glu Val Pro Pro Gly Pro Leu Pro Glu Ala Val Leu Ala Leu
65                  70                  75                  80

Tyr Asn Ser Thr Arg Asp Arg Val Ala Gly Glu Ser Ala Glu Pro Glu
                85                  90                  95

Pro Glu Pro Glu Ala Asp Tyr Tyr Ala Lys Glu Val Thr Arg Val Leu
            100                 105                 110

Met Val Glu Thr His Asn Glu Ile Tyr Asp Lys Phe Lys Gln Ser Thr
        115                 120                 125

His Ser Ile Tyr Met Phe Phe Asn Thr Ser Glu Leu Arg Glu Ala Val
    130                 135                 140

Pro Glu Pro Val Leu Leu Ser Arg Ala Glu Leu Arg Leu Leu Arg Leu
145                 150                 155                 160

Lys Leu Lys Val Glu Gln His Val Glu Leu Tyr Gln Lys Tyr Ser Asn
                165                 170                 175

Asn Ser Trp Arg Tyr Leu Ser Asn Arg Leu Leu Ala Pro Ser Asp Ser
            180                 185                 190

Pro Glu Trp Leu Ser Phe Asp Val Thr Gly Val Val Arg Gln Trp Leu
        195                 200                 205

Ser Arg Gly Gly Glu Ile Glu Gly Phe Arg Leu Ser Ala His Ser Ser
    210                 215                 220

Ser Asp Ser Arg Asp Asn Thr Leu Gln Val Asp Ile Asn Gly Phe Thr
225                 230                 235                 240

Thr Gly Arg Arg Gly Asp Leu Ala Thr Ile His Gly Met Asn Arg Pro
                245                 250                 255

Phe Leu Leu Leu Met Ala Thr Pro Leu Glu Arg Ala Gln His Leu Gln
            260                 265                 270

Ser Ser Arg His Arg Arg Ala Leu Asp Thr Asn Tyr Cys Phe Ser Ser
        275                 280                 285

Thr Glu Lys Asn Cys Cys Val Arg Gln Leu Tyr Ile Asp Phe Arg Lys
    290                 295                 300

Asp Leu Gly Trp Lys Trp Ile His Glu Pro Lys Gly Tyr His Ala Asn
305                 310                 315                 320

Phe Cys Leu Gly Pro Cys Pro Tyr Ile Trp Ser Leu Asp Thr Gln Tyr
                325                 330                 335

Ser Lys Val Leu Ala Leu Tyr Asn Gln His Asn Pro Gly Ala Ser Ala
            340                 345                 350

Ala Pro Cys Cys Val Pro Gln Ala Leu Glu Pro Leu Pro Ile Val Tyr
        355                 360                 365

Tyr Val Gly Arg Lys Pro Lys Val Glu Gln Leu Ser Asn Met Ile Val
    370                 375                 380

Arg Ser Cys Lys Cys Ser
385                 390

<210>  26
<211>  6389
<212>  DNA
<213>  synthetic

<400>  26
gactcttcgc gatgtacggg ccagatatac gcgttgacat tgattattga ctagttatta      60

atagtaatca attacggggt cattagttca tagcccatat atggagttcc gcgttacata     120

acttacggta aatggcccgc ctggctgacc gcccaacgac ccccgcccat tgacgtcaat     180

aatgacgtat gttcccatag taacgccaat agggactttc cattgacgtc aatgggtgga     240

ctatttacgg taaactgccc acttggcagt acatcaagtg tatcatatgc caagtacgcc     300

ccctattgac gtcaatgacg gtaaatggcc cgcctggcat tatgcccagt acatgacctt     360

atgggacttt cctacttggc agtacatcta cgtattagtc atcgctatta ccatggtgat     420

gcggttttgg cagtacatca atgggcgtgg atagcggttt gactcacggg gatttccaag     480

tctccacccc attgacgtca atgggagttt gttttggcac caaaatcaac gggactttcc     540

aaaatgtcgt aacaactccg ccccattgac gcaaatgggc ggtaggcgtg tacggtggga     600

ggtctatata agcagagctc tctggctaac tagagaaccc actgcttact ggcttatcga     660

aattaatacg actcactata gggagaccca agctggctag cgtttaaact taagcttggt     720

accgagctcg gatccactag tccagtgtgg tggaattctg cactgcagct cgcatctctc     780

cttcacgcgc ccgccgccct acctgaggcc gccatccacg ccggttgagt cgcgttctgc     840

cgcctcccgc ctgtggtgcc tcctgaactg cgtccgccgt ctaggtaagt ttaaagctca     900

ggtcgagacc gggcctttgt ccggcgctcc cttggagcct acctagactc agccggctct     960

ccacgctttg cctgaccctg cttgctcaac tctaggtaag ttaatgagac agatagaaac 1020

tggtcttgta gaaacagagt agtcgcctgc ttttctgcca ggtgctgact tctctcccct 1080

gggctttttt ctttttctca ggttgaaaag aagaagacga agaagacgaa gaagacaaac 1140

cgtcgtcgac tgccatgcgc cgctgattaa cgccgccacc atggcccacc gacgcagatc 1200

cagaagctgc cgtgaggacc agaagcccgt gatggatgat cagagggacc ttatctctaa 1260

caatgaacaa ctgccaatgc tcggcagacg gcctggggcc ccggagagca agtgcagcag 1320

aggagccttg tacacggggt tctccatttt agtgactctc cttctcgccg gccaagctac 1380

caccgcctac tttctgtacc aacagcaagg cagactagac aaactgacaa tcacaagcca 1440

gaaccttcag ctggagtctc tgcggatgaa gctgcccgct ttgtggatga gattgcttcc 1500

tctacttgct ctcctggcgc tctggggacc tgaccccgag caagagtttg ttaatcagca 1560

cctgtgtggg agtcatctgg tggaggcact ctatttagtg tgcggagaga ggggcttctt 1620

ctacactcca aagaccagac gggaggccga agaccttcaa gtggggcaag tagaactggg 1680

tggcggaccc ggtgccggga gccttcagcc gctcgccctg gagggctctc ttcagaaacg 1740

cggcatcgtg gagcagtgtt gcacatccat ttgctcactc taccagctgg agaactactg 1800

caacggaagc ggagtgaagc agacgttgaa ttttgatttg ttgaagttgg cgggggatgt 1860

ggagagcaat ccggggccga tgccccctag tggcctcaga cttttgttat tgttattacc 1920

gcttttatgg ctcttggtgc tgacaccggg ccgtccggct gctggcttgt cgacttgtaa 1980

gacaattgat atggaattgg tgaaacgaaa acggattgag gccatccgag gacagatttt 2040

gagcaagctg cggcttgcct cgccaccctc gcaaggggaa gtcccacccg gacctctacc 2100

agaagcagtc ctagcgctgt acaacagtac aagagataga gtggccgggg aatccgcaga 2160

accagagcct gagcctgaag ccgattatta tgcaaaggaa gtgactaggg tcctgatggt 2220

cgagacccat aacgaaatct acgacaaatt caaacaaagt acccactcta tctacatgtt 2280

cttcaacacc agtgagctaa gagaagccgt gcccgaacct gtgcttcttt cccgcgcaga 2340

actccgcctc ttgagactca aattgaaagt tgaacaacac gtagagcttt accagaaata 2400

ctctaataat tcatggcgat atctttctaa tcgtctcctc gccccatctg acagccctga 2460

atggctctcc ttcgacgtta cgggagttgt gcgccagtgg ctcagcagag gcggagagat 2520

agagggcttt cggctgagcg cacatagctc tagcgactca agggacaaca cattgcaagt 2580

ggatattaac ggttttacaa ctggacggag aggggacctg gcgaccatcc acggcatgaa 2640

tagacctttc ctgctgctga tggctactcc cctggagagg gcacagcact tacagtcttc 2700

cagacaccgg cgcgccctgg atacaaacta ctgcttcagc tccaccgaaa agaactgttg 2760

cgtgcggcag ctgtacattg acttcagaaa ggatctgggc tggaagtgga ttcatgagcc 2820

caaggggtat catgccaact tctgtcttgg gccatgccca tacatctggt cactggatac 2880

ccagtactcc aaagttctgg ccttgtacaa tcaacacaac cctggagctt ccgccgctcc 2940

ttgctgtgtg ccccaagccc tagagcccct gcccatcgtt tattatgtcg gacgcaagcc 3000

caaagtagaa cagctatcaa atatgatcgt gagaagctgc aagtgtagct gataaacgcg 3060

tcgagcatgc atctagggcg gccaattccg cccctctccc ccccacccct ctccctcccc 3120

cccccctaac gttactggcc gaagccgctt ggaataaggc cggtgtgcgt ttgtctatat 3180

gttattttcc accatattgc cgtcttttgg caatgtgagg gcccggaaac ctggccctgt 3240

cttcttgacg agcattccta ggggtctttc ccctctcgcc aaaggaatgc aaggtctgtt 3300

gaatgtcgtg aaggaagcag ttcctctgga agcttcttga agacaaacaa cgtctgtagc 3360

gaccctttgt agacagcgga accccccacc tggcgataga tgcctctgcg gccaaaagcc 3420

acgtgtataa gatacacctg caaaggcggc acaaccccag tgccacgttg tgagttggat 3480

agttgtggaa agagtcaaat ggctctcctc aagcgtattc aacaaggggc tgaaggatgc 3540

ccagaaggta ccccattgta tgggatctga tctggggcct cggtgcacat gctttacatg 3600

tgtttagtcg aggttaaaaa acgtctaggc cccccgaacc acggggacgt ggttttcctt 3660

tgaaaaacac gatgataata tgatgcacag ctcagcactg ctctgttgcc tggtcctcct 3720

gactggggtg agggccagcc caggccaggg cacccagtct gagaacagct gcacccactt 3780

cccaggcaac ctgcctaaca tgcttcgaga tctccgagat gccttcagca gagtgaagac 3840

tttctttcaa atgaaggatc agctggacaa cttgttgtta aaggagtcct tgctggagga 3900

ctttaagggt tacctgggtt gccaagcctt gtctgagatg atccagtttt acctggagga 3960

ggtgatgccc caagctgaga accaagaccc agacatcaag gcgcatgtga actccctggg 4020

ggagaacctg aagaccctca ggctgaggct acggcgctgt catcgatttc ttccctgtga 4080

aaacaagagc aaggccgtgg agcaggtgaa gaatgccttt aataagctcc aagagaaagg 4140

catctacaaa gccatgagtg agtttgacat cttcatcaac tacatagaag cctacatgac 4200

aatgaagata cgaaacggga gcggcgctac taacttcagc ctgctgaagc aggctggaga 4260

cgtggaggag aaccctggac ctatgtacag aatgcagctg ctgagctgca tcgccctgag 4320

cctggccctg gtgaccaaca gcgcacccac gtcctctagc accaagaaga cccagttaca 4380

gttggagcat ctacttttag acctgcaaat gattttgaac ggcatcaaca actacaagaa 4440

tcctaaactt actcgcatgc ttaccttcaa attttacatg cccaagaagg ccaccgaact 4500

gaagcacttg caatgtctgg aggaagaact caagccgctg gaggaagttc tcaacctcgc 4560

gcagtccaag aatttccacc tccggccaag agacctgatc agtaacatta atgtgatagt 4620

gctggagctg aagggaagcg agactacatt tatgtgcgag tacgccgatg aaaccgctac 4680

aatcgtcgag ttcctgaata gatggatcac attttgccag tcaattatct ctactctgac 4740

atgataactc gaggtctaga gggcccgttt aaacccgctg atcagcctcg actgtgcctt 4800

ctagttgcca gccatctgtt gtttgcccct cccccgtgcc ttccttgacc ctggaaggtg 4860

ccactcccac tgtcctttcc taataaaatg aggaaattgc atcgcattgt ctgagtaggt 4920

gtcattctat tctggggggt ggggtggggc aggacagcaa gggggaggat tgggaagaca 4980

atagcaggca tgctggggat gcggtgggct ctatggcttc tactgggcgg ttttatggac 5040

agcaagcgaa ccggaattgc cagctggggc gccctctggt aaggttggga agccctgcaa 5100

agtaaactgg atggctttct cgccgccaag gatctgatgg cgcaggggat caagctctga 5160

tcaagagaca ggatgaggat cgtttcgcat ggccaaagaa gacaatattg aaatgcaagg 5220

taccgttctt gaaacgttgc ctaataccat gttccgcgta gagttagaaa acggtcacgt 5280

ggttactgca cacatctccg gtaaaatgcg caaaaactac atccgcatcc tgacgggcga 5340

caaagtgact gttgaactga ccccgtacga cctgagcaaa ggccgcattg tcttccgtag 5400

tcgctgataa attattaacg cttacaattt cctgatgcgg tattttctcc ttacgcatct 5460

gtgcggtatt tcacaccgca tacaggtggc acttttcggg gaaatgtgcg cggaacccct 5520

atttgtttat ttttctaaat acattcaaat atgtatccgc tcatgagaca ataaccctga 5580

taaatgcttc aataatagca cgtgctaaaa cttcattttt aatttaaaag gatctaggtg 5640

aagatccttt ttgataatct catgaccaaa atcccttaac gtgagttttc gttccactga 5700

gcgtcagacc ccgtagaaaa gatcaaagga tcttcttgag atcctttttt tctgcgcgta 5760

atctgctgct tgcaaacaaa aaaaccaccg ctaccagcgg tggtttgttt gccggatcaa 5820

gagctaccaa ctctttttcc gaaggtaact ggcttcagca gagcgcagat accaaatact 5880

gttcttctag tgtagccgta gttaggccac cacttcaaga actctgtagc accgcctaca 5940

tacctcgctc tgctaatcct gttaccagtg gctgctgcca gtggcgataa gtcgtgtctt 6000

accgggttgg actcaagacg atagttaccg gataaggcgc agcggtcggg ctgaacgggg 6060

ggttcgtgca cacagcccag cttggagcga acgacctaca ccgaactgag atacctacag 6120

cgtgagctat gagaaagcgc cacgcttccc gaagggagaa aggcggacag gtatccggta 6180

agcggcaggg tcggaacagg agagcgcacg agggagcttc cagggggaaa cgcctggtat 6240

ctttatagtc ctgtcgggtt tcgccacctc tgacttgagc gtcgattttt gtgatgctcg 6300

tcaggggggc ggagcctatg gaaaaacgcc agcaacgcgg cctttttacg gttcctgggc 6360

ttttgctggc cttttgctca catgttctt 6389

<210> 27
<211> 15
<212> PRT
<213> synthetic

<400>  27

Ser His Leu Val Glu Ala Leu Tyr Leu Val Cys Gly Gly Glu Gly
1               5                   10                  15


<210>  28
<211>  6383
<212>  DNA
<213>  synthetic

<400>  28
gactcttcgc gatgtacggg ccagatatac gcgttgacat tgattattga ctagttatta      60

atagtaatca attacggggt cattagttca tagcccatat atggagttcc gcgttacata     120

acttacggta aatggcccgc ctggctgacc gcccaacgac ccccgcccat tgacgtcaat     180

aatgacgtat gttcccatag taacgccaat agggactttc cattgacgtc aatgggtgga     240

ctatttacgg taaactgccc acttggcagt acatcaagtg tatcatatgc caagtacgcc     300

ccctattgac gtcaatgacg gtaaatggcc cgcctggcat tatgcccagt acatgacctt     360

atgggacttt cctacttggc agtacatcta cgtattagtc atcgctatta ccatggtgat     420

gcggttttgg cagtacatca atgggcgtgg atagcggttt gactcacggg gatttccaag     480

tctccacccc attgacgtca atgggagttt gttttggcac caaaatcaac gggactttcc     540

aaaatgtcgt aacaactccg ccccattgac gcaaatgggc ggtaggcgtg tacggtggga     600

ggtctatata agcagagctc tctggctaac tagagaaccc actgcttact ggcttatcga     660

aattaatacg actcactata gggagaccca agctggctag cgtttaaact taagcttggt     720

accgagctcg gatccactag tccagtgtgg tggaattctg cagctcgcat ctctccttca     780

cgcgcccgcc gccctacctg aggccgccat ccacgccggt tgagtcgcgt tctgccgcct     840

cccgcctgtg gtgcctcctg aactgcgtcc gccgtctagg taagtttaaa gctcaggtcg     900

agaccgggcc tttgtccggc gctcccttgg agcctaccta gactcagccg gctctccacg     960

ctttgcctga ccctgcttgc tcaactctag gtaagttaat gagacagata gaaactggtc    1020

ttgtagaaac agagtagtcg cctgcttttc tgccaggtgc tgacttctct cccctgggct    1080

tttttctttt tctcaggttg aaaagaagaa gacgaagaag acgaagaaga caaaccgtcg    1140

tcgactgcca tgcgccgctg attaacgccg ccaccatggc ccaccgacgc agatccagaa    1200

gctgccgtga ggaccagaag cccgtgatgg atgatcagag ggaccttatc tctaacaatg    1260

aacaactgcc aatgctcggc agacggcctg gggccccgga gagcaagtgc agcagaggag    1320

ccttgtacac ggggttctcc attttagtga ctctccttct cgccggccaa gctaccaccg    1380

cctactttct gtaccaacag caaggcagac tagacaaact gacaatcaca agccagaacc    1440

ttcagctgga gtctctgcgg atgaagctgc ccgctttgtg gatgagattg cttcctctac    1500

ttgctctcct ggcgctctgg ggacctgacc ccgagcaaga gtttgttaat cagcacctgt    1560

gtgggagtca tctggtggag gcactctatt tagtgtgcgg agagaggggc ttcttctaca 1620

ctccaaagac cagacgggag gccgaagacc ttcaagtggg gcaagtagaa ctgggtggcg 1680

gacccggtgc cgggagcctt cagccgctcg ccctggaggg ctctcttcag aaacgcggca 1740

tcgtggagca gtgttgcaca tccatttgct cactctacca gctggagaac tactgcaacg 1800

gaagcggagt gaagcagacg ttgaattttg atttgttgaa gttggcgggg gatgtggaga 1860

gcaatccggg gccgatgccc cctagtggcc tcagactttt gttattgtta ttaccgcttt 1920

tatggctctt ggtgctgaca ccgggccgtc cggctgctgg cttgtcgact tgtaagacaa 1980

ttgatatgga attggtgaaa cgaaaacgga ttgaggccat ccgaggacag attttgagca 2040

agctgcggct tgcctcgcca ccctcgcaag gggaagtccc acccggacct ctaccagaag 2100

cagtcctagc gctgtacaac agtacaagag atagagtggc cggggaatcc gcagaaccag 2160

agcctgagcc tgaagccgat tattatgcaa aggaagtgac tagggtcctg atggtcgaga 2220

cccataacga aatctacgac aaattcaaac aaagtaccca ctctatctac atgttcttca 2280

acaccagtga gctaagagaa gccgtgcccg aacctgtgct tctttcccgc gcagaactcc 2340

gcctcttgag actcaaattg aaagttgaac aacacgtaga gctttaccag aaatactcta 2400

ataattcatg gcgatatctt tctaatcgtc tcctcgcccc atctgacagc cctgaatggc 2460

tctccttcga cgttacggga gttgtgcgcc agtggctcag cagaggcgga gagatagagg 2520

gctttcggct gagcgcacat agctctagcg actcaaggga caacacattg caagtggata 2580

ttaacggttt tacaactgga cggagagggg acctggcgac catccacggc atgaatagac 2640

ctttcctgct gctgatggct actcccctgg agagggcaca gcacttacag tcttccagac 2700

accggcgcgc cctggataca aactactgct tcagctccac cgaaaagaac tgttgcgtgc 2760

ggcagctgta cattgacttc agaaaggatc tgggctggaa gtggattcat gagcccaagg 2820

ggtatcatgc caacttctgt cttgggccat gcccatacat ctggtcactg gatacccagt 2880

actccaaagt tctggccttg tacaatcaac acaaccctgg agcttccgcc gctccttgct 2940

gtgtgcccca agccctagag cccctgccca tcgtttatta tgtcggacgc aagcccaaag 3000

tagaacagct atcaaatatg atcgtgagaa gctgcaagtg tagctgataa acgcgtcgag 3060

catgcatcta gggcggccaa ttccgcccct ctccccccca cccctctccc tccccccccc 3120

ctaacgttac tggccgaagc cgcttggaat aaggccggtg tgcgtttgtc tatatgttat 3180

tttccaccat attgccgtct tttggcaatg tgagggcccg gaaacctggc cctgtcttct 3240

tgacgagcat tcctaggggt ctttcccctc tcgccaaagg aatgcaaggt ctgttgaatg 3300

tcgtgaagga agcagttcct ctggaagctt cttgaagaca aacaacgtct gtagcgaccc 3360

tttgtagaca gcggaacccc ccacctggcg atagatgcct ctgcggccaa aagccacgtg 3420

tataagatac acctgcaaag gcggcacaac cccagtgcca cgttgtgagt tggatagttg 3480

tggaaagagt caaatggctc tcctcaagcg tattcaacaa ggggctgaag gatgcccaga 3540

aggtacccca ttgtatggga tctgatctgg ggcctcggtg cacatgcttt acatgtgttt 3600

agtcgaggtt aaaaaacgtc taggcccccc gaaccacggg gacgtggttt tcctttgaaa 3660

aacacgatga taatatgatg cacagctcag cactgctctg ttgcctggtc ctcctgactg 3720

gggtgagggc cagcccaggc cagggcaccc agtctgagaa cagctgcacc cacttcccag 3780

gcaacctgcc taacatgctt cgagatctcc gagatgcctt cagcagagtg aagactttct 3840

ttcaaatgaa ggatcagctg gacaacttgt tgttaaagga gtccttgctg gaggacttta 3900

agggttacct gggttgccaa gccttgtctg agatgatcca gttttacctg gaggaggtga 3960

tgccccaagc tgagaaccaa gacccagaca tcaaggcgca tgtgaactcc ctgggggaga 4020

acctgaagac cctcaggctg aggctacggc gctgtcatcg atttcttccc tgtgaaaaca 4080

agagcaaggc cgtggagcag gtgaagaatg cctttaataa gctccaagag aaaggcatct 4140

acaaagccat gagtgagttt gacatcttca tcaactacat agaagcctac atgacaatga 4200

agatacgaaa cgggagcggc gctactaact tcagcctgct gaagcaggct ggagacgtgg 4260

aggagaaccc tggacctatg tacagaatgc agctgctgag ctgcatcgcc ctgagcctgg 4320

ccctggtgac caacagcgca cccacgtcct ctagcaccaa gaagacccag ttacagttgg 4380

agcatctact tttagacctg caaatgattt tgaacggcat caacaactac aagaatccta 4440

aacttactcg catgcttacc ttcaaatttt acatgcccaa gaaggccacc gaactgaagc 4500

acttgcaatg tctggaggaa gaactcaagc cgctggagga agttctcaac ctcgcgcagt 4560

ccaagaattt ccacctccgg ccaagagacc tgatcagtaa cattaatgtg atagtgctgg 4620

agctgaaggg aagcgagact acatttatgt gcgagtacgc cgatgaaacc gctacaatcg 4680

tcgagttcct gaatagatgg atcacatttt gccagtcaat tatctctact ctgacatgat 4740

aactcgagtc tagagggccc gtttaaaccc gctgatcagc ctcgactgtg ccttctagtt 4800

gccagccatc tgttgtttgc ccctcccccg tgccttcctt gaccctggaa ggtgccactc 4860

ccactgtcct ttcctaataa aatgaggaaa ttgcatcgca ttgtctgagt aggtgtcatt 4920

ctattctggg gggtggggtg gggcaggaca gcaaggggga ggattgggaa gacaatagca 4980

ggcatgctgg ggatgcggtg ggctctatgg cttctactgg gcggttttat ggacagcaag 5040

cgaaccggaa ttgccagctg gggcgccctc tggtaaggtt gggaagccct gcaaagtaaa 5100

ctggatggct ttctcgccgc caaggatctg atggcgcagg ggatcaagct ctgatcaaga 5160

gacaggatga ggatcgtttc gcatggccaa agaagacaat attgaaatgc aaggtaccgt 5220

tcttgaaacg ttgcctaata ccatgttccg cgtagagtta gaaaacggtc acgtggttac 5280

tgcacacatc tccggtaaaa tgcgcaaaaa ctacatccgc atcctgacgg gcgacaaagt 5340

gactgttgaa ctgaccccgt acgacctgag caaaggccgc attgtcttcc gtagtcgctg 5400

ataaattatt aacgcttaca atttcctgat gcggtatttt ctccttacgc atctgtgcgg 5460

tatttcacac cgcatacagg tggcactttt cggggaaatg tgcgcggaac ccctatttgt 5520

ttatttttct aaatacattc aaatatgtat ccgctcatga gacaataacc ctgataaatg 5580

cttcaataat agcacgtgct aaaacttcat ttttaattta aaaggatcta ggtgaagatc 5640

ctttttgata atctcatgac caaaatccct taacgtgagt tttcgttcca ctgagcgtca 5700

gaccccgtag aaaagatcaa aggatcttct tgagatcctt tttttctgcg cgtaatctgc 5760

tgcttgcaaa caaaaaaacc accgctacca gcggtggttt gtttgccgga tcaagagcta 5820

ccaactcttt ttccgaaggt aactggcttc agcagagcgc agataccaaa tactgttctt 5880

ctagtgtagc cgtagttagg ccaccacttc aagaactctg tagcaccgcc tacatacctc 5940

gctctgctaa tcctgttacc agtggctgct gccagtggcg ataagtcgtg tcttaccggg 6000

ttggactcaa gacgatagtt accggataag gcgcagcggt cgggctgaac ggggggttcg 6060

tgcacacagc ccagcttgga gcgaacgacc tacaccgaac tgagatacct acagcgtgag 6120

ctatgagaaa gcgccacgct tcccgaaggg agaaaggcgg acaggtatcc ggtaagcggc 6180

agggtcggaa caggagagcg cacgagggag cttccagggg gaaacgcctg gtatctttat 6240

agtcctgtcg ggtttcgcca cctctgactt gagcgtcgat ttttgtgatg ctcgtcaggg 6300

gggcggagcc tatggaaaaa cgccagcaac gcggcctttt tacggttcct gggcttttgc 6360

tggccttttg ctcacatgtt ctt 6383

<210> 29
<211> 6383
<212> DNA
<213> synthetic

<400> 29
gactcttcgc gatgtacggg ccagatatac gcgttgacat tgattattga ctagttatta 60

atagtaatca attacggggt cattagttca tagcccatat atggagttcc gcgttacata 120

acttacggta aatggcccgc ctggctgacc gcccaacgac ccccgcccat tgacgtcaat 180

aatgacgtat gttcccatag taacgccaat agggactttc cattgacgtc aatgggtgga 240

ctatttacgg taaactgccc acttggcagt acatcaagtg tatcatatgc caagtacgcc 300

ccctattgac gtcaatgacg gtaaatggcc cgcctggcat tatgcccagt acatgacctt 360

atgggacttt cctacttggc agtacatcta cgtattagtc atcgctatta ccatggtgat 420

gcggttttgg cagtacatca atgggcgtgg atagcggttt gactcacggg gatttccaag 480

tctccacccc attgacgtca atgggagttt gttttggcac caaaatcaac gggactttcc 540

aaaatgtcgt aacaactccg ccccattgac gcaaatgggc ggtaggcgtg tacggtggga 600

ggtctatata agcagagctc tctggctaac tagagaaccc actgcttact ggcttatcga 660

aattaatacg actcactata gggagaccca agctggctag cgtttaaact taagcttggt 720

accgagctcg gatccactag tccagtgtgg tggaattctg cagctcgcat ctctccttca 780

cgcgcccgcc gccctacctg aggccgccat ccacgccggt tgagtcgcgt tctgccgcct 840

cccgcctgtg gtgcctcctg aactgcgtcc gccgtctagg taagtttaaa gctcaggtcg 900

agaccgggcc tttgtccggc gctcccttgg agcctaccta gactcagccg gctctccacg 960

ctttgcctga ccctgcttgc tcaactctag gtaagttaat gagacagata gaaactggtc 1020

ttgtagaaac agagtagtcg cctgcttttc tgccaggtgc tgacttctct cccctgggct 1080

tttttctttt tctcaggttg aaaagaagaa gacgaagaag acgaagaaga caaaccgtcg 1140

tcgactgcca tgcgccgctg attaacgccg ccaccatggc ccaccgacgc agatccagaa 1200

gctgccgtga ggaccagaag cccgtgatgg atgatcagag ggaccttatc tctaacaatg 1260

aacaactgcc aatgctcggc agacggcctg gggccccgga gagcaagtgc agcagaggag 1320

ccttgtacac ggggttctcc attttagtga ctctccttct cgccggccaa gctaccaccg 1380

cctactttct gtaccaacag caaggcagac tagacaaact gacaatcaca agccagaacc 1440

ttcagctgga gtctctgcgg atgaagctgc ccgctttgtg gatgagattg cttcctctac 1500

ttgctctcct ggcgctctgg ggacctgacc ccgagcaaga gtttgttaat cagcacctgt 1560

gtgggagtca tctggtggag gcactctatt tagtgtgcgg agagaggggc ttcttctaca 1620

ctccaaagac cagacgggag gccgaagacc ttcaagtggg gcaagtagaa ctgggtggcg 1680

gacccggtgc cgggagcctt cagccgctcg ccctggaggg ctctcttcag aaacgcggca 1740

tcgtggagca gtgttgcaca tccatttgct cactctacca gctggagaac tactgcaacg 1800

gaagcggagt gaagcagacg ttgaattttg atttgttgaa gttggcgggg gatgtggaga 1860

gcaatccggg gccgatgccc cctagtggcc tcagactttt gttattgtta ttaccgcttt 1920

tatggctctt ggtgctgaca ccgggccgtc cggctgctgg cttgtcgact tgtaagacaa 1980

ttgatatgga attggtgaaa cgaaaacgga ttgaggccat ccgaggacag attttgagca 2040

agctgcggct tgcctcgcca ccctcgcaag gggaagtccc acccggacct ctaccagaag 2100

cagtcctagc gctgtacaac agtacaagag atagagtggc cggggaatcc gcagaaccag 2160

agcctgagcc tgaagccgat tattatgcaa aggaagtgac tagggtcctg atggtcgaga 2220

cccataacga aatctacgac aaattcaaac aaagtaccca ctctatctac atgttcttca 2280

acaccagtga gctaagagaa gccgtgcccg aacctgtgct tctttcccgc gcagaactcc 2340

gcctcttgag actcaaattg aaagttgaac aacacgtaga gctttaccag aaatactcta 2400

ataattcatg gcgatatctt tctaatcgtc tcctcgcccc atctgacagc cctgaatggc 2460

tctccttcga cgttacggga gttgtgcgcc agtggctcag cagaggcgga gagatagagg 2520
gctttcggct gagcgcacat agctctagcg actcaaggga caacacattg caagtggata 2580
ttaacggttt tacaactgga cggagagggg acctggcgac catccacggc atgaatagac 2640
ctttcctgct gctgatggct actcccctgg agagggcaca gcacttacag tcttccagac 2700
accggcgcgc cctggataca aactactgct tcagctccac cgaaaagaac tgttgcgtgc 2760
ggcagctgta cattgacttc agaaaggatc tgggctggaa gtggattcat gagcccaagg 2820
ggtatcatgc caacttctgt cttgggccat gcccatacat ctggtcactg gatacccagt 2880
actccaaagt tctggccttg tacaatcaac acaaccctgg agcttccgcc gctccttgct 2940
gtgtgcccca agccctagag cccctgccca tcgtttatta tgtcggacgc aagcccaaag 3000
tagaacagct atcaaatatg atcgtgagaa gctgcaagtg tagctgataa acgcgtcgag 3060
catgcatcta gggcggccaa ttccgcccct ctccccccca cccctctccc tccccccccc 3120
ctaacgttac tggccgaagc cgcttggaat aaggccggtg tgcgtttgtc tatatgttat 3180
tttccaccat attgccgtct tttggcaatg tgagggcccg gaaacctggc cctgtcttct 3240
tgacgagcat tcctaggggt ctttcccctc tcgccaaagg aatgcaaggt ctgttgaatg 3300
tcgtgaagga agcagttcct ctggaagctt cttgaagaca aacaacgtct gtagcgaccc 3360
tttgtagaca gcggaacccc ccacctggcg atagatgcct ctgcggccaa aagccacgtg 3420
tataagatac acctgcaaag gcggcacaac cccagtgcca cgttgtgagt tggatagttg 3480
tggaaagagt caaatggctc tcctcaagcg tattcaacaa ggggctgaag gatgcccaga 3540
aggtacccca ttgtatggga tctgatctgg ggcctcggtg cacatgcttt acatgtgttt 3600
agtcgaggtt aaaaaacgtc taggcccccc gaaccacggg gacgtggttt tcctttgaaa 3660
aacacgatga taatatgatg cacagctcag cactgctctg ttgcctggtc ctcctgactg 3720
gggtgagggc cagcccaggc cagggcaccc agtctgagaa cagctgcacc cacttcccag 3780
gcaacctgcc taacatgctt cgagatctcc gagatgcctt cagcagagtg aagactttct 3840
ttcaaatgaa ggatcagctg gacaacttgt tgttaaagga gtccttgctg gaggacttta 3900
agggttacct gggttgccaa gccttgtctg agatgatcca gttttacctg gaggaggtga 3960
tgccccaagc tgagaaccaa gacccagaca tcaaggcgca tgtgaactcc ctgggggaga 4020
acctgaagac cctcaggctg aggctacggc gctgtcatcg atttcttccc tgtgaaaaca 4080
agagcaaggc cgtggagcag gtgaagaatg cctttaataa gctccaagag aaaggcatct 4140
acaaagccat gagtgagttt gacatcttca tcaactacat agaagcctac atgacaatga 4200
agatacgaaa cgggagcggc gctactaact tcagcctgct gaagcaggct ggagacgtgg 4260
aggagaaccc tggacctatg tacagaatgc agctgctgag ctgcatcgcc ctgagcctgg 4320
ccctggtgac caacagcgca cccacgtcct ctagcaccaa gaagacccag ttacagttgg 4380

agcatctact tttagacctg caaatgattt tgaacggcat caacaactac aagaatccta 4440

aacttactcg catgcttacc ttcaaatttt acatgcccaa gaaggccacc gaactgaagc 4500

acttgcaatg tctggaggaa gaactcaagc cgctggagga agttctcaac ctcgcgcagt 4560

ccaagaattt ccacctccgg ccaagagacc tgatcagtaa cattaatgtg atagtgctgg 4620

agctgaaggg aagcgagact acatttatgt gcgagtacgc cgatgaaacc gctacaatcg 4680

tcgagttcct gaatagatgg atcacatttt gccagtcaat tatctctact ctgacatgat 4740

aactcgagtc tagagggccc gtttaaaccc gctgatcagc ctcgactgtg ccttctagtt 4800

gccagccatc tgttgtttgc ccctcccccg tgccttcctt gaccctggaa ggtgccactc 4860

ccactgtcct ttcctaataa aatgaggaaa ttgcatcgca ttgtctgagt aggtgtcatt 4920

ctattctggg gggtggggtg gggcaggaca gcaaggggga ggattgggaa gacaatagca 4980

ggcatgctgg ggatgcggtg ggctctatgg cttctactgg gcggttttat ggacagcaag 5040

cgaaccggaa ttgccagctg gggcgccctc tggtaaggtt gggaagccct gcaaagtaaa 5100

ctggatggct ttctcgccgc caaggatctg atggcgcagg ggatcaagct ctgatcaaga 5160

gacaggatga ggatcgtttc gcatggccaa agaagacaat attgaaatgc aaggtaccgt 5220

tcttgaaacg ttgcctaata ccatgttccg cgtagagtta gaaaacggtc acgtggttac 5280

tgcacacatc tccggtaaaa tgcgcaaaaa ctacatccgc atcctgacgg gcgacaaagt 5340

gactgttgaa ctgaccccgt acgacctgag caaaggccgc attgtcttcc gtagtcgctg 5400

ataaattatt aacgcttaca atttcctgat gcggtatttt ctccttacgc atctgtgcgg 5460

tatttcacac cgcatacagg tggcactttt cggggaaatg tgcgcggaac ccctatttgt 5520

ttatttttct aaatacattc aaatatgtat ccgctcatga gacaataacc ctgataaatg 5580

cttcaataat agcacgtgct aaaacttcat ttttaattta aaaggatcta ggtgaagatc 5640

ctttttgata atctcatgac caaaatccct taacgtgagt tttcgttcca ctgagcgtca 5700

gaccccgtag aaaagatcaa aggatcttct tgagatcctt tttttctgcg cgtaatctgc 5760

tgcttgcaaa caaaaaaacc accgctacca gcggtggttt gtttgccgga tcaagagcta 5820

ccaactcttt ttccgaaggt aactggcttc agcagagcgc agataccaaa tactgttctt 5880

ctagtgtagc cgtagttagg ccaccacttc aagaactctg tagcaccgcc tacatacctc 5940

gctctgctaa tcctgttacc agtggctgct gccagtggcg ataagtcgtg tcttaccggg 6000

ttggactcaa gacgatagtt accggataag gcgcagcggt cgggctgaac ggggggttcg 6060

tgcacacagc ccagcttgga gcgaacgacc tacaccgaac tgagatacct acagcgtgag 6120

ctatgagaaa gcgccacgct tcccgaaggg agaaaggcgg acaggtatcc ggtaagcggc 6180

agggtcggaa caggagagcg cacgagggag cttccagggg gaaacgcctg gtatctttat 6240

agtcctgtcg ggtttcgcca cctctgactt gagcgtcgat ttttgtgatg ctcgtcaggg 6300

gggcggagcc tatggaaaaa cgccagcaac gcggcctttt tacggttcct ggccttttgc 6360

tggccttttg ctcacatgtt ctt 6383

## Description

### TECHNICAL FIELD

The present invention relates to tolerogeneic DNA immuno-therapy vaccines for reducing antigen-specific T cell reactivity.

### BACKGROUND

According to traditional vaccine approaches, purified protein/antigen is injected in a person/patient/animal in order to stimulate immune responses specifically to that protein/antigen. This vaccine approach tends to impact primarily antibody production, while the T cells tend not to be significantly affected, other than to generate T cell memory of the antigen. Traditional vaccine approaches are thus not considered suitable in connection with treatment and/or prevention of T cell driven diseases such as e.g. Type 1 diabetes (T1D), as activation of T cells, especially CD8⁺ T cells, are considered the causative agent of this disease. Experimental approaches with tolerogenic, protein-based vaccines have targeted primarily antibody producing B cells rather than disease relevant T cells.

DNA based vaccines, in contrast to protein-based vaccines, are usually plasmids encoding particular antigens - these plasmids are taken up by cells in the host's body ("transfected"). These transfected host cells then produce the antigen and process the antigen into small fragments (T-cell epitopes) for presentation to the immune system, in particular to circulating T cells. As T cells can only detect these small antigen fragments and not whole proteins, this approach preferentially leads to a modification of T cell responses, especially for CD8⁺ T cells (or cytotoxic T cells), the key drivers of e.g. T1D pathology. Thus, DNA vaccines, rather than protein vaccines, are suitable for inducing T cell responses. While no DNA vaccines are currently available for human use, there are three stimulatory plasmid DNA vaccines licensed for veterinary use, inducing immunity to Equine Infectious Anemia Virus, West Nile Virus, and certain canine cancers.

In contrast to stimulatory DNA vaccines, tolerogenic DNA immuno-therapy vaccines are intended to suppress immune reactivity towards an antigen, rather than activating immune responses against it. These vaccines do not stimulate immunity against the encoded antigen, or change the type of stimulation (as e.g. antigenic desensitization vaccination approaches for allergies does), but instead cause depletion, and/or lack of function, and/or death of self-reactive T cells. In order to do so, the antigen must be presented to the immune system without co-stimulation or inflammatory effects, which would otherwise prime stimulatory immune responses. This approach of presenting an antigen to be ignored by the immune system, or tolerized against, could be of value in treating autoimmune diseases, as the specific mechanism of the disease would thus be targeted rather than systemically suppressing the entire immune response. A tolerogenic DNA immuno-therapy vaccine is thus a mild method of modulating undesired immune responses.

The end goal of a T1D-specific tolerogenic DNA immuno-therapy vaccine is to preserve beta cell function and endogenous insulin production. This may occur through prevention or delay of disease (especially valuable in pediatric and young adult cohorts where monitoring is difficult and "normalcy" of life is a major patient driver) or extension of the "honey moon phase" of minimal monitoring and insulin usage that often occurs for the first six months after T1D diagnosis.

While DNA based vaccines are known to be safe, none of the (stimulatory or tolerogenic) DNA vaccines that have been tested in clinical studies have sufficient potency as a stand-alone approach for treatment of e.g. T1D. Tolerogenic DNA vaccines known in the art showed little efficacy and typically required highly artificial systems to induce the desired effects. There is thus a need in the art for tolerogenic DNA immuno-therapy vaccines with significantly increased potency, without compromising the safety profile and preferably also without requiring an inconvenient administration regimen.

### SUMMARY

The present invention relates to a multi-cistronic vector/plasmid which co-expresses/encodes a cellularly retained antigen, such as insulin, as well as secreted immune modifiers such as TGF-β, IL-10, and optionally IL-2. The present invention furthermore relates to DNA immuno-therapy vaccines comprising such plasmids as well as such pharmaceutical formulations and kits thereof. The present invention finally relates to the medicinal use of such products as well as methods for producing such plasmids.

The plasmids/DNA immuno-therapy vaccines herein have therapeutic potential in treatment of autoimmune diseases that are mainly T cell driven, such as e.g. type 1 diabetes (T1D).

In one aspect the present invention provides plasmid which encodes:
i. an insulin antigen;
ii. TGF-β; and
iii. IL-10.

### BRIEF DESCRIPTION OF DRAWINGS

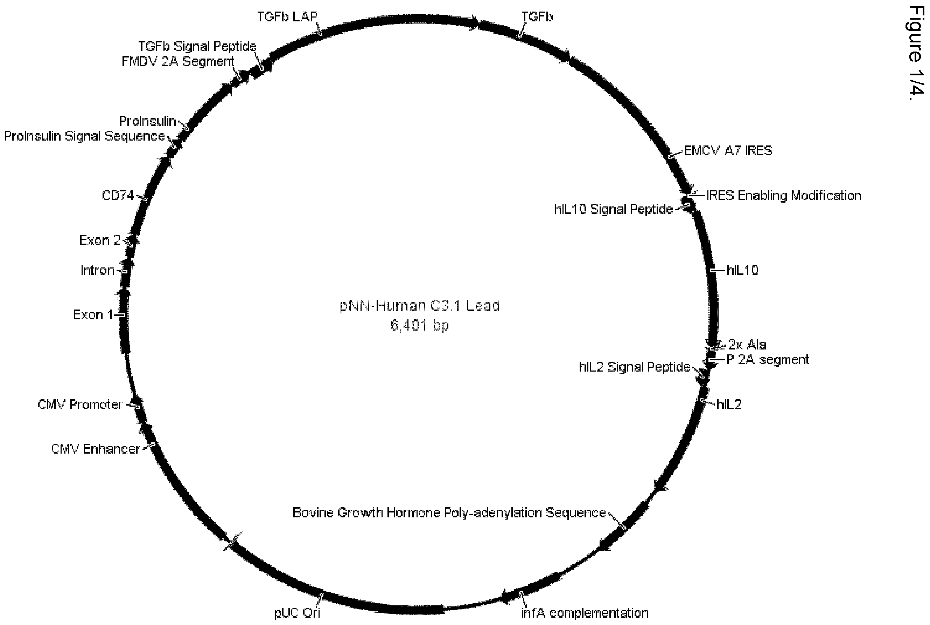

Figure 1. Circular plasmid map.

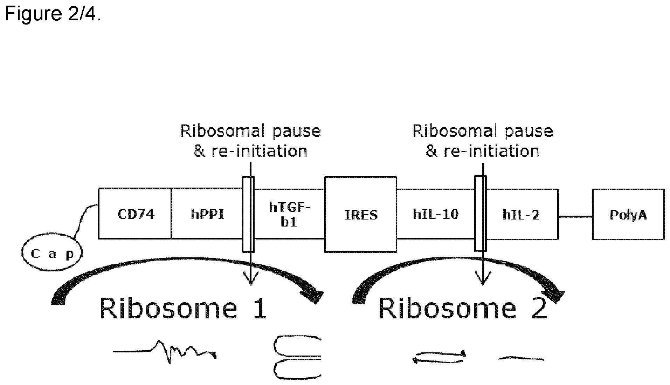

Figure 2. mRNA and translated protein map for the vector products of the plasmid from Fig 1.
Figure 3. Plasmid shear stability on three injection passages via a G30 needle.
Figure 4. Confirmation of plasmid retention phenotype by growth at 30°C (passages 1-50 using 17 hours incubation and passages 51-100 using 22 hours incubation).

### DESCRIPTION

The inventor of the present invention has herein provided a single vector which drives expression of multiple secreted cytokines, as well as an cellularly retained antigen, from a single promoter/multi-cistronic mRNA.

DNA immuno-therapy vaccination with a single vector encoding all components of the therapy in a single cell is highly preferred over immuno-therapy vaccination with a mixture of separate vectors/plasmids each driving expression of single components, as random transfection of cells with different vectors does not guarantee expression of all components, or even any specific ratio of components, from a given, specific transfected cell.

Transfection of a single multi-cistronic plasmid/vector results in a specifically engineered local environment/micro-environment around the transfected cell. In this way, combinations of immuno-modulators can be added to the antigen such that they potentiate the desired immunologic effect of single T cells without the requirement of high systemic immuno-modulator doses that could otherwise cause adverse events and broad immunosuppression.

This local restriction of immuno-modulator production of host cells transfected with the DNA immuno-therapy vaccine allows for the safe use of highly potent cytokine hormones, which are synergistic for modification of T cell responses, but cannot be dosed either frequently enough for effect, and/or titrated to give the desired response, without unacceptable adverse events.

For example, Interleukin-10 (IL-10) and Transforming Growth Factor-beta1 (TGF-β1) are both known to be able to induce regulatory T cells (Tregs) from naive CD4⁺ T cells. However, the IL-10/TGF-β1 combination provides a synergistic effect (15 to 20 fold more efficacious) in inducing Tregs than either of the two cytokines alone (US6083919 A) and this combination furthermore results in immune tolerance in a broader population of target cells than either cytokine alone (Zeller JC, Panoskaltsis-Mortari A, Murphy WJ, et. al. 1999 J Immunol. 163(7):3684-91).

Additionally, Interleukin-2 (IL-2) is known to both expand and stabilize Tregs but may on the other hand also contribute to inflammatory responses. The combination of IL-2 and IL-10, however, results in suppressive Tregs rather than inflammatory stimulation. As circulating T cells encounter cells that are transfected with the DNA immuno-therapy vaccine herein, they are temporarily exposed to sub-optimal concentrations of IL-10 and IL-2. The circulating T cells are slightly biased toward tolerance, and if they are also reactive toward the co-expressed antigen (e.g. insulin) they will bind to the transfected cell and thus receive a longer duration of immuno-modulator exposure and in addition they will also receive another signal that programs/re-educates them for suppressive effects. In this way, those T cells which are responsive to the encoded antigen are selectively re-educated to a suppressive phenotype when they encounter the transfected cell.

The plasmids/vectors/DNA immuno-therapy vaccines herein are thus designed for induction of antigen specific Tregs accumulating at sites of autoimmunity to dampen disease (e.g. the pancreas in T1D) rather than to directly impact disease through the expressed cytokine hormones.

In addition to an antigen (insulin in the example of T1D), the vector/operon/plasmid herein encodes at least two cytokines (e.g. TGF-β1 and IL-10) which together synergistically suppress antigen presenting cells, as well as T cell function, and drive induction of Tregs. This effect is enhanced if it also occurs in combination with effective exposure to antigen.

In one embodiment, TGF-β1 is in a constitutively active form that does not require processing or an inflammatory environment for function. While Tregs can be produced from naïve T cells via exposure to antigen and TGF-β1, Tregs are, however, "plastic" meaning that they can de-differentiate and convert into Th17 effector cells and then cause more, not less, autoimmune destruction. The combination of IL-10 with TGF-β1, in addition to being a more potent immuno-modulator, suppresses the environment that would produce pathogenic Th17 cells rather than Tregs.

In one embodiment, the multi-cistronic vector herein also encodes IL-2 in addition to antigen, TGF-β1, IL-10. IL-2 expands Treg numbers and stabilizes their phenotype (prevents Treg cells from de-differentiation into effector T cells) and thus increases their functional lifespan in inflamed target tissues.

These three cytokines (TGF-β1, IL-10, and IL-2), in combination with antigen, thus have well-known synergistic effects for inducing tolerance by the following mechanisms: (i) significantly enhanced generation of antigen-specific suppressive Tregs, (ii) longer Treg lifespan, and (iii) greater efficacy per individual Treg cell in suppressing inflammation/auto-reactivity. However, the required concentrations of systemically infused purified cytokine would have a number of serious, or maybe even lethal, side effects, such as: (i) lethal fibrosis from excess TGF-β1, (ii) flu-like symptoms, (iii) capillary leak syndrome from excess IL-2, (iv) broad immunosuppression leading to chronic infections, (v) enhanced tumor development as well as (vi) anemia from excess IL-10.

By co-expressing these cytokines from the same vector/plasmid, and therefore by the same cell presenting the antigen to the immune system, the vector achieves the desired local environment for tolerance induction without systemic action and corresponding side-effects that would otherwise result from high-dose purified cytokine administration.

Injection of "naked"/"bare" plasmid/vector DNA (vector and buffer alone) has a very low uptake and transfection rate - fewer than one in about 100,000 plasmid molecules transfects a cell, while the rest are degraded and thus without any biological effect. This extremely low inefficiency of transfection provides a safety mechanism for distributing and limiting the transfected cells.

Administration of systemically active quantities of any of these cytokines, either by administration of mature proteins or by high-efficiency viral vector transduction, would be difficult, if not impossible, to titrate for a safe and effective dose. Limiting the total exposure to a very small systemic dose distributed in a few high expressing micro-environments leads to a highly advantageous safety and efficacy profile.

The combination of antigen and these three cytokines herein produces an efficient protection from T1D development and even appears to be able to stably reverse disease progression. Due to the low transfection efficiency of the bare DNA plasmid/vector injection, very few cells produce these recombinant proteins and there is thus no detectable change in serum cytokine levels from plasmid/vector encoded cytokines - and therefore no detectable immune stimulation or immuno-suppression toward any other antigens than the antigen encoded by the plasmid/vector (pre-proinsulin). This results in a desirable safety profile.

Normally, DNA vaccines perform poorly in connection with subcutaneous (s.c.) injection and are therefore typically administered using intramuscular injection (often with electroporation) or alternatively using intradermal jet injection requiring a cumbersome device as well as significant maintenance and calibration. As most side effect issues with intramuscular injection are adjuvant-related (injection site irritation) they are therefore not a concern for the bare DNA immuno-therapy vaccine format herein. Additionally, the volumes injected are usually relatively small and therefore do not cause significant muscle distension and pain. In one embodiment, the volumes injected are 1 ml or less. In another embodiment, the volumes injected are approximately 0.6 or 0.5 ml. Regardless, the multi cytokine plasmid/vector provided herein unexpectedly appears to provide protection from T1D even when administered through the s.c. route, thereby allowing multiple potential dosing formats for patients.

In addition to providing local synergy, by encoding all three or four of the translated products by a single plasmid/vector and a single promoter, the regulatory burden and drug substance release criteria are furthermore simplified with the provision of the multi-cistronic plasmid herein.

In contrast, if each of the protein products is produced from a separate plasmid, then the synergistic value of co-expression from the same transfected cell would then potentially be lost or reduced as each plasmid/vector transfection would be an independent event, likely targeting different cells. If the three to four recombinant proteins are produced from two, three, or four individual plasmids/vectors, any synergistic effects in the local environment of the transfected cell are potentially lost; in addition, several individual clinical trials would thus be necessary (one for each plasmid and each combination). Producing all proteins from a single plasmid/vector and single mRNA relieves the requirements to test multiple individual molecules and determining ideal co-packaging ratios inherent to a multiple plasmid/vector format.

Any vector formats suitable for the present invention can be used herein, such as plasmids (replicating or passive), mini-circles, linear vectors (MiLVs), viral vectors (both integrating [e.g. lentiviral] and non-integrating [e.g. adenoviral]), cosmids, bacterial artificial chromosomes (BACs), human artificial chromosomes (HACs), etc.

Furthermore, any permissible transfection enhancement method can be used herein: e.g. electoporation, sonoporation (ultrasound enhancement, with or without microbubble contrast enhancement), lipid/polymer aggregates, hydrodynamics (pressure via high injection volume), bio-ballistics / gene-gun (deposition through skin via compressed gas), etc.

In one embodiment, non-replicating episomal plasmid DNA is used herein due to: i) multiple copies of mRNA derived from a single plasmid transfection, and ii) extended stability and function of plasmid nucleic acids over mRNA and other DNA vector formats. Thus, while both mRNA and DNA-based expression systems can provide intracellular delivery and co-localization, plasmid based systems provide greater control and persistence of dosing.

In one embodiment, plasmids/vectors encode four proteins:
i) an antigen,
ii) TGF beta 1 (TGF-β1),
iii) Interleukin-10, and
iv) Interleukin-2.

In one embodiment, the antigen is an endosomally-targeted T1D relevant antigen, such as insulin or GAD65. Endosomal targeting can be done via e.g. a li/CD74 fusion, a LIMPII/SCARB fusion, or a transferrin receptor fusion.

In one embodiment, TGF-β1 is in an activated form.

Expression of four proteins from one plasmid/vector is possible e.g. if the desired sequences are separated either with A) separate promoters, B) an IRES (Internal Ribosome Entry Site) sequences which recruit a new ribosome to translate each segment, or C) viral 2A sequences (e.g. FMDV 2A or TaV 2A sequences) which are translated and induce a ribosomal pause/skip which results in production of separate polypeptides from a single open reading frame. However, in practice, each of these strategies is complex and difficult to enable.

Expression of four independent proteins from a single plasmid/vector is most easily achieved by having a separate promoter for each gene. However, this format has significant disadvantages in that it A) results in a very large, unstable, and hard to produce plasmid due to the excess length of multiple promoters, B) results in unpredictable behaviour of the translated proteins relative to each other (they are no longer produced in fixed ratios to each other), C) each promoter may be independently silenced, leading to selective expression of some genes but not others required for full efficacy, and D) a lack of regulatory simplicity. In contrast, IRES elements and 2A sequences operate on the mRNA and translation levels and reproducibly co-express fixed ratios of each protein from a single promoter.

Each of the four classes of IRES elements has different co-factor requirements for function as well as different sequence requirements for the downstream gene to be translated. For instance, the EMCV (EndoMyoCarditis Virus) IRES is a 630 base pair type 1 IRES which utilizes all eukaryotic translation initiation factors while the CrPv (Cricket Paralysis virus) IRES is a 200 base pair type 4 IRES that has no required cofactors but utilizes a non-standard initiation codon.

When IRES elements from different classes are utilized, they interfere with each other such that each type of IRES element can only be used once in each plasmid, and when used together, different types of IRES elements attenuate each other (decrease in efficacy) in ways that are difficult to predict.

Furthermore, shuffling the gene / IRES combinations result in unpredictable ratios of translated products as the interactions of the genes with the IRES elements are not static but context dependant on the flanking nucleotide sequences. In addition, IRES elements impose restrictions on the first few amino acid positions at or immediately following initiation. For instance, the CrPv IRES requires that the first amino acid be an alanine rather than the standard methionine and the EMCV IRES cannot tolerate P, W, C, R, or K amino acids within the first three codons. In one embodiment, to accommodate the N-terminal amino acid restrictions imposed by the EMCV IRES, the DNA vaccine contains a three Alanine extension to the N-terminal of the IL-10 gene.

In addition, each IRES element comprises a substantial number of base pairs, ranging from 230 bp to over 700 bp; the inclusion of multiple IRES elements thus increases the size and complexity of plasmids/vectors to the extent that many become unstable and difficult to be industrially produced due to spontaneous deletions and recombinations. Further, due to the high degree of secondary structure that IRES elements impart on the transcribed mRNAs that contain them, they increase the probability of activating pathogen recognition receptors (Dabo S, Meurs EF. 2012 Viruses 4(11):2598-635.) in the transfected cell and producing stimulatory effects counter to the tolerance induction that is intended.

2A sequences, unlike IRES elements, do not interact with each other and therefore provide stable and consistent performance. However, they are translated themselves and therefore affect the folding, function, and stability of the final translated protein products. All 2A sequences result in a significant C-terminal fusion (19-22 aa) onto the 5' end of the sequences to be separated and also begin the 3' sequence with a proline. Some proteins are permissive of these modifications and some are not, leading to practical restrictions to the use of 2A sequences. For instance, the Interleukin-10 product is permissive of the 2A tail but both Interleukin-2 and TGF-β1 mis-fold and lose function if expressed upstream of a 2A tag. Therefore, while it is possible to express several independent proteins separated by 2A sequences, two of the four proteins herein cannot terminate in 2A tags and therefore other strategies must be utilized.

As each type of 2A amino acid sequence modifies ribosomal function during protein translation, it will have different efficiencies in the two core properties of the 2A family namely (i) separation of the juxtaposed gene products and (ii) processivity (re-initiation) into the second gene product. Different 2A sequences have different efficiencies at generating the ribosomal pause that breaks the peptide backbone (resulting in the two separate proteins) as well as different efficiencies at re-initiating the peptide synthesis of the second gene product.

The ability of the 2A sequences to separate protein products and re-initiate protein translation are dependent on the 2A amino acid sequence (Donnelly ML, Hughes LE, Luke G, et. al. 2001 J Gen Virol. 82(Pt 5):1027-41). Small variations in 2A amino acid sequences result in significantly different mixes of separated and fused flanking gene products, ranging from under 5% (>95% fused) to completely separated (0% fused or 100% separated).

Furthermore, the inventor has herein discovered that adjacent amino acid sequences encoding the two flanking protein products also affect efficiency of re-initiation and separation of the 2A sequences, leading to significant deviations from reported results. Re-initiation efficiency thus varies depending on the type of 2A amino acid sequence used as well as the environment provided by the adjacent amino acid sequences, and thus the ratio of the pre-2A gene product and separation of the proteins will be determined by both the 2A amino acid sequence used and its context.

In one embodiment, "FMDV 2A" is inserted between the antigen encoding sequence and the TGF-β1 encoding sequence herein; resulting in 100% separation, as well as a 1:1 ratio, of the protein products.

In another embodiment, "TaV 2A" may be inserted between the IL-10 encoding sequence and the IL-2 encoding sequence herein, resulting in about 50% separate products as well as a 10 to 6 ratio of the protein products. Each transfected cell thus delivers a relatively low dose of Interleukin-2, that is incapable of stimulating effector T cells, and a higher dose of Interleukin-10 to bias the T cells toward the Treg phenotype. Since the production of fused IL-10/IL-2 is disadvantageous, attempts to engineer increased cleavage efficiency of the TaV 2A segment were made. An attempt to precede the 2A segment with an "insulator segment", which is an element that extends the translated region upstream of the TaV 2A to reduce upstream sequence impact on the 2A element, did not improve separation. In a different attempt to solve the fusion problem, an upstream uncoupler segment with a translated protein sequence of GSG was added; however, this approach resulted only in an incremental improvement of cleavage efficiency.

As such, cytokine fusions, resulting from separation of the IL-10 and IL-2 encoding genes by a TAV 2A, are likely to be immunogenic,

In a further embodiment, the vector/plasmid herein has a "P 2A" segment. Separation of the IL-10 and IL-2 encoding genes by a P2A results in complete or near-complete separation of the protein products as well as a ratio of at least twice as much (or maybe even up to four or five times as much) IL-10 compared to IL-2.

In order to address the shortcomings of the IRES-only and 2A-only systems described above, the four cDNA sequences herein (antigen, TGF-β1, IL-10, IL-2) are arranged in pairs before and after a single IRES. Each pair is further separated by a 2A sequence, which induces ribosomal skipping and production of independent proteins from each sequence in the polyprotein pair. As TGF-β1 and IL-2 may not be on the N-terminal side of the fusion, one of them must terminate at the central IRES site and the other one must end the translated portion of the mRNA sequence.

The chronology/sequence of expressed proteins and IRES/2A elements herein may therefore be as follows: (i) Antigen, (ii) FMDV 2A, (iii) TGF beta 1, (iv) IRES, (v) IL-10, (vi) P 2A, and (vii) IL-2. As a consequence, all four proteins can be independently expressed from a single operon/gene segment in a stable and predictable fashion. As each of these proteins is expressed from a single mRNA, the ratios of each product are fixed - it is not possible to generate an excess of IL-2 over IL-10 for instance.

Besides using a combination of IRES and 2A elements for separation of encoded genes, an alternative solution herein could be use of a bidirectional promoter to generate 2 mRNAs - these mRNAs would each encode a pair of proteins rather than all four in one mRNA molecule. Equivalent arrangements may therefore be constructed utilizing pairs of expression cassettes appropriately arranged around a bidirectional mammalian promoter and utilizing separating 2A sequences and/or IRES elements. This approach is, however, associated with disadvantages, primarily due to the large size of bi-directional promoters but also a potential increased regulatory burden having separate mRNA elements included in one medicinal product. Preferred embodiments herein therefore utilize a single promoter and a combination of IRES and 2A elements rather than a bidirectional promoter.

In theory, some 2A sequences could be replaced with intracellular endogenous protease sensitive sequences. However, the inventor has herein discovered that such proteases are associated with significant disadvantages (e.g. lack of reported function resulting in secretion of fused protein products).

In order for the antigen to be processed and presented to the immune system within the local environment of the plasmid encoded cytokine hormones, the antigen must be retained within the transfected cell. In the case of type 1 diabetes, production of active insulin would potentially lead to undesirable lowering of blood glucose if it were to be secreted or otherwise released from the transfected cell.

In order to avoid antigen secretion, any secretion signals can be removed from the antigen encoding sequence, e.g. remove secretion signal coding sequence from the nucleic acid sequence encoding pre-proinsulin, thus proinsulin rather than pre-proinsulin would be generated, thus allowing the antigen to accumulate inside the transfected cell. While this translated antigen product (e.g. insulin) would not be actively secreted, it could be released during lysis due to necrosis resulting from attack by CD8⁺ T cells. Additionally, the signal sequence of insulin is a region known to contain disease-relevant epitopes (potentially inducing auto-immunity) and the inclusion of the signal sequence therefore ensures broader tolerance induction and a higher probability of reducing disease.

Additionally, cytoplasmic retention of antigen only allows for processing via the proteasome and presentation via the MHC class I pathway, which detects intracellular pathogens via CD8⁺ T cells. As CD4⁺ T cells are significant contributors to pro-inflammatory cytokines and most, if not all, autoimmunity suppressing Tregs are CD4⁺, broadening the presentation of antigen to include MHC class II, which is recognized by CD4⁺ T cells, may be advantageous.

MHC class II processing and CD4+ T cell stimulation normally do not include intracellular antigen, as access to this pathway is via endocytosis of extra cellular antigen. Normally, protein products produced within a transfected cell are only presented via the default intracellular / proteasomal processing pathway and MHC class I, resulting in CD8+ T cell effects but not CD4+ T cell effects. In order to target both CD4+ and CD8+ T cells for immunomodulation the preferred embodiment also includes factors leading to MHC class II presentation.

In principle, to induce MHC class II presentation, the antigen can be fused to any partner that directs the fusion to an endosomal compartment, but there are functional differences in activity and exposure. Transferrin receptor, also known as iron transporting protein receptor, fusions cycle from the plasma membrane/extracellular space to the endosome and therefore may also expose other immune cells to whole antigen, such as B cells, macrophages, etc.. Limpll/SCARB fusions target directly to the endosome, but preferentially to the early endosome and sometimes result in over processing and total destruction of the antigen. li (CD74) fusions, utilizing the same chaperone signal that MHC class II uses for late endosome localization, deliver the antigen and MHC class II to the same vesicles at the same developmental stage and maximize the likelihood of effectively presenting antigen in the context of MHC class II. Additionally, even with endosomal sorting from li fusions, the preproinsulin secretion sequence must be rendered inactive or the antigen would also be secreted and lost prior to processing.

Blockade of insulin antigen secretion has alternatively been accomplished herein by mutating two amino acids required for secretion tag removal by the SRP (Signal Recognition Particle) on the Rough Endoplasmic Reticulum. Ala (A) to Glu (E) mutations completely abolish pre-proinsulin maturation and secretion, while maintaining the required epitope structure of the antigen for best tolerance induction.

In one embodiment, the plasmid DNA vaccine is used herein. The plasmid is grown/replicated for example in *E. coli,* and isolated/purified from the media, and subsequently formulated in liquid formulations e.g. water, saline, PBS liquid formulations, or as a lyophilized powder for intrademal jet injection, intranasal administration, or inhalation. In one embodiment, the plasmid herein is formulated in an aqueous pharmaceutical formulation optionally comprising stabilisers. Any suitable microbial system may be utilized for plasmid production.

Stabilizers in the formulation include, but are not limited to, chelating agents, such as EDTA, EGTA, or DPTA for scavenging Mg⁺⁺ and Fe⁺⁺⁺ which may otherwise be involved in degradation of DNA, and/or citrate, which protects the plasmid from non-specific degradation effects. In one embodiment, the plasmid herein may be formulated in isotonic PBS or alternatively TRIS + citrate + EDTA. Such plasmids have the advantages of being stable, easy to produce and being safe and convenient in use.

In another embodiment, delivery agents, such as virus, lipids, liposomes, co-packaging etc., could be added in connection with the present invention. However, the use of delivery agents herein may have potential problems with immunity, viral integration, etc.

### Definitions

Antigen: the DNA immuno-therapy vaccine herein encodes an antigen. The antigen herein can be any type of immunogenic disease-associated protein or fragment thereof that can be recognized by the T cell component of the immune system. For example, in the case of type 1 diabetes treatment or prevention, an insulin antigen may be used. In one example, the insulin antigen is the InsB 9-23 immunodominant peptide. For multiple sclerosis DNA immuno-therapy vaccines herein, a myelin basic protein (MBP), myelin oligodendrocyte protein (MOG), and/or proteolipid protein (PLP) antigen may be used as antigen. Similar protein antigen encoding sequences for representative antigens from alopecia, polymyositis/dermatomyositis, celiac sprue, and protein allergens (e.g. peanut protein ara h 2) are also examples of antigens suitable for use in the DNA immuno-therapy vaccines herein.

Antigen targeting: In one embodiment, antigen herein is endosomally targeted. Antigens herein include whole protein, secretion-deficient pre-proteins, or a functional or immuno-dominant peptide fragment thereof.

For example, insulin antigen herein is an antigen for use in immune modulatory therapy and not a glucose lowering agent. It should therefore not be fully processed/matured or secreted in order to make sure that it is presented on MHC molecules to circulatory T cells. The DNA immuno-therapy vaccine herein does therefore not result in increased insulin levels in the blood but rather results in an increased presentation of antigens to the immune system, in particular the T cells.

Therefore, insulin antigen herein can be small immuno-dominant peptide encoding fragments (e.g. insulin B chain 9-23 peptide, including shifted register peptides displaying equivalent T cell epitopes), whole proinsulin, which lacks the required secretion sequence but otherwise intact, or pre-proinsulin muteins that contain the secretion sequence but are modified to prevent secretory function.

Examples of Insulin antigens herein include:
Mouse proinsulin (SEQ ID NO 1): 
FVNQHLCGSHLVEALYLVCGERGFFYTPKTRREAEDLQVGQVELGGGPGAGSLQPLALEG
SLQKRGIVEQCCTSICSLYQLENYCN
Human proinsulin (SEQ ID NO 2): 
FVNQHLCGSHLVEALYLVCGERGFFYTPKTRREAEDLQVGQVELGGGPGAGSLQ
PLALEGSLQKRGIVEQCCTSICSLYQLENYCN
Modified mouse pre-proinsulin that is not secreted (substitutions in relation to wt pre-proinsulin shown with bold and underline (SEQ ID NO 3)): 
MALWMRLLPLLALLALWGPDPEQEFVNQHLCGSHLVEALYLVCGERGFFYTPKTRREAEDL
QVGQVELGGGPGAGSLQPLALEGSLQKRGIVEQCCTSICSLYQLENYCN
Modified Human pre-proinsulin that is not secreted (substitutions in relation to wt pre-proinsulin shown with bold and underline (SEQ ID NO 4)): 
MALWMRLLPLLALLALWGPDPEQEFVNQHLCGSHLVEALYLVCGERGFFYTPKTR
REAEDLQVGQVELGGGPGAGSLQPLALEGSLQKRGIVEQCCTSICSLYQLENYCN
*Mouse wt* pre-proinsulin (SEQ ID NO 5): 
ALWMRLLPLLALLALWGPDPAQAFVNQHLCGSHLVEALYLVCGERGFFYTPKTRREAEDLQ
VGQVELGGGPGAGSLQPLALEGSLQKRGIVEQCCTSICSLYQLENYCN
*Human wt* pre-proinsulin (SEQ ID NO 6): 
MALWMRLLPLLALLALWGPDPAQAFVNQHLCGSHLVEALYLVCGERGFFYTPKTR
REAEDLQVGQVELGGGPGAGSLQPLALEGSLQKRGIVEQCCTSICSLYQLENYCN
Insulin peptide "InsB 9-23" identical between mouse and human:
   SHLVEALYLVCGERG (SEQ ID NO 7)
Modified InsB 9-23 (substitutions in relation to wt InsB 9-23 shown with bold and underline (SEQ ID NO 8) and (SEQ ID NO 27)):
   SHLVEALYLVCGE**E**G and SHLVEALYLVCG**GE**G

Insulin antigens herein may thus accumulate in the cytosol of the transfected host cell and can thus be presented via MHC class I, or be released upon cytolysis.

Endosomal targeting resulting in MHC class II presentation may be accomplished herein via fusion of the antigen sequence with leader sequences which form transmembrane segments with cytoplasmic "YXXØ" sequences, in which Y is tyrosine, X is any amino acid, and Ø is a bulky hydrophobic amino acid such as tryptophan or isoleucine, "[DE]XXXL[LI]" where D and E are aspartic or glutamic acid respectively, while L and I are leucine and isoleucine respectively, or "DXXLL" endosomal/lysosomal sorting signals, which are underlined in the following exemplary sequences. Protein domains that include these signals therefore target or cycle to the endosome/lysosome include: transferrin receptor, Limpll, or CD74, also known as Invariant chain, MHC II chaperone, or li, or any similar domain.

Examples of endosomal targeting domains herein include, but are not limited to:
Mouse CD74 / Invariant chain (li) endosomal targeting domain (SEQ ID NO 9): 
MDDQRDLISNHEQLPILGNRPREPERCSRGALYTGVSVLVALLLAGQATTAYFLYQ
QQGRLDKLTITSQNLQLESLRMKLP
Human CD74 / Invariant chain (li) endosomal targeting domain (SEQ ID NO 10):

MHRRRSRSCREDQKPVMDDQRDLISNNEQLPMLGRRPGAPESKCSRGALYTGFS
ILVTLLLAGQATTAYFLYQQQGRLDKLTITSQNLQLESLRMKLP

Type 1 diabetes: Type 1 diabetes (T1D) is considered to be a chronic autoimmune disease, where auto-aggressive T cells infiltrate the islets of Langerhans in the pancreas and play an important role by specifically destroying the insulin-producing beta-cell population. Once a significant number of islet cells are destroyed, reduced amounts of insulin, or no insulin at all, will result in insulin deficiency and hyper-glycemia in the patient. T1D patients are thus unable to produce enough insulin and need regular injections of the hormone are needed throughout life. Some Type 1 Diabetes patients are diagnosed with "type 1.5 Diabetes", "latent autoimmune diabetes"/LADA, "double diabetes" etc., which are diabetes diseases carrying symptoms of both Type 1 Diabetes and Type 2 Diabetes - all diabetes diseases carrying trains of both Type 1 and Type 2 Diabetes are thus also contained in the term "Type 1 Diabetes" herein.

Tolerogenic DNA vaccine: DNA-based immuno-therapy vaccines/vectors/plasmids herein are designed to switch off or down-regulate the part of the immune system responsible for destroying normal healthy "self" cells and thus prevent or ameliorate T cell-based autoimmunity.

The term "DNA immuno-therapy vaccine" as used herein is intended to mean a compound or composition comprising a DNA molecule and which is administered to a subject in order to reduce the risk of said subject developing one or more diseases.

In some embodiments, DNA based immuno-therapy vaccines herein are plasmids/vectors encoding particular antigens. Following vaccination, these plasmids are taken up by, in other words, transfected into antigen presenting cells in the host's body. The "transfected" host cells then produce the antigen and present small fragments of the antigen to the immune system, in particular the T cells. This approach leads to a modification of specific T cell responses to the encoded antigen as well as minimal modification to immune responses to other (non-encoded or "irrelevant") antigens. Only a very few host cells are typically transformed with the DNA vaccine plasmid/vector herein, meaning that likely fewer than one out of hundred thousand, one out of five hundred thousand, or even fewer than one out of a million plasmid/vector molecules eventually enter a host cell. DNA vaccines herein thus represent a very mild and specific approach for modulating immune responses to antigens such as insulin in T1D patients or patients at risk of developing T1D.

Plasmid: A plasmid is a small DNA molecule that is most commonly found in bacteria as small, circular, double-stranded DNA molecules. Artificial plasmids are widely used as vectors in molecular cloning, serving to drive the replication of recombinant DNA sequences within host organisms. Plasmids can be engineered to be suitable for use as immuno-therapy DNA vaccines. Plasmids are considered replicons, a unit of DNA capable of replicating autonomously within a suitable host. Plasmids can be transmitted from one bacterium to another bacterium, which could be of the same or different bacterial species via three main mechanisms: transformation, transduction, and conjugation. DNA vaccine plasmids can be taken up by a host cell by passive transformation - usually at a relatively low rate. The plasmids herein replicate efficiently - but do not drive protein expression - in bacteria. The plasmids herein furthermore drive protein expression - but not replication of plasmid - in humans and other mammals, e.g. mice. In one embodiment, a pVAX1 vector (Invitrogen/LifeTechnologies) is used as a scaffold herein for inserting the elements that are part of the present invention. Other suitable vector scaffolds herein include any vector backbone containing a eukaryotic promoter element, a prokaryotic high copy origin of replication, and a selection system for plasmid maintenance.

Selection gene and selection system: In one aspect, DNA immuno-therapy vaccines herein comprise a selection gene/selection marker for manufacturing purposes. The selectable marker herein is e.g. a gene that confers resistance to a cell toxin - e.g. an antibiotic such as ampicillin, kanamycin, chloramphenicol, streptomycin, etc.

Other types of suitable selection systems herein include e.g. conditional lethal silencing systems (e.g. CcdA/CcdB or ParD/ParE Hok/Sok type systems), or sequences that complements a genomic defect in the production cell strain and thus permits growth of an otherwise inviable host (e.g. dapD⁻ or pyrF⁻ auxotrophic complementation, infA⁻ translation initiation complementation, etc.)

Production cells harbouring the plasmid/DNA vaccine, which includes the selection marker, will survive when exposed to the toxin/antibiotic/condition, while those that have failed to take up plasmid sequences will die. As such, in one embodiment, DNA vaccines herein comprises the nucleic acid sequence encoding a selection marker in order to provide for higher yield/purity and more efficient production/replication in production cells, such as *E. coli.*

While antibiotic selection is a common laboratory strategy there may be advantages associated with antibiotic-free selection systems - e.g. in relation to more efficient regulatory processes. While vectors which do not contain a selection mechanism such as minicircles, synthetic linear vectors, etc., can also be used herein, these implementations are associated with certain drawbacks in production, in particular due to increased production and quality control costs.

Examples of complementation ("rescue") strategies are known in the prior art, however these strategies suffer from various disadvantages.

Metabolic complementation systems such as dapD [lysine biosynthesis] or pyrF [uridine biosynthesis] systems, often result in "cross-feeding" during high density *E.coli* production, where a plasmid-containing bacterium will produce and secrete an excess of the required compound and thereby "relaxing" the selection pressure for neighbouring bacteria without the plasmid.

Another example of a suitable selection system herein are plasmids encoding essential proteins, such as infA, encoding IF1 / Initiation Factor 1 which is required for protein synthesis. In this selection system, cross-feeding does not occur because the infA protein is not secreted. However, it is not possible to further modify the plasmid or expand plasmid-deficient cells as there is no way to exogenously complement the required protein/infA (J Bacteriol. 1994 Jan;176(1):198-205 and J Biotechnol. 2004 Jul 1;111(1):17-30).

In order to circumvent the disadvantages associated with the infA selection system, an alternative selection system has been provided herein with a temperature-sensitive translation switch (or "thermosensor") from the invasion protein gene prfA of *L*. *monocytogenes* (Cell. 2002 Sep 6;110(5):551-61). By placing the hairpin forming portion of an RNA "thermosensor" sequence upstream of the *E.coli* genomic copies of infA via standard recombination technology, expression thereof becomes regulated via control of the fermentation temperature, enabling slow growth of plasmid free cells at 37°C, and rapid cell death at temperatures <30°C. Transformation of the engineered thermo sensitive *E.coli* production strain with plasmids expressing *wt* infA thus allow full normal growth rates at all temperatures, allowing for plasmid-free expansion at 37°C as well as stringent selection for plasmid at 30°C. Additionally, this system generates no selective pressure for *wt E.coli* to retain the plasmid and it is thus lost within 8 hours in culture - ensuring no environmental persistence of the therapeutic plasmid.
*wt* E.coli infA nucleotide sequence (SEQ ID NO 11): 
ATGGCCAAAGAAGACAATATTGAAATGCAAGGTACCGTTCTTGAAACGTTGCCT
AATACCATGTTCCGCGTAGAGTTAGAAAACGGTCACGTGGTTACTGCACACATCTCCGG
TAAAATGCGCAAAAACTACATCCGCATCCTGACGGGCGACAAAGTGACTGTTGAACTGA
CCCCGTACGACCTGAGCAAAGGCCGCATTGTCTTCCGTAGTCGCTGA
*wt* E.coli IF1 protein sequence resulting from translation of the infA gene (initial methionine/M not included in prfA fusion - (SEQ ID NO 12)):

*E. coli* production cell lines used herein for production of DNA immuno-therapy vaccine plasmids may thus harbour the following thermo sensitive prfA nucleotide sequence:
wt *L.monocytogenes* prfA ("thermo sensor hairpin") nucleotide sequence (Shine Dalgarno underlined, ATG start bolded - (SEQ ID NO 13)): 
TGTAAAAACATCATTTAGCGTGACTTTCTTTCAACAGCTAACAATTGTTGTTAC
TGCCTAATGTTTTTAGGGTATTTTAAAAAAGGGCGATAAAAAACGATTGGGGGATGAGAA
ATGAACGCTCAA
*wt L.monocytogenes* prfA protein sequence (fused upstream of E.coli IF1 - resulting from translation of SEQ ID NO 13):
   MNAQ

Origin of replication ("Ori"): The origin of replication, also called the replication origin, is a particular sequence in a genome at which replication of the DNA strand is initiated. In one embodiment, origin of replication sites herein includes the "pUC Ori" which allows replication in the bacterial *E. coli* production cell line - but not in the mammalian host cells, i.e., cells from the body of the vaccinated subject/person/patient. Other suitable bacterial replication origins herein include but are not limited to: R6K, pBR322, ColE1, pMB1, 15A, pSC101, etc. In one aspect, the origin of replication herein is a high copy version which yields a high plasmid/biomass ratio for more efficient production. Vectors which do not contain an origin of replication, such as minicircles, synthetic linear vectors, etc., can also be used herein.

Promoter: A promoter is a region of DNA that initiates transcription of a particular gene. Promoters are located near the transcription start sites of genes, on the same strand and upstream on the DNA, which towards the 5' region of the sense strand. For the transcription to take place, the RNA polymerase must attach to the DNA near a gene. Promoters contain specific DNA sequences, such as response elements, that provide a secure initial binding site for RNA polymerase and for transcription factors that recruit RNA polymerase. Transcription factors have specific activator or repressor sequences that attach to specific promoters and regulate gene expression. Promoters thus represent critical elements that can work in concert with other regulatory regions, such as enhancers, silencers, boundary elements/insulators, to direct the level of transcription of a given gene. A classical promoter drives the production of a single messenger RNA (mRNA), whereas bidirectional promoters herein drive the production of two mRNAs immediately adjacent to the promoter, both upstream and downstream of the promoter.

In one embodiment, eukaryotic promoters are used herein. Eukaryotic promoters do not necessarily obey the one gene/one promoter rule, such as several viral promoters as well as promoters that exhibit broad expression (i.e. do not have narrow cell type specificities such as neuron-only expression). Examples of promoters herein that are capable of driving broad transcription of large multi-gene mRNA molecules include: the viral CMV immediate-early (IE) and SV40 promoters; endogenous EF1a, PGK1, Ubc, and beta actin promoters; and synthetic promoters such as the CAG hybrid promoter. Many other suitable mammalian promoters exist and more are being designed via synthetic biology efforts. Any promoter that results in the desired expression characteristics in human cells may be used in the DNA immuno-therapy vaccine plasmids herein.

Enhancers: Enhancers are DNA elements that increase the efficiency of promoters in producing mRNA transcripts. The enhancers herein may be matched (e.g. SV40 enhancer/CMV promoter) or unmatched. Any suitable enhancer/promoter combination for eukaryotic function can be used herein.

Eukaryotic translation start: The eukaryotic translation start sequence is usually referred to as the "Kozak" consensus sequence. The Kozak sequence on an mRNA molecule is recognized by the ribosome as the translational start site, from which a protein is encoded. The eukaryotic ribosome requires this sequence, or a variation thereof, to initiate protein translation. Kozak sequences are degenerate or variable and rarely match consensus sequences. In fact, consensus Kozak sequences are typically less efficient than wild type variants isolated from mammalian mRNAs. While weak Kozak sequences are regularly isolated from native mRNAs and likely play a role in translational control of low abundance proteins, DNA immuno-therapy vaccines herein preferably encode a medium or high efficiency Kozak sequence. Examples of useful Kozak sequences herein comprise the following nucleotide sequence: gccRccATGG (SEQ ID NO 14), where lower case bases are the most common nucleotides but may vary while upper case nucleotides are fixed (R is the IUPAC uncertainty code for A or G bases), and the ATG indicates the translational start site of Methionine codon at position +1.

Endosome sorting signal: An endosome is a membrane-bounded compartment inside eukaryotic cells. Some proteins can be transported to endosomes and therein be degraded into peptide fragments. The peptide fragments can bind to MHC molecules present in the endosome to form MHC/peptide complexes, which can subsequently be transported to the cell surface in order to be presented to circulating T cells, particularly CD4⁺ T cells.Sorting of proteins to endosomes is mediated by signals present within the cytosolic domains of the proteins. The endosomal signals are usually short linear amino acid sequences. Antigens herein are preferably targeted to the endosomes using an endosome sorting signal, such as e.g. YXXØ, [DE]XXXL[LI], or DXXLL endosomal/lysosomal sorting signals. Endosome sorting signals include various naturally occurring or synthetic endosomal sorting signals. Examples herein include the endosome sorting signals present on Cd74/invariant chain/li, Limpll/SCARB, or transferrin receptor. Any endosomal targeting domain which is pharmaceutically acceptable and provides the desired function may be utilized. Fusion of such endosomal targeting domains to the antigens directs them to the endosomal compartment upon translation for increased efficacy. Endosomal sorting of antigens confers processing and presentation to the immune system in MHC class II complexes, in addition to constitutive presentation in MHC class I complexes, for more complete and robust induction of tolerance and possible expansion of Tregs (which cannot be accomplished via MHC class I / antigen complexes). In one embodiment, tolerogenic DNA vaccines herein encode a fusion of the antigen with the CD74/invariant chain/li to drive endosomal targeting and presentation of the antigen via MHC class II.

Introns: Introns are non-coding sequences within an mRNA. It is known that some introns significantly increase translation and function of mRNA. Accordingly, the inclusion of intron sequences may also be used herein. Standard introns, such as beta-globin, or any intron obeying mammalian splicing conventions, such as MCM7, may be utilized. In one embodiment, DNA immuno-therapy vaccine vectors herein comprise sequences encoding one or more introns. In another embodiment, DNA immunotherapy vaccine vectors herein do not possess sequences encoding introns.

Ribosomal pause tag: In connection with the present invention, it may be an advantage to include one or more ribosomal pause tag sequence(-s) between the protein coding sequences in the DNA immuno-therapy vaccine vector/plasmid herein in order to separate protein products.

An example is the viral "FMDV 2A tag" (Foot-and-mouth disease virus 2A tag). The translated amino acid sequence of FMDV 2A is APVKQTLNFDLLKLAGDVESNPGP - (SEQ ID NO 15). FMDV 2A tag is capable of pausing and reinitiating the ribosome. The ratio of translated product before and after the FMDV 2A tag is close to 1:1 and the resulting protein products are normally completely separated. These types of ribosome tags have previously been used in connection with co-expression of two different domains, e.g. heavy chain and light chain in recombinant antibody production. However, the inventor of the present invention has made the surprising discovery that they are useful in connection with multi-cistronic DNA vaccines both for separation of flanking products and for control of the ratios of expressed proteins due to inherent efficiencies of ribosomal re-initiation. Sequence tags which favour a 1:1 ratio of translated products are herein preferably inserted between two protein encoding sequences that should preferably be produced in (or close to) a 1:1 ratio such as e.g. an insulin antigen and a potent cytokine such as e.g. TGF-β.

Another example of a ribosomal pause tag sequence herein is the viral sequence tag "TaV 2A" (*Thosea asigna virus* 2A - translated amino acid sequence of TaV 2A: RAEGRGSLLTCGDVEENPGP (SEQ ID NO 16). The ratio of translated product before/upstream and after/downstream of this tag is reported to be 50:1 (or close to). The inventor of the present invention has made the surprising discovery that while this type of tag can be used to control expression levels in cases where it is vital that one translated product absolutely dominates another, the separation of flanking cytokine products is less than 50% relative to the sequences disclosed in literature and the expression ratio is thus about 10:6. In connection with the present invention, a 2A type of ribosomal pause tag sequence should preferably result in different expression levels of two proteins encoded by the same vector/plasmid. Expression of small amounts of a pleiotropic cytokine (such as IL-2) relative to an anti-inflammatory cytokine, such as IL-10, is desirable herein and fused products are not desirable.

A further example of a ribosomal pause tag amino acid sequence herein is the viral sequence "P 2A" (*Porcine teschovirus-1 2A,* ATNFSLLKQAGDVEENPGP - (SEQ ID NO 17)). P 2A sequences function appropriately when inserted between IL-10 and IL-2 herein, resulting in near complete separation with an expression ratio of >5:1 between IL-10 and IL-2.

Alternatively, proteinase sensitive sequences, allowing for endogenous cleavage between plasmid expressed poly proteins, may be used herein. A furin sensitive sequence (recognizing RAKR motifs) or carboxypeptidase sensitive sequence (recognizing RRRR, RKRR, or RRKR motifs) may be used herein for separating protein products. However, the inventor of the present invention has made the surprising discovery that neither furin nor carboxypeptidase cleavage sequences result in separated products herein - thus leading to secretion of undesired IL-10/IL-2 fusion proteins.

TGF-b/β/β1 (Transforming growth factor beta/β1): TGF-β is a secreted protein that controls proliferation, cellular differentiation, and other functions in most cells. TGF-β is a very potent cytokine with significant effects on cell fate and phenotype in a context-dependent manner, e.g. depending upon the other cytokine signals received contemporaneously. Endogenous TGF-β is produced in a latent form associated with the outer membrane surface of the producing cell and requires activation (e.g. by inflammatory macrophages expressing CD36 and plasmin proteinase) for maturation and release of the active form. In one embodiment, TGF-β herein is a modified form that is constitutively active. This is achieved by replacing the cysteines at positions 223 and 225 with amino acids incapable of forming disulfide bridges. For example, serine or valine are used to replace cysteines at positions 223 and 225. This results in an active pro-protein structure that is released into the local microenvironment.
Human endogenous TGF-β1sequence - SEQ ID NO 18: 
MPPSGLRLLLLLLPLLWLLVLTPGRPAAGLSTCKTIDMELVKRKRIEAIRGQILSKLRLASPPS
QGEVPPGPLPEAVLALYNSTRDRVAGESAEPEPEPEADYYAKEVTRVLMVETHNEIYDKFK
QSTHSIYMFFNTSELREAVPEPVLLSRAELRLLRLKLKVEQHVELYQKYSNNSWRYLSNRLL
APSDSPEWLSFDVTGVVRQWLSRGGEIEGFRLSAHCSCDSRDNTLQVDINGFTTGRRGDL
ATIHGMNRPFLLLMATPLERAQHLQSSRHRRALDTNYCFSSTEKNCCVRQLYIDFRKDLGW
KWIHEPKGYHANFCLGPCPYIWSLDTQYSKVLALYNQHNPGASAAPCCVPQALEPLPIVYYV
GRKPKVEQLSNMIVRSCKCS.
Modified human TGF-β1 sequence that is constitutively active and secreted (substitutions in relation to wt TGF-β1 shown with bold and underline) - SEQ ID NO 19: 
MPPSGLRLLLLLLPLLWLLVLTPGRPAAGLSTCKTIDMELVKRKRIEAIRGQILSKLRLASPPS
QGEVPPGPLPEAVLALYNSTRDRVAGESAEPEPEPEADYYAKEVTRVLMVETHNEIYDKFK
QSTHSIYMFFNTSELREAVPEPVLLSRAELRLLRLKLKVEQHVELYQKYSNNSWRYLSNRLL
APSDSPEWLSFDVTGVVRQWLSRGGEIEGFRLSAHVSVDSRDNTLQVDINGFTTGRRGDL
ATIHGMNRPFLLLMATPLERAQHLQSSRHRRALDTNYCFSSTEKNCCVRQLYIDFRKDLGW
KWIHEPKGYHANFCLGPCPYIWSLDTQYSKVLALYNQHNPGASAAPCCVPQALEPLPIVYYV
GRKPKVEQLSNMIVRSCKCS.
Another modified human TGF-β1 sequence that may be used is SEQ ID NO 25:

MPPSGLRLLLLLLPLLWLLVLTPGRPAAGLSTCKTIDMELVKRKRIEAIRGQILSKLRLASPPS
QGEVPPGPLPEAVLALYNSTRDRVAGESAEPEPEPEADYYAKEVTRVLMVETHNEIYDKFK

QSTHSIYMFFNTSELREAVPEPVLLSRAELRLLRLKLKVEQHVELYQKYSNNSWRYLSNRLL
APSDSPEWLSFDVTGVVRQWLSRGGEIEGFRLSAHSSSDSRDNTLQVDINGFTTGRRGDL
ATIHGMNRPFLLLMATPLERAQHLQSSRHRRALDTNYCFSSTEKNCCVRQLYIDFRKDLGW
KWIHEPKGYHANFCLGPCPYIWSLDTQYSKVLALYNQHNPGASAAPCCVPQALEPLPIVYYV
GRKPKVEQLSNMIVRSCKCS.

Terminator sequence: a transcription terminator is a section of a nucleic acid sequence that marks the end of a gene during transcription. Release of the transcriptional complex frees RNA polymerase and related transcriptional machinery to begin transcription of new mRNAs. Additionally, the same cellular factors add a non-templated "poly-A tail" which significantly enhances the lifetime and functionality of the mRNA. An example of a suitable transcription terminator herein includes the "bGH_PA" terminator,

CGACTGTGCCTTCTAGTTGCCAGCCATCTGTTGTTTGCCCCTCCCCCGTGCCTTCCTTG
ACCCTGGAAGGTGCCACTCCCACTGTCCTTTCCTAATAAAATGAGGAAATTGCATCGCA
TTGTCTGAGTAGGTGTCATTCTATTCTGGGGGGTGGGGTGGGGCAGGACAGCAAGGGG
GAGGATTGGGAAGACAATAGCAGGCATGCTGGGGATGCGGTGGGCTCTATGG (SEQ ID
NO 20).

Any acceptable terminator sequence may be utilized herein. Variations include use of two different flanking terminator sequences in the instance of bidirectional promoters producing two oppositely-oriented mRNAs.

In one embodiment the plasmid of the invention has the sequence as set out in SEQ ID NO 24.

In a second embodiment the plasmid of the invention has the sequence SEQ ID NO 26: full (non-annotated) plasmid sequence

GACTCTTCGCGATGTACGGGCCAGATATACGCGTTGACATTGATTATTGACTAG
TTATTAATAGTAATCAATTACGGGGTCATTAGTTCATAGCCCATATATGGAGTTCCGCGT
TACATAACTTACGGTAAATGGCCCGCCTGGCTGACCGCCCAACGACCCCCGCCCATTG
ACGTCAATAATGACGTATGTTCCCATAGTAACGCCAATAGGGACTTTCCATTGACGTCAA
TGGGTGGACTATTTACGGTAAACTGCCCACTTGGCAGTACATCAAGTGTATCATATGCC
AAGTACGCCCCCTATTGACGTCAATGACGGTAAATGGCCCGCCTGGCATTATGCCCAGT
ACATGACCTTATGGGACTTTCCTACTTGGCAGTACATCTACGTATTAGTCATCGCTATTA
CCATGGTGATGCGGTTTTGGCAGTACATCAATGGGCGTGGATAGCGGTTTGACTCACG
GGGATTTCCAAGTCTCCACCCCATTGACGTCAATGGGAGTTTGTTTTGGCACCAAAATC
AACGGGACTTTCCAAAATGTCGTAACAACTCCGCCCCATTGACGCAAATGGGCGGTAG
GCGTGTACGGTGGGAGGTCTATATAAGCAGAGCTCTCTGGCTAACTAGAGAACCCACT
GCTTACTGGCTTATCGAAATTAATACGACTCACTATAGGGAGACCCAAGCTGGCTAGCG

TTTAAACTTAAGCTTGGTACCGAGCTCGGATCCACTAGTCCAGTGTGGTGGAATTCTGC
ACTGCAGCTCGCATCTCTCCTTCACGCGCCCGCCGCCCTACCTGAGGCCGCCATCCAC
GCCGGTTGAGTCGCGTTCTGCCGCCTCCCGCCTGTGGTGCCTCCTGAACTGCGTCCGC
CGTCTAGGTAAGTTTAAAGCTCAGGTCGAGACCGGGCCTTTGTCCGGCGCTCCCTTGG
AGCCTACCTAGACTCAGCCGGCTCTCCACGCTTTGCCTGACCCTGCTTGCTCAACTCTA
GGTAAGTTAATGAGACAGATAGAAACTGGTCTTGTAGAAACAGAGTAGTCGCCTGCTTT
TCTGCCAGGTGCTGACTTCTCTCCCCTGGGCTTTTTTCTTTTTCTCAGGTTGAAAAGAAG
AAGACGAAGAAGACGAAGAAGACAAACCGTCGTCGACTGCCATGCGCCGCTGATTAAC
GCCGCCACCATGGCCCACCGACGCAGATCCAGAAGCTGCCGTGAGGACCAGAAGCCC
GTGATGGATGATCAGAGGGACCTTATCTCTAACAATGAACAACTGCCAATGCTCGGCAG
ACGGCCTGGGGCCCCGGAGAGCAAGTGCAGCAGAGGAGCCTTGTACACGGGGTTCTC
CATTTTAGTGACTCTCCTTCTCGCCGGCCAAGCTACCACCGCCTACTTTCTGTACCAACA
GCAAGGCAGACTAGACAAACTGACAATCACAAGCCAGAACCTTCAGCTGGAGTCTCTGC
GGATGAAGCTGCCCGCTTTGTGGATGAGATTGCTTCCTCTACTTGCTCTCCTGGCGCTC
TGGGGACCTGACCCCGAGCAAGAGTTTGTTAATCAGCACCTGTGTGGGAGTCATCTGG
TGGAGGCACTCTATTTAGTGTGCGGAGAGAGGGGCTTCTTCTACACTCCAAAGACCAGA
CGGGAGGCCGAAGACCTTCAAGTGGGGCAAGTAGAACTGGGTGGCGGACCCGGTGCC
GGGAGCCTTCAGCCGCTCGCCCTGGAGGGCTCTCTTCAGAAACGCGGCATCGTGGAG
CAGTGTTGCACATCCATTTGCTCACTCTACCAGCTGGAGAACTACTGCAACGGAAGCGG
AGTGAAGCAGACGTTGAATTTTGATTTGTTGAAGTTGGCGGGGGATGTGGAGAGCAATC
CGGGGCCGATGCCCCCTAGTGGCCTCAGACTTTTGTTATTGTTATTACCGCTTTTATGG
CTCTTGGTGCTGACACCGGGCCGTCCGGCTGCTGGCTTGTCGACTTGTAAGACAATTG
ATATGGAATTGGTGAAACGAAAACGGATTGAGGCCATCCGAGGACAGATTTTGAGCAAG
CTGCGGCTTGCCTCGCCACCCTCGCAAGGGGAAGTCCCACCCGGACCTCTACCAGAAG
CAGTCCTAGCGCTGTACAACAGTACAAGAGATAGAGTGGCCGGGGAATCCGCAGAACC
AGAGCCTGAGCCTGAAGCCGATTATTATGCAAAGGAAGTGACTAGGGTCCTGATGGTC
GAGACCCATAACGAAATCTACGACAAATTCAAACAAAGTACCCACTCTATCTACATGTTC
TTCAACACCAGTGAGCTAAGAGAAGCCGTGCCCGAACCTGTGCTTCTTTCCCGCGCAG
AACTCCGCCTCTTGAGACTCAAATTGAAAGTTGAACAACACGTAGAGCTTTACCAGAAAT
ACTCTAATAATTCATGGCGATATCTTTCTAATCGTCTCCTCGCCCCATCTGACAGCCCTG
AATGGCTCTCCTTCGACGTTACGGGAGTTGTGCGCCAGTGGCTCAGCAGAGGCGGAGA
GATAGAGGGCTTTCGGCTGAGCGCACATAGCTCTAGCGACTCAAGGGACAACACATTG
CAAGTGGATATTAACGGTTTTACAACTGGACGGAGAGGGGACCTGGCGACCATCCACG
GCATGAATAGACCTTTCCTGCTGCTGATGGCTACTCCCCTGGAGAGGGCACAGCACTTA
CAGTCTTCCAGACACCGGCGCGCCCTGGATACAAACTACTGCTTCAGCTCCACCGAAAA

GAACTGTTGCGTGCGGCAGCTGTACATTGACTTCAGAAAGGATCTGGGCTGGAAGTGG
ATTCATGAGCCCAAGGGGTATCATGCCAACTTCTGTCTTGGGCCATGCCCATACATCTG
GTCACTGGATACCCAGTACTCCAAAGTTCTGGCCTTGTACAATCAACACAACCCTGGAG
CTTCCGCCGCTCCTTGCTGTGTGCCCCAAGCCCTAGAGCCCCTGCCCATCGTTTATTAT
GTCGGACGCAAGCCCAAAGTAGAACAGCTATCAAATATGATCGTGAAGCTGCAAGTG
TAGCTGATAAACGCGTCGAGCATGCATCTAGGGCGGCCAATTCCGCCCCTCTCCCCCC
CACCCCTCTCCCTCCCCCCCCCCTAACGTTACTGGCCGAAGCCGCTTGGAATAAGGCC
GGTGTGCGTTTGTCTATATGTTATTTTCCACCATATTGCCGTCTTTTGGCAATGTGAGGG
CCCGGAAACCTGGCCCTGTCTTCTTGACGAGCATTCCTAGGGGTCTTTCCCCTCTCGCC
AAAGGAATGCAAGGTCTGTTGAATGTCGTGAAGGAAGCAGTTCCTCTGGAAGCTTCTTG
AAGACAAACAACGTCTGTAGCGACCCTTTGTAGACAGCGGAACCCCCCACCTGGCGAT
AGATGCCTCTGCGGCCAAAAGCCACGTGTATAAGATACACCTGCAAAGGCGGCACAAC
CCCAGTGCCACGTTGTGAGTTGGATAGTTGTGGAAAGAGTCAAATGGCTCTCCTCAAGC
GTATTCAACAAGGGGCTGAAGGATGCCCAGAAGGTACCCCATTGTATGGGATCTGATCT
GGGGCCTCGGTGCACATGCTTTACATGTGTTTAGTCGAGGTTAAAAAACGTCTAGGCCC
CCCGAACCACGGGGACGTGGTTTTCCTTTGAAAAACACGATGATAATATGATGCACAGC
TCAGCACTGCTCTGTTGCCTGGTCCTCCTGACTGGGGTGAGGGCCAGCCCAGGCCAG
GGCACCCAGTCTGAGAACAGCTGCACCCACTTCCCAGGCAACCTGCCTAACATGCTTC
GAGATCTCCGAGATGCCTTCAGCAGAGTGAAGACTTTCTTTCAAATGAAGGATCAGCTG
GACAACTTGTTGTTAAAGGAGTCCTTGCTGGAGGACTTTAAGGGTTACCTGGGTTGCCA
AGCCTTGTCTGAGATGATCCAGTTTTACCTGGAGGAGGTGATGCCCCAAGCTGAGAACC
AAGACCCAGACATCAAGGCGCATGTGAACTCCCTGGGGGAGAACCTGAAGACCCTCAG
GCTGAGGCTACGGCGCTGTCATCGATTTCTTCCCTGTGAAAACAAGAGCAAGGCCGTG
GAGCAGGTGAAGAATGCCTTTAATAAGCTCCAAGAGAAAGGCATCTACAAAGCCATGAG
TGAGTTTGACATCTTCATCAACTACATAGAAGCCTACATGACAATGAAGATACGAAACGG
GAGCGGCGCTACTAACTTCAGCCTGCTGAAGCAGGCTGGAGACGTGGAGGAGAACCCT
GGACCTATGTACAGAATGCAGCTGCTGAGCTGCATCGCCCTGAGCCTGGCCCTGGTGA
CCAACAGCGCACCCACGTCCTCTAGCACCAAGAAGACCCAGTTACAGTTGGAGCATCTA
CTTTTAGACCTGCAAATGATTTTGAACGGCATCAACAACTACAAGAATCCTAAACTTACT
CGCATGCTTACCTTCAAATTTTACATGCCCAAGAAGGCCACCGAACTGAAGCACTTGCA
ATGTCTGGAGGAAGAACTCAAGCCGCTGGAGGAAGTTCTCAACCTCGCGCAGTCCAAG
AATTTCCACCTCCGGCCAAGAGACCTGATCAGTAACATTAATGTGATAGTGCTGGAGCT
GAAGGGAAGCGAGACTACATTTATGTGCGAGTACGCCGATGAAACCGCTACAATCGTC
GAGTTCCTGAATAGATGGATCACATTTTGCCAGTCAATTATCTCTACTCTGACATGATAA
CTCGAGGTCTAGAGGGCCCGTTTAAACCCGCTGATCAGCCTCGACTGTGCCTTCTAGTT

GCCAGCCATCTGTTGTTTGCCCCTCCCCCGTGCCTTCCTTGACCCTGGAAGGTGCCACT
CCCACTGTCCTTTCCTAATAAAATGAGGAAATTGCATCGCATTGTCTGAGTAGGTGTCAT
TCTATTCTGGGGGGTGGGGTGGGGCAGGACAGCAAGGGGGAGGATTGGGAAGACAAT
AGCAGGCATGCTGGGGATGCGGTGGGCTCTATGGCTTCTACTGGGCGGTTTTATGGAC
AGCAAGCGAACCGGAATTGCCAGCTGGGGCGCCCTCTGGTAAGGTTGGGAAGCCCTG
CAAAGTAAACTGGATGGCTTTCTCGCCGCCAAGGATCTGATGGCGCAGGGGATCAAGC
TCTGATCAAGAGACAGGATGAGGATCGTTTCGCATGGCCAAAGAAGACAATATTGAAAT
GCAAGGTACCGTTCTTGAAACGTTGCCTAATACCATGTTCCGCGTAGAGTTAGAAAACG
GTCACGTGGTTACTGCACACATCTCCGGTAAAATGCGCAAAAACTACATCCGCATCCTG
ACGGGCGACAAAGTGACTGTTGAACTGACCCCGTACGACCTGAGCAAAGGCCGCATTG
TCTTCCGTAGTCGCTGATAAATTATTAACGCTTACAATTTCCTGATGCGGTATTTTCTCCT
TACGCATCTGTGCGGTATTTCACACCGCATACAGGTGGCACTTTTCGGGGAAATGTGCG
CGGAACCCCTATTTGTTTATTTTTCTAAATACATTCAAATATGTATCCGCTCATGAGACAA
TAACCCTGATAAATGCTTCAATAATAGCACGTGCTAAAACTTCATTTTTAATTTAAAAGGA
TCTAGGTGAAGATCCTTTTTGATAATCTCATGACCAAAATCCCTTAACGTGAGTTTTCGTT
CCACTGAGCGTCAGACCCCGTAGAAAAGATCAAAGGATCTTCTTGAGATCCTTTTTTTCT
GCGCGTAATCTGCTGCTTGCAAACAAAAAAACCACCGCTACCAGCGGTGGTTTGTTTGC
CGGATCAAGAGCTACCAACTCTTTTTCCGAAGGTAACTGGCTTCAGCAGAGCGCAGATA
CCAAATACTGTTCTTCTAGTGTAGCCGTAGTTAGGCCACCACTTCAAGAACTCTGTAGCA
CCGCCTACATACCTCGCTCTGCTAATCCTGTTACCAGTGGCTGCTGCCAGTGGCGATAA
GTCGTGTCTTACCGGGTTGGACTCAAGACGATAGTTACCGGATAAGGCGCAGCGGTCG
GGCTGAACGGGGGGTTCGTGCACACAGCCCAGCTTGGAGCGAACGACCTACACCGAA
CTGAGATACCTACAGCGTGAGCTATGAGAAAGCGCCACGCTTCCCGAAGGGAGAAAGG
CGGACAGGTATCCGGTAAGCGGCAGGGTCGGAACAGGAGAGCGCACGAGGGAGCTTC
CAGGGGGAAACGCCTGGTATCTTTATAGTCCTGTCGGGTTTCGCCACCTCTGACTTGAG
CGTCGATTTTTGTGATGCTCGTCAGGGGGGCGGAGCCTATGGAAAAACGCCAGCAACG
CGGCCTTTTTACGGTTCCTGGGCTTTTGCTGGCCTTTTGCTCACATGTTCTT.

In a third embodiment the plasmid of the invention has the sequence SEQ ID NO 28: full (non-annotated) plasmid sequence

GACTCTTCGCGATGTACGGGCCAGATATACGCGTTGACATTGATTATTGACTAG
TTATTAATAGTAATCAATTACGGGGTCATTAGTTCATAGCCCATATATGGAGTTCCGCGT
TACATAACTTACGGTAAATGGCCCGCCTGGCTGACCGCCCAACGACCCCCGCCCATTG
ACGTCAATAATGACGTATGTTCCCATAGTAACGCCAATAGGGACTTTCCATTGACGTCAA
TGGGTGGACTATTTACGGTAAACTGCCCACTTGGCAGTACATCAAGTGTATCATATGCC
AAGTACGCCCCCTATTGACGTCAATGACGGTAAATGGCCCGCCTGGCATTATGCCCAGT

ACATGACCTTATGGGACTTTCCTACTTGGCAGTACATCTACGTATTAGTCATCGCTATTA
CCATGGTGATGCGGTTTTGGCAGTACATCAATGGGCGTGGATAGCGGTTTGACTCACG
GGGATTTCCAAGTCTCCACCCCATTGACGTCAATGGGAGTTTGTTTTGGCACCAAAATC
AACGGGACTTTCCAAAATGTCGTAACAACTCCGCCCCATTGACGCAAATGGGCGGTAG
GCGTGTACGGTGGGAGGTCTATATAAGCAGAGCTCTCTGGCTAACTAGAGAACCCACT
GCTTACTGGCTTATCGAAATTAATACGACTCACTATAGGGAGACCCAAGCTGGCTAGCG
TTTAAACTTAAGCTTGGTACCGAGCTCGGATCCACTAGTCCAGTGTGGTGGAATTCTGC
AGCTCGCATCTCTCCTTCACGCGCCCGCCGCCCTACCTGAGGCCGCCATCCACGCCGG
TTGAGTCGCGTTCTGCCGCCTCCCGCCTGTGGTGCCTCCTGAACTGCGTCCGCCGTCT
AGGTAAGTTTAAAGCTCAGGTCGAGACCGGGCCTTTGTCCGGCGCTCCCTTGGAGCCT
ACCTAGACTCAGCCGGCTCTCCACGCTTTGCCTGACCCTGCTTGCTCAACTCTAGGTAA
GTTAATGAGACAGATAGAAACTGGTCTTGTAGAAACAGAGTAGTCGCCTGCTTTTCTGC
CAGGTGCTGACTTCTCTCCCCTGGGCTTTTTCTTTTTCTCAGGTTGAAAAGAAGAAGAC
GAAGAAGACGAAGAAGACAAACCGTCGTCGACTGCCATGCGCCGCTGATTAACGCCGC
CACCATGGCCCACCGACGCAGATCCAGAAGCTGCCGTGAGGACCAGAAGCCCGTGAT
GGATGATCAGAGGGACCTTATCTCTAACAATGAACAACTGCCAATGCTCGGCAGACGGC
CTGGGGCCCCGGAGAGCAAGTGCAGCAGAGGAGCCTTGTACACGGGGTTCTCCATTTT
AGTGACTCTCCTTCTCGCCGGCCAAGCTACCACCGCCTACTTTCTGTACCAACAGCAAG
GCAGACTAGACAAACTGACAATCACAAGCCAGAACCTTCAGCTGGAGTCTCTGCGGATG
AAGCTGCCCGCTTTGTGGATGAGATTGCTTCCTCTACTTGCTCTCCTGGCGCTCTGGGG
ACCTGACCCCGAGCAAGAGTTTGTTAATCAGCACCTGTGTGGGAGTCATCTGGTGGAG
GCACTCTATTTAGTGTGCGGAGAGAGGGGCTTCTTCTACACTCCAAAGACCAGACGGG
AGGCCGAAGACCTTCAAGTGGGGCAAGTAGAACTGGGTGGCGGACCCGGTGCCGGGA
GCCTTCAGCCGCTCGCCCTGGAGGGCTCTCTTCAGAAACGCGGCATCGTGGAGCAGTG
TTGCACATCCATTTGCTCACTCTACCAGCTGGAGAACTACTGCAACGGAAGCGGAGTGA
AGCAGACGTTGAATTTTGATTTGTTGAAGTTGGCGGGGGATGTGGAGAGCAATCCGGG
GCCGATGCCCCCTAGTGGCCTCAGACTTTTGTTATTGTTATTACCGCTTTTATGGCTCTT
GGTGCTGACACCGGGCCGTCCGGCTGCTGGCTTGTCGACTTGTAAGACAATTGATATG
GAATTGGTGAAACGAAAACGGATTGAGGCCATCCGAGGACAGATTTTGAGCAAGCTGC
GGCTTGCCTCGCCACCCTCGCAAGGGGAAGTCCCACCCGGACCTCTACCAGAAGCAGT
CCTAGCGCTGTACAACAGTACAAGAGATAGAGTGGCCGGGGAATCCGCAGAACCAGAG
CCTGAGCCTGAAGCCGATTATTATGCAAAGGAAGTGACTAGGGTCCTGATGGTCGAGA
CCCATAACGAAATCTACGACAAATTCAAACAAAGTACCCACTCTATCTACATGTTCTTCA
ACACCAGTGAGCTAAGAGAAGCCGTGCCCGAACCTGTGCTTCTTTCCCGCGCAGAACT
CCGCCTCTTGAGACTCAAATTGAAAGTTGAACAACACGTAGAGCTTTACCAGAAATACTC

TAATAATTCATGGCGATATCTTTCTAATCGTCTCCTCGCCCCATCTGACAGCCCTGAATG
GCTCTCCTTCGACGTTACGGGAGTTGTGCGCCAGTGGCTCAGCAGAGGCGGAGAGATA
GAGGGCTTTCGGCTGAGCGCACATAGCTCTAGCGACTCAAGGACAACACATTGCAAG
TGGATATTAACGGTTTTACAACTGGACGGAGAGGGGACCTGGCGACCATCCACGGCAT
GAATAGACCTTTCCTGCTGCTGATGGCTACTCCCCTGGAGAGGGCACAGCACTTACAGT
CTTCCAGACACCGGCGCGCCCTGGATACAAACTACTGCTTCAGCTCCACCGAAAAGAA
CTGTTGCGTGCGGCAGCTGTACATTGACTTCAGAAAGGATCTGGGCTGGAAGTGGATT
CATGAGCCCAAGGGGTATCATGCCAACTTCTGTCTTGGGCCATGCCCATACATCTGGTC
ACTGGATACCCAGTACTCCAAAGTTCTGGCCTTGTACAATCAACACAACCCTGGAGCTT
CCGCCGCTCCTTGCTGTGTGCCCCAAGCCCTAGAGCCCCTGCCCATCGTTTATTATGTC
GGACGCAAGCCCAAAGTAGAACAGCTATCAAATATGATCGTGAGAAGCTGCAAGTGTAG
CTGATAAACGCGTCGAGCATGCATCTAGGGCGGCCAATTCCGCCCCTCTCCCCCCCAC
CCCTCTCCCTCCCCCCCCCCTAACGTTACTGGCCGAAGCCGCTTGGAATAAGGCCGGT
GTGCGTTTGTCTATATGTTATTTTCCACCATATTGCCGTCTTTTGGCAATGTGAGGGCCC
GGAAACCTGGCCCTGTCTTCTTGACGAGCATTCCTAGGGGTCTTTCCCCTCTCGCCAAA
GGAATGCAAGGTCTGTTGAATGTCGTGAAGGAAGCAGTTCCTCTGGAAGCTTCTTGAAG
ACAAACAACGTCTGTAGCGACCCTTTGTAGACAGCGGAACCCCCCACCTGGCGATAGA
TGCCTCTGCGGCCAAAAGCCACGTGTATAAGATACACCTGCAAAGGCGGCACAACCCC
AGTGCCACGTTGTGAGTTGGATAGTTGTGGAAAGAGTCAAATGGCTCTCCTCAAGCGTA
TTCAACAAGGGGCTGAAGGATGCCCAGAAGGTACCCCATTGTATGGGATCTGATCTGG
GGCCTCGGTGCACATGCTTTACATGTGTTTAGTCGAGGTTAAAAAACGTCTAGGCCCCC
CGAACCACGGGGACGTGGTTTTCCTTTGAAAAACACGATGATAATATGATGCACAGCTC
AGCACTGCTCTGTTGCCTGGTCCTCCTGACTGGGGTGAGGGCCAGCCCAGGCCAGGG
CACCCAGTCTGAGAACAGCTGCACCCACTTCCCAGGCAACCTGCCTAACATGCTTCGA
GATCTCCGAGATGCCTTCAGCAGAGTGAAGACTTTCTTTCAAATGAAGGATCAGCTGGA
CAACTTGTTGTTAAAGGAGTCCTTGCTGGAGGACTTTAAGGGTTACCTGGGTTGCCAAG
CCTTGTCTGAGATGATCCAGTTTTACCTGGAGGAGGTGATGCCCCAAGCTGAGAACCAA
GACCCAGACATCAAGGCGCATGTGAACTCCCTGGGGGAGAACCTGAAGACCCTCAGGC
TGAGGCTACGGCGCTGTCATCGATTTCTTCCCTGTGAAAACAAGAGCAAGGCCGTGGA
GCAGGTGAAGAATGCCTTTAATAAGCTCCAAGAGAAAGGCATCTACAAAGCCATGAGTG
AGTTTGACATCTTCATCAACTACATAGAAGCCTACATGACAATGAAGATACGAAACGGGA
GCGGCGCTACTAACTTCAGCCTGCTGAAGCAGGCTGGAGACGTGGAGGAGAACCCTG
GACCTATGTACAGAATGCAGCTGCTGAGCTGCATCGCCCTGAGCCTGGCCCTGGTGAC
CAACAGCGCACCCACGTCCTCTAGCACCAAGAAGACCCAGTTACAGTTGGAGCATCTAC
TTTTAGACCTGCAAATGATTTTGAACGGCATCAACAACTACAAGAATCCTAAACTTACTC

GCATGCTTACCTTCAAATTTACATGCCCAAGAAGGCCACCGAACTGAAGCACTTGCAA
TGTCTGGAGGAAGAACTCAAGCCGCTGGAGGAAGTTCTCAACCTCGCGCAGTCCAAGA
ATTTCCACCTCCGGCCAAGAGACCTGATCAGTAACATTAATGTGATAGTGCTGGAGCTG
AAGGGAAGCGAGACTACATTTATGTGCGAGTACGCCGATGAAACCGCTACAATCGTCGA
GTTCCTGAATAGATGGATCACATTTTGCCAGTCAATTATCTCTACTCTGACATGATAACT
CGAGTCTAGAGGGCCCGTTTAAACCCGCTGATCAGCCTCGACTGTGCCTTCTAGTTGCC
AGCCATCTGTTGTTTGCCCCTCCCCCGTGCCTTCCTTGACCCTGGAAGGTGCCACTCCC
ACTGTCCTTTCCTAATAAAATGAGGAAATTGCATCGCATTGTCTGAGTAGGTGTCATTCT
ATTCTGGGGGGTGGGGTGGGGCAGGACAGCAAGGGGGAGGATTGGGAAGACAATAGC
AGGCATGCTGGGGATGCGGTGGGCTCTATGGCTTCTACTGGGCGGTTTTATGGACAGC
AAGCGAACCGGAATTGCCAGCTGGGGCGCCCTCTGGTAAGGTTGGGAAGCCCTGCAAA
GTAAACTGGATGGCTTTCTCGCCGCCAAGGATCTGATGGCGCAGGGGATCAAGCTCTG
ATCAAGAGACAGGATGAGGATCGTTTCGCATGGCCAAAGAAGACAATATTGAAATGCAA
GGTACCGTTCTTGAAACGTTGCCTAATACCATGTTCCGCGTAGAGTTAGAAAACGGTCA
CGTGGTTACTGCACACATCTCCGGTAAAATGCGCAAAAACTACATCCGCATCCTGACGG
GCGACAAAGTGACTGTTGAACTGACCCCGTACGACCTGAGCAAAGGCCGCATTGTCTT
CCGTAGTCGCTGATAAATTATTAACGCTTACAATTTCCTGATGCGGTATTTTCTCCTTAC
GCATCTGTGCGGTATTTCACACCGCATACAGGTGGCACTTTTCGGGGAAATGTGCGCG
GAACCCCTATTTGTTTATTTTTCTAAATACATTCAAATATGTATCCGCTCATGAGACAATA
ACCCTGATAAATGCTTCAATAATAGCACGTGCTAAAACTTCATTTTTAATTTAAAAGGATC
TAGGTGAAGATCCTTTTTGATAATCTCATGACCAAAATCCCTTAACGTGAGTTTTCGTTC
CACTGAGCGTCAGACCCCGTAGAAAAGATCAAAGGATCTTCTTGAGATCCTTTTTTTCTG
CGCGTAATCTGCTGCTTGCAAACAAAAAAACCACCGCTACCAGCGGTGGTTTGTTTGCC
GGATCAAGAGCTACCAACTCTTTTTCCGAAGGTAACTGGCTTCAGCAGAGCGCAGATAC
CAAATACTGTTCTTCTAGTGTAGCCGTAGTTAGGCCACCACTTCAAGAACTCTGTAGCAC
CGCCTACATACCTCGCTCTGCTAATCCTGTTACCAGTGGCTGCTGCCAGTGGCGATAAG
TCGTGTCTTACCGGGTTGGACTCAAGACGATAGTTACCGGATAAGGCGCAGCGGTCGG
GCTGAACGGGGGGTTCGTGCACACAGCCCAGCTTGGAGCGAACGACCTACACCGAACT
GAGATACCTACAGCGTGAGCTATGAGAAAGCGCCACGCTTCCCGAAGGGAGAAAGGCG
GACAGGTATCCGGTAAGCGGCAGGGTCGGAACAGGAGAGCGCACGAGGGAGCTTCCA
GGGGGAAACGCCTGGTATCTTTATAGTCCTGTCGGGTTTCGCCACCTCTGACTTGAGCG
TCGATTTTTGTGATGCTCGTCAGGGGGGCGGAGCCTATGGAAAAACGCCAGCAACGCG
GCCTTTTTACGGTTCCTGGGCTTTTGCTGGCCTTTTGCTCACATGTTCTT.

In a fourth embodiment the plasmid of the invention has the sequence SEQ ID NO 29: full (non-annotated) plasmid sequence

GACTCTTCGCGATGTACGGGCCAGATATACGCGTTGACATTGATTATTGACTAG
TTATTAATAGTAATCAATTACGGGGTCATTAGTTCATAGCCCATATATGGAGTTCCGCGT
TACATAACTTACGGTAAATGGCCCGCCTGGCTGACCGCCCAACGACCCCCGCCCATTG
ACGTCAATAATGACGTATGTTCCCATAGTAACGCCAATAGGGACTTTCCATTGACGTCAA
TGGGTGGACTATTTACGGTAAACTGCCCACTTGGCAGTACATCAAGTGTATCATATGCC
AAGTACGCCCCCTATTGACGTCAATGACGGTAAATGGCCCGCCTGGCATTATGCCCAGT
ACATGACCTTATGGGACTTTCCTACTTGGCAGTACATCTACGTATTAGTCATCGCTATTA
CCATGGTGATGCGGTTTTGGCAGTACATCAATGGGCGTGGATAGCGGTTTGACTCACG
GGGATTTCCAAGTCTCCACCCCATTGACGTCAATGGGAGTTTGTTTTGGCACCAAAATC
AACGGGACTTTCCAAAATGTCGTAACAACTCCGCCCCATTGACGCAAATGGGCGGTAG
GCGTGTACGGTGGGAGGTCTATATAAGCAGAGCTCTCTGGCTAACTAGAGAACCCACT
GCTTACTGGCTTATCGAAATTAATACGACTCACTATAGGGAGACCCAAGCTGGCTAGCG
TTTAAACTTAAGCTTGGTACCGAGCTCGGATCCACTAGTCCAGTGTGGTGGAATTCTGC
AGCTCGCATCTCTCCTTCACGCGCCCGCCGCCCTACCTGAGGCCGCCATCCACGCCGG
TTGAGTCGCGTTCTGCCGCCTCCCGCCTGTGGTGCCTCCTGAACTGCGTCCGCCGTCT
AGGTAAGTTTAAAGCTCAGGTCGAGACCGGGCCTTTGTCCGGCGCTCCCTTGGAGCCT
ACCTAGACTCAGCCGGCTCTCCACGCTTTGCCTGACCCTGCTTGCTCAACTCTAGGTAA
GTTAATGAGACAGATAGAAACTGGTCTTGTAGAAACAGAGTAGTCGCCTGCTTTTCTGC
CAGGTGCTGACTTCTCTCCCCTGGGCTTTTTCTTTTTCTCAGGTTGAAAAGAAGAAGAC
GAAGAAGACGAAGAAGACAAACCGTCGTCGACTGCCATGCGCCGCTGATTAACGCCGC
CACCATGGCCCACCGACGCAGATCCAGAAGCTGCCGTGAGGACCAGAAGCCCGTGAT
GGATGATCAGAGGGACCTTATCTCTAACAATGAACAACTGCCAATGCTCGGCAGACGGC
CTGGGGCCCCGGAGAGCAAGTGCAGCAGAGGAGCCTTGTACACGGGGTTCTCCATTTT
AGTGACTCTCCTTCTCGCCGGCCAAGCTACCACCGCCTACTTTCTGTACCAACAGCAAG
GCAGACTAGACAAACTGACAATCACAAGCCAGAACCTTCAGCTGGAGTCTCTGCGGATG
AAGCTGCCCGCTTTGTGGATGAGATTGCTTCCTCTACTTGCTCTCCTGGCGCTCTGGGG
ACCTGACCCCGAGCAAGAGTTTGTTAATCAGCACCTGTGTGGGAGTCATCTGGTGGAG
GCACTCTATTTAGTGTGCGGAGAGAGGGGCTTCTTCTACACTCCAAAGACCAGACGGG
AGGCCGAAGACCTTCAAGTGGGGCAAGTAGAACTGGGTGGCGGACCCGGTGCCGGGA
GCCTTCAGCCGCTCGCCCTGGAGGGCTCTCTTCAGAAACGCGGCATCGTGGAGCAGTG
TTGCACATCCATTTGCTCACTCTACCAGCTGGAGAACTACTGCAACGGAAGCGGAGTGA
AGCAGACGTTGAATTTTGATTTGTTGAAGTTGGCGGGGGATGTGGAGAGCAATCCGGG
GCCGATGCCCCCTAGTGGCCTCAGACTTTTGTTATTGTTATTACCGCTTTTATGGCTCTT

GGTGCTGACACCGGGCCGTCCGGCTGCTGGCTTGTCGACTTGTAAGACAATTGATATG
GAATTGGTGAAACGAAAACGGATTGAGGCCATCCGAGGACAGATTTTGAGCAAGCTGC
GGCTTGCCTCGCCACCCTCGCAAGGGAAGTCCCACCCGGACCTCTACCAGAAGCAGT
CCTAGCGCTGTACAACAGTACAAGAGATAGAGTGGCCGGGGAATCCGCAGAACCAGAG
CCTGAGCCTGAAGCCGATTATTATGCAAAGGAAGTGACTAGGGTCCTGATGGTCGAGA
CCCATAACGAAATCTACGACAAATTCAAACAAAGTACCCACTCTATCTACATGTTCTTCA
ACACCAGTGAGCTAAGAGAAGCCGTGCCCGAACCTGTGCTTCTTTCCCGCGCAGAACT
CCGCCTCTTGAGACTCAAATTGAAAGTTGAACAACACGTAGAGCTTTACCAGAAATACTC
TAATAATTCATGGCGATATCTTTCTAATCGTCTCCTCGCCCCATCTGACAGCCCTGAATG
GCTCTCCTTCGACGTTACGGGAGTTGTGCGCCAGTGGCTCAGCAGAGGCGGAGAGATA
GAGGGCTTTCGGCTGAGCGCACATAGCTCTAGCGACTCAAGGGACAACACATTGCAAG
TGGATATTAACGGTTTTACAACTGGACGGAGAGGGGACCTGGCGACCATCCACGGCAT
GAATAGACCTTTCCTGCTGCTGATGGCTACTCCCCTGGAGAGGGCACAGCACTTACAGT
CTTCCAGACACCGGCGCGCCCTGGATACAAACTACTGCTTCAGCTCCACCGAAAAGAA
CTGTTGCGTGCGGCAGCTGTACATTGACTTCAGAAAGGATCTGGGCTGGAAGTGGATT
CATGAGCCCAAGGGGTATCATGCCAACTTCTGTCTTGGGCCATGCCCATACATCTGGTC
ACTGGATACCCAGTACTCCAAAGTTCTGGCCTTGTACAATCAACACAACCCTGGAGCTT
CCGCCGCTCCTTGCTGTGTGCCCCAAGCCCTAGAGCCCCTGCCCATCGTTTATTATGTC
GGACGCAAGCCCAAAGTAGAACAGCTATCAAATATGATCGTGAGAAGCTGCAAGTGTAG
CTGATAAACGCGTCGAGCATGCATCTAGGGCGGCCAATTCCGCCCCTCTCCCCCCCAC
CCCTCTCCCTCCCCCCCCCCTAACGTTACTGGCCGAAGCCGCTTGGAATAAGGCCGGT
GTGCGTTTGTCTATATGTTATTTTCCACCATATTGCCGTCTTTTGGCAATGTGAGGGCCC
GGAAACCTGGCCCTGTCTTCTTGACGAGCATTCCTAGGGGTCTTTCCCCTCTCGCCAAA
GGAATGCAAGGTCTGTTGAATGTCGTGAAGGAAGCAGTTCCTCTGGAAGCTTCTTGAAG
ACAAACAACGTCTGTAGCGACCCTTTGTAGACAGCGGAACCCCCCACCTGGCGATAGA
TGCCTCTGCGGCCAAAAGCCACGTGTATAAGATACACCTGCAAAGGCGGCACAACCCC
AGTGCCACGTTGTGAGTTGGATAGTTGTGGAAAGAGTCAAATGGCTCTCCTCAAGCGTA
TTCAACAAGGGGCTGAAGGATGCCCAGAAGGTACCCCATTGTATGGGATCTGATCTGG
GGCCTCGGTGCACATGCTTTACATGTGTTTAGTCGAGGTTAAAAAACGTCTAGGCCCCC
CGAACCACGGGGACGTGGTTTTCCTTTGAAAAACACGATGATAATATGATGCACAGCTC
AGCACTGCTCTGTTGCCTGGTCCTCCTGACTGGGGTGAGGGCCAGCCCAGGCCAGGG
CACCCAGTCTGAGAACAGCTGCACCCACTTCCCAGGCAACCTGCCTAACATGCTTCGA
GATCTCCGAGATGCCTTCAGCAGAGTGAAGACTTTCTTTCAAATGAAGGATCAGCTGGA
CAACTTGTTGTTAAAGGAGTCCTTGCTGGAGGACTTTAAGGGTTACCTGGGTTGCCAAG
CCTTGTCTGAGATGATCCAGTTTTACCTGGAGGAGGTGATGCCCCAAGCTGAGAACCAA

GACCCAGACATCAAGGCGCATGTGAACTCCCTGGGGGAGAACCTGAAGACCCTCAGGC
TGAGGCTACGGCGCTGTCATCGATTTCTTCCCTGTGAAAACAAGAGCAAGGCCGTGGA
GCAGGTGAAGAATGCCTTTAATAAGCTCCAAGAGAAAGGCATCTACAAAGCCATGAGTG
AGTTTGACATCTTCATCAACTACATAGAAGCCTACATGACAATGAAGATACGAAACGGGA
GCGGCGCTACTAACTTCAGCCTGCTGAAGCAGGCTGGAGACGTGGAGGAGAACCCTG
GACCTATGTACAGAATGCAGCTGCTGAGCTGCATCGCCCTGAGCCTGGCCCTGGTGAC
CAACAGCGCACCCACGTCCTCTAGCACCAAGAAGACCCAGTTACAGTTGGAGCATCTAC
TTTTAGACCTGCAAATGATTTTGAACGGCATCAACAACTACAAGAATCCTAAACTTACTC
GCATGCTTACCTTCAAATTTTACATGCCCAAGAAGGCCACCGAACTGAAGCACTTGCAA
TGTCTGGAGGAAGAACTCAAGCCGCTGGAGGAAGTTCTCAACCTCGCGCAGTCCAAGA
ATTTCCACCTCCGGCCAAGAGACCTGATCAGTAACATTAATGTGATAGTGCTGGAGCTG
AAGGGAAGCGAGACTACATTTATGTGCGAGTACGCCGATGAAACCGCTACAATCGTCGA
GTTCCTGAATAGATGGATCACATTTTGCCAGTCAATTATCTCTACTCTGACATGATAACT
CGAGTCTAGAGGGCCCGTTTAAACCCGCTGATCAGCCTCGACTGTGCCTTCTAGTTGCC
AGCCATCTGTTGTTTGCCCCTCCCCCGTGCCTTCCTTGACCCTGGAAGGTGCCACTCCC
ACTGTCCTTTCCTAATAAAATGAGGAAATTGCATCGCATTGTCTGAGTAGGTGTCATTCT
ATTCTGGGGGGTGGGGTGGGGCAGGACAGCAAGGGGGAGGATTGGGAAGACAATAGC
AGGCATGCTGGGGATGCGGTGGGCTCTATGGCTTCTACTGGGCGGTTTTATGGACAGC
AAGCGAACCGGAATTGCCAGCTGGGGCGCCCTCTGGTAAGGTTGGGAAGCCCTGCAAA
GTAAACTGGATGGCTTTCTCGCCGCCAAGGATCTGATGGCGCAGGGGATCAAGCTCTG
ATCAAGAGACAGGATGAGGATCGTTTCGCATGGCCAAAGAAGACAATATTGAAATGCAA
GGTACCGTTCTTGAAACGTTGCCTAATACCATGTTCCGCGTAGAGTTAGAAAACGGTCA
CGTGGTTACTGCACACATCTCCGGTAAAATGCGCAAAAACTACATCCGCATCCTGACGG
GCGACAAAGTGACTGTTGAACTGACCCCGTACGACCTGAGCAAAGGCCGCATTGTCTT
CCGTAGTCGCTGATAAATTATTAACGCTTACAATTTCCTGATGCGGTATTTTCTCCTTAC
GCATCTGTGCGGTATTTCACACCGCATACAGGTGGCACTTTTCGGGGAAATGTGCGCG
GAACCCCTATTTGTTTATTTTTCTAAATACATTCAAATATGTATCCGCTCATGAGACAATA
ACCCTGATAAATGCTTCAATAATAGCACGTGCTAAAACTTCATTTTTAATTTAAAAGGATC
TAGGTGAAGATCCTTTTTGATAATCTCATGACCAAAATCCCTTAACGTGAGTTTTCGTTC
CACTGAGCGTCAGACCCCGTAGAAAAGATCAAAGGATCTTCTTGAGATCCTTTTTTTCTG
CGCGTAATCTGCTGCTTGCAAACAAAAAAACCACCGCTACCAGCGGTGGTTTGTTTGCC
GGATCAAGAGCTACCAACTCTTTTTCCGAAGGTAACTGGCTTCAGCAGAGCGCAGATAC
CAAATACTGTTCTTCTAGTGTAGCCGTAGTTAGGCCACCACTTCAAGAACTCTGTAGCAC
CGCCTACATACCTCGCTCTGCTAATCCTGTTACCAGTGGCTGCTGCCAGTGGCGATAAG
TCGTGTCTTACCGGGTTGGACTCAAGACGATAGTTACCGGATAAGGCGCAGCGGTCGG

GCTGAACGGGGGGTTCGTGCACACAGCCCAGCTTGGAGCGAACGACCTACACCGAACT
GAGATACCTACAGCGTGAGCTATGAGAAAGCGCCACGCTTCCCGAAGGGAGAAAGGCG
GACAGGTATCCGGTAAGCGGCAGGGTCGGAACAGGAGAGCGCACGAGGGAGCTTCCA
GGGGGAAACGCCTGGTATCTTTATAGTCCTGTCGGGTTTCGCCACCTCTGACTTGAGCG
TCGATTTTTGTGATGCTCGTCAGGGGGGCGGAGCCTATGGAAAAACGCCAGCAACGCG
GCCTTTTTACGGTTCCTGGGCTTTTGCTGGCCTTTTGCTCACATGTTCTT

The term "GLP-1/GLP-1 peptide/GLP-1R agonist peptide" as used herein refers to GLP-1 molecules/peptides/proteins/variants/agonists herein are molecules having GLP-1R agonist function meaning that they are agonists of the GLP-1 receptor. This class of drugs is normally used for the treatment of diabetes, in particular type 2 diabetes. The amino acid sequence of mature "human GLP-1" is: HAEGTFTSDVSSYLEGQAAKEFIAWLVKGRG (SEQ ID NO: 21).

The term "GLP-1 analogue" as used herein refers to a peptide or a compound, which is a variant of GLP-1 (SEQ ID NO: 15). The terms "GLP-1 analog" and "analogue" may be used interchangeably herein.

GLP-1 analogues may be described by reference to i) the number of the amino acid residue in human GLP-1 (SEQ ID NO: 15) which corresponds to the amino acid residue which is modified (i.e. the corresponding position in GLP-1 (SEQ ID NO: 15)), and to ii) the actual modification.

The term GLP-1 Derivatives refer to derivatives of GLP-1 analogues. The term "derivative" as used herein in the context of a GLP-1 analogue means a chemically modified GLP-1 analogue in which one or more substituents have been covalently attached to the GLP-1 analogue. The term "substituent" as used herein, means a chemical moiety or group/side group conjugated to the GLP-1 protein/agonist/analogue. The derivative may comprise one or more modifications selected from amides, carbohydrates, alkyl groups, acyl groups, esters and the like.

In some embodiments the substituent is covalently attached via an amino acid residue in said polypeptide e.g. at one of the amino acid positions selected from the group consisting of position 22, 23, 27, 34, 35, and 36.

In some embodiments the GLP-1 derivative comprises a substituent comprising a lipophilic moiety. The term "lipophilic moiety" as used herein, means an aliphatic or cyclic hydrocarbon moiety with more than 6 and less than 30 carbon atoms, wherein said hydrocarbon moiety may comprise additional substituents.

Examples of GLP-1 agonists include (but are not limited to) exenatide, liraglutide, lixisentide, albiglutide, dulaglutide, taspoglutide, and semaglutide. DNA immuno-therapy vaccines using the plasmids herein may be combined initially with a parallel GLP-1R agonist treatment in treatment of e.g. recent onset T1D patients. GLP-1 co-administration may be chronic or temporary and include oral routes in addition to parenteral routes.

Liraglutide: (SEQ ID NO 22):

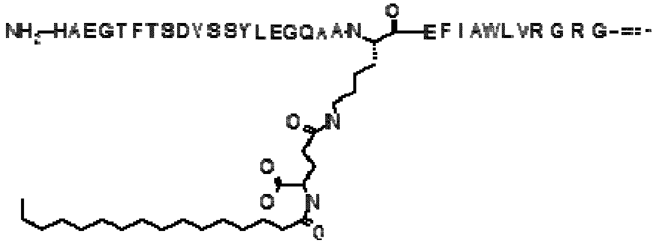

Semaglutide (SEQ ID NO 23):

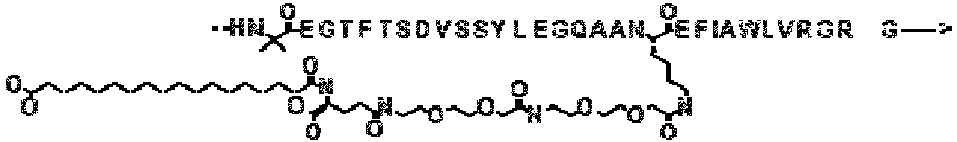

Pharmaceutical compositions herein are preferably aqueous formulations comprising at least 50% water, more preferably at least 60% water, more preferably at least 75% water, more preferably at least 90% water, more preferably at least 95% water, and most preferably at least 99% water. The pharmaceutical compositions herein may alternatively be dry formulations, such as lyophilized formulations, intended for reconstitution, inhalation, intranasal instillation, intradermal administration, etc.

Pharmaceutical formulations herein are preferably administered without the use of methods for enhancing transformation, such as electroporation. In one embodiment, pharmaceutical formulations are intended for parenteral administration, e.g. subcutaneous administration, intradermal administration, intravenous administration, intramuscular administration, etc. In another embodiment, pharmaceutical compositions herein may furthermore be administered topically, orally, rectally, or by inhalation.

Pharmaceutical compositions herein are preferably without addition of any condensation agents or other excipients that may induce local reactions. Formulations herein preferably contain free-radical scavengers (e.g. 1% ethanol) and/or chelators such as e.g. divalent cation scavengers (e.g. EDTA [CAS #60-00-4], EGTA [CAS #67-42-5], or DPTA [CAS #67-43-6]) in order to enhance stability of aqueous plasmid DNA. Pharmaceutical compositions herein may furthermore be in the form of a saline solution and/or a buffer solution or comprise a saline solution and/or comprise a buffer solution (e.g. PBS - phosphate buffered saline, TRIS buffer, or equivalent pharmaceutically acceptable buffers). Pharmaceutical formulations herein are preferably free from any adjuvants as well other typical vaccine ingredients such as e.g. aluminium hydroxide, phenol, sorbitol, silicone, etc.

Administration: The DNA immuno-therapy vaccine herein may be administered to a T1D patient, or a patient in risk of developing T1D. The vaccine may be administered e.g on a daily basis, every second day, twice a week, once a week, twice monthly, once a month, every second month, four times a year, or once a year - frequency may be adjusted according to general or individual needs. The immuno-therapy herein may be chronic. The duration of therapy may be e.g. one month, two months, three months, 6 months, one year, two years, three years, five years, six years, seven years, eight years, nine years, or 10 years.

### Embodiments

The following embodiments illustrate the invention and are not to be understood in any limiting way. It is understood that all embodiments can be combined in all possible ways.
1. A plasmid which encodes:
   i. an antigen;
   ii. TGF-β; and
   iii. IL-10.
2. The plasmid according to embodiment 1, which said antigen is an insulin antigen.
3. A plasmid which co-expresses/encodes (preferably from a single operon): (i) an antigen, such as e.g. an insulin antigen; (ii) TGF-β/TGF-β1 (such as in a constitutively active form); and (iii) IL-10.
4. The plasmid according to any of the preceding embodiments, wherein said insulin antigen is selected from the group consisting of: proinsulin, secretion-incapable pre-proinsulin, or a functional or immuno-dominant peptide fragment thereof.
5. The plasmid according to any of the preceding embodiments, wherein said insulin antigen is selected from the group consisting of: proinsulin, pre-proinsulin, and a functional or immuno-dominant peptide fragment thereof.
6. The plasmid according to any of the preceding embodiments, wherein said insulin antigen is endosomally targeted insulin.
7. The plasmid according to any of the preceding embodiments, wherein said plasmid expresses the insulin antigen and TGF-β in a ratio of about 1:1.
8. The plasmid according to any of the preceding embodiments, wherein said plasmid expresses insulin antigen and TGF-β in an amount of at least 200 fold lower than IL-10.
9. The plasmid according to any of the preceding embodiments, wherein said plasmid expresses insulin antigen and TGF-β in an amount of at least 2 fold lower than IL-10.
10. The plasmid according to any of the preceding embodiments, wherein said plasmid furthermore co-expresses Interleukin-2 (IL-2).
11. The plasmid according to any of the preceding embodiments, wherein said plasmid expresses an excess of IL-10 and IL-2 over the antigen (e.g. insulin) and TGF-β.
12. The plasmid according to any of the preceding embodiments, wherein said plasmid expresses IL-10 and IL-2 at least about one fold, two fold, five fold or at least about one hundred fold over TGF-β and insulin antigen (ratio of IL-10+IL-2 to insulin+TGF-β may be at least 1:1, or 2:1, or 5:1 or 100:1).
13. The plasmid according to any of the preceding embodiments, wherein said plasmid expresses IL-10 and IL-2 at least about one hundred fold, two hundred fold, five hundred fold or at least about one thousand fold over TGF-β and insulin antigen (ratio of IL-10+IL-2 to insulin+TGF-β may be at least 100:1, or 200:1, or 500:1 or 1000:1).
14. The plasmid according to any of the preceding embodiments, wherein said plasmid expresses IL-10 and IL-2 in a ratio of about 1:1 - 100:1, such as e.g. 1:1 - 50:1, such as e.g. 1:1 - 25:1, such as e.g. 1:1 - 10:1, alternatively 1:1 - 5:1, alternatively 1:1 - 3:1, alternatively 1:1 - 2:1. Alternatively, the ratio between expressed IL-10 and expressed IL-2 may be about 1:1, 1:0.9, 1:0.8, 1:0,7, 1:0.6, 1:0.5, 1:0.4; 1:0.3, 1:0.2, or 1:0.1.
15. The plasmid according to any of the preceding embodiments, wherein said plasmid comprises: (i) an FMDV 2A element separating the insulin antigen encoding sequence and the TGF-β encoding sequence, (ii) an EMCV IRES element separating the TGF-β encoding sequence and the IL-10 encoding sequence, and (iii) a 2A element separating the IL-10 encoding sequence and the IL-2 encoding sequence.
16. The plasmid according to any of the preceding embodiments, wherein said plasmid comprises:
   (i) a 2A element (such as an FMDV 2A or a P 2A element) separating the insulin antigen encoding sequence and the TGF-β encoding sequence,
   (ii) an EMCV IRES element (alternatively a bi-directional promoter) separating the TGF-β encoding sequence and the IL-10 encoding sequence (preferably, three alanine amino acids are encoded immediately N-terminal to the IL-10 gene), and
   (iii) a 2A element (such as a P 2A element) separating the IL-10 encoding sequence and the IL-2 encoding sequence.
17. The plasmid according to any of the preceding embodiments, wherein the TGF-β encoding sequence encodes constitutively active TGF-β, preferably constitutively active human TGF-β1.
18. The plasmid according to any of the preceding embodiments, wherein said plasmid comprises: (i) an endosomally targeted pre-pro-insulin encoding sequence, (ii) an FMDV 2A element, (iii) a TGF-β encoding sequence, (iv) an EMCV IRES element, (v) an IL-10 encoding sequence, (vi) a P 2A element, (vii) an IL-2 encoding sequence, (viii) a polyadenylation / termination element, (ix) a selection gene, (x) an origin of replication, (xi) a eukaryotic promoter element, (xii) a eukaryotic translational start sequence, (xiii) an endosomal sorting sequence, and (xiv) optionally an intron.
19. The plasmid according to any of the preceding embodiments, wherein said plasmid comprises the following elements:
   (i) a promoter (such as a CMV IE promoter),
   (ii) an intron (located within the noncoding leader sequence), and
   (iii) a eukaryotic translational start sequence (such as a Kozak element),
   (iv) an endosomally targeted antigen encoding sequence (such as an endosomally targeted human secretion-defective pre-pro-insulin encoding sequence),
   (v) an FMDV 2A element preferably separating the antigen encoding sequence and the TGF-β encoding sequence,
   (vi) a TGF-β encoding sequence (such as a constitutively active human TGF-β encoding sequence, preferably a constitutively active human TGF-β1 encoding sequence),
   (vii) an EMCV IRES element (or alternatively a bi-directional eukaryotic promoter), wherein said EMCV IRES element separates the TGF-β encoding sequence and the IL-10 encoding sequences,
   (viii) an IL-10 encoding sequence (such as a human IL-10 encoding sequence with a three alanine amino acid N-terminal addition),
   (ix) a 2A element, such as a P 2A element, wherein said 2A element separates the IL-10 encoding sequence and the IL-2 encoding sequence,
   (x) an IL-2 encoding sequence (such as a human IL-2 encoding sequence),
   (xi) a termination element (such as a bGH_PA termination element),
   (xii) a selection gene (such as a kanamycin encoding sequence or a *wt* infA encoding sequence),
   (xiii) an origin of replication (such as a prokaryotic origin of replication, such as e.g. pUC ori).
20. The plasmid according to embodiment 18, wherein the elements (i)-(xiii) are arranged by order of expression.
21. The plasmid according to any of the preceding embodiments, wherein the DNA sequence of the plasmid is as set forth SEQ ID NO 24, or essentially as set forth in SEQ ID NO 24.
22. The plasmid according to embodiment 21, wherein a few minor modifications, resulting in e.g. one, two, three, or four amino acid substitution in one or more of the antigen and/or the cytokines are made to SEQ ID NO 24 herein.
23. The plasmid according to any of embodiments 1-20, wherein the DNA sequence of the plasmid is as set forth in SEQ ID NO:26 or a modification of SEQ ID NO:26 resulting in e.g. one, two, three or four amino acid substitutions in one or more of the antigen and/or cytokines, or a modification of SEQ ID NO:26 which results in expression of the same polypeptide sequences as from SEQ ID NO:26.
24. The plasmid according to any of embodiments 1-20, wherein the DNA sequence of the plasmid is as set forth in SEQ ID NO:26 or a modification of SEQ ID NO:26 having less than 100 bases which are different than SEQ ID NO:26.
25. The plasmid according to any of embodiments 1-20, wherein the DNA sequence of the plasmid is as set forth in SEQ ID NO:28 or a modification of SEQ ID NO:28 resulting in e.g. one, two, three or four amino acid substitutions in one or more of the antigen and/or cytokines, or a modification of SEQ ID NO:28 which results in expression of the same polypeptide sequences as from SEQ ID NO:28.
26. The plasmid according to any of embodiments 1-20, wherein the DNA sequence of the plasmid is as set forth in SEQ ID NO:28 or a modification of SEQ ID NO:28 having less than 100 bases which are different than SEQ ID NO:28.
27. The plasmid according to any of embodiments 1-20, wherein the DNA sequence of the plasmid is as set forth in SEQ ID NO:29 or a modification of SEQ ID NO:29 resulting in e.g. one, two, three or four amino acid substitutions in one or more of the antigen and/or cytokines, or a modification of SEQ ID NO:29 which results in expression of the same polypeptide sequences as from SEQ ID NO:29.
28. The plasmid according to any of embodiments 1-20, wherein the DNA sequence of the plasmid is as set forth in SEQ ID NO:29 or a modification of SEQ ID NO:29 having less than 100 bases which are different than SEQ ID NO:29.
29. The plasmid according to any of embodiments 1-20, wherein said plasmid comprises a TGF-β gene comprising SEQ ID NO:25 or SEQ ID NO:25 having less than 10 base substitutions.
30. The plasmid according to any of the preceding embodiments for use in delaying or preventing type I diabetes.
31. The plasmid according to any of the preceding embodiments for intra-muscular, intradermal, intranasal, or subcutaneous administration.
32. The plasmid according to embodiment 31 for subcutaneous administration.
33. The plasmid according to embodiment 31 for intra-muscular injection.
34. The plasmid according to any of the preceding embodiments for use in treating a medical condition in a subject, such as e.g. type I diabetes, early-onset type I diabetes, or increased risk of developing type I diabetes (including type 1,5 diabetes type of conditions).
35. A DNA immuno-therapy vaccine comprising a plasmid according to any of the preceding embodiments.
36. The DNA immuno-therapy vaccine according to embodiment 35 for use in delaying or preventing type I diabetes.
37. The DNA immuno-therapy vaccine according to any of embodiments 35-36 for intra-muscular, intradermal, intranasal, or subcutaneous administration.
38. The DNA immuno-therapy vaccine according to embodiment 37 for subcutaneous administration.
39.The DNA immuno-therapy vaccine according to embodiment 37 for intra-muscular administration.
40. The DNA immuno-therapy vaccine according to any of embodiments 35-39 used in association with, or in parallel with other types of medical treatments such as e.g. beta cell/beta stem cell therapy, beta cell/beta stem cell grafting, etc. to prolong the survival and efficacy of engrafted cells.
41. A pharmaceutical composition comprising the DNA immuno-therapy vaccine according to any of embodiments 34-39, or a plasmid according to any of embodiments 1-34, wherein said pharmaceutical composition comprises a saline solution and/or a buffer and/or a chelator.
42. A pharmaceutical composition comprising the DNA immuno-therapy vaccine according to any of embodiments 35-40, or a plasmid according to any of embodiments 1-34, wherein said pharmaceutical composition comprises a saline solution and/or a buffer and/or a chelator and/or ethanol.
43. The pharmaceutical composition according to any of embodiments 41-42, wherein the volume/volume percentage of ethanol is less than 5%, less than 4%, less than 3%, less than 2%, or less than 1%.
44. The pharmaceutical composition according to any of embodiments 41-43, wherein said composition does not comprise any virus, lipid co-packing agent, or condensation agent.
45. The pharmaceutical composition according to any of embodiments 41-44, wherein said composition further comprises a GLP-1R agonist.
46. The pharmaceutical composition according to any of embodiments 41-44, wherein said composition furthermore comprises a GLP-1 analogue/GLP-1R agonist.
47. The pharmaceutical composition according to any of embodiments 45-46 wherein said GLP-1 analogue or said GLP-1R agonist is selected from liraglutide, semaglutide or a mixture thereof.
48. A kit comprising a pharmaceutical composition according to any of embodiments 41-47 and a pharmaceutical composition comprising a GLP-1 analogue/GLP-1R agonist (e.g. liraglutide and/or semaglutide).
49. A method of producing a plasmid according to any of embodiments 1-34, wherein said method comprises (i) incubating a host cell, such as a host cell of bacterial origin such as e.g. *E. coli*) transfected with said plasmid under suitable conditions and (ii) recovering/purifying said plasmid.
50. The method according to embodiment 49, wherein said host cell is a *E. coli* infA thermosensitive strain.
51. A method of delaying the onset of Type-1 diabetes (T1D) or symptoms thereof in a patient at risk of developing T1D, or recently diagnosed with T1D, said method comprising administering a DNA immuno-therapy vaccine comprising the plasmid according to any of embodiments 1-31, optionally in combination with a GLP-1 analogue/GLP-1R agonist.
52. A method of preserving beta cell function and/or endogenous insulin production in an individual, said method comprising administering a DNA immuno-therapy vaccine comprising the plasmid according to any of embodiments 1-34, optionally in combination with a GLP-1 analogue/GLP-1R agonist.
53. A method of treating a diabetic individual comprising administering a vaccine comprising the plasmid according to any of embodiments 1-34, optionally in combination with a GLP-1 analogue/GLP-1R agonist (e.g. liraglutide and/or semaglutide).
54. A vaccine for preventing or delaying the onset of Type-1 diabetes (T1D) symptoms in a patient at risk of developing, or recently diagnosed with, T1D said vaccine comprising the plasmid according to any of embodiments 1-34.
55. A method of reducing the dosage of insulin in an individual having Type-1 diabetes (T1D), or a person at risk of developing T1D, said method comprising administering a DNA immuno-therapy vaccine comprising a plasmid according to any of embodiments 1-33, optionally in combination with a GLP-1 analogue/GLP-1R agonist (e.g. liraglutide and/or semaglutide).

### EXAMPLES

Non Obese Diabetic mice (NOD mouse model of type 1 diabetes): Immune function in autoimmunity relies on a complex network of cellular interactions that cannot be adequately evaluated *in vitro.*

Disease suppression and/or treatment evaluations herein were carried out in the NOD mouse model, this model is a polygenic spontaneous onset model where most mice develop elevated blood glucose concentrations (BGV, blood glucose value, determined from tail-vein needlestick and handheld meter) between 12 and 30 weeks of age. Incidence and progression of disease is unpredictable, with total incidence ranging from 60% to 95% at 30 weeks of age (WoA) and progression from diagnosis (two sequential BGV readings of >250) to terminal (two sequential BGV readings of 600 or higher) ranging from 2 days to 4 weeks. Replication of elevated BGVs on sequential readings are necessary as mice are allowed food and water ad libitum which results in moderate BGV variability beyond that caused by immuno-pathology.

An example of a plasmid nucleotide sequence herein:
SEQ ID NO 24: full (non-annotated) plasmid sequence (6,401 base pairs)

GACTCTTCGCGATGTACGGGCCAGATATACGCGTTGACATTGATTATTGACTAG
TTATTAATAGTAATCAATTACGGGGTCATTAGTTCATAGCCCATATATGGAGTTCCGCGT
TACATAACTTACGGTAAATGGCCCGCCTGGCTGACCGCCCAACGACCCCCGCCCATTG
ACGTCAATAATGACGTATGTTCCCATAGTAACGCCAATAGGGACTTTCCATTGACGTCAA
TGGGTGGACTATTTACGGTAAACTGCCCACTTGGCAGTACATCAAGTGTATCATATGCC
AAGTACGCCCCCTATTGACGTCAATGACGGTAAATGGCCCGCCTGGCATTATGCCCAGT
ACATGACCTTATGGGACTTTCCTACTTGGCAGTACATCTACGTATTAGTCATCGCTATTA
CCATGGTGATGCGGTTTTGGCAGTACATCAATGGGCGTGGATAGCGGTTTGACTCACG
GGGATTTCCAAGTCTCCACCCCATTGACGTCAATGGGAGTTTGTTTTGGCACCAAAATC
AACGGGACTTTCCAAAATGTCGTAACAACTCCGCCCCATTGACGCAAATGGGCGGTAG
GCGTGTACGGTGGGAGGTCTATATAAGCAGAGCTCTCTGGCTAACTAGAGAACCCACT
GCTTACTGGCTTATCGAAATTAATACGACTCACTATAGGGAGACCCAAGCTGGCTAGCG
TTTAAACTTAAGCTTGGTACCGAGCTCGGATCCACTAGTCCAGTGTGGTGGAATTCTGC
ACTGCAGCTCGCATCTCTCCTTCACGCGCCCGCCGCCCTACCTGAGGCCGCCATCCAC
GCCGGTTGAGTCGCGTTCTGCCGCCTCCCGCCTGTGGTGCCTCCTGAACTGCGTCCGC
CGTCTAGGTAAGTTTAAAGCTCAGGTCGAGACCGGGCCTTTGTCCGGCGCTCCCTTGG
AGCCTACCTAGACTCAGCCGGCTCTCCACGCTTTGCCTGACCCTGCTTGCTCAACTCTA
GGTAAGTTAATGAGACAGATAGAAACTGGTCTTGTAGAAACAGAGTAGTCGCCTGCTTT
TCTGCCAGGTGCTGACTTCTCTCCCCTGGGCTTTTTCTTTTTCTCAGGTTGAAAAGAAG
AAGACGAAGAAGACGAAGAAGACAAACCGTCGTCGACTGCCATGCGCCGCTGATTAAC
GCCGCCACCATGGCCCACCGACGCAGATCCAGAAGCTGCCGTGAGGACCAGAAGCCC
GTGATGGATGATCAGAGGGACCTTATCTCTAACAATGAACAACTGCCAATGCTCGGCAG
ACGGCCTGGGGCCCCGGAGAGCAAGTGCAGCAGAGGAGCCTTGTACACGGGGTTCTC
CATTTTAGTGACTCTCCTTCTCGCCGGCCAAGCTACCACCGCCTACTTTCTGTACCAACA
GCAAGGCAGACTAGACAAACTGACAATCACAAGCCAGAACCTTCAGCTGGAGTCTCTGC
GGATGAAGCTGCCCGCTTTGTGGATGAGATTGCTTCCTCTACTTGCTCTCCTGGCGCTC
TGGGGACCTGACCCCGAGCAAGAGTTTGTTAATCAGCACCTGTGTGGGAGTCATCTGG
TGGAGGCACTCTATTTAGTGTGCGGAGAGAGGGGCTTCTTCTACACTCCAAAGACCAGA
CGGGAGGCCGAAGACCTTCAAGTGGGGCAAGTAGAACTGGGTGGCGGACCCGGTGCC
GGGAGCCTTCAGCCGCTCGCCCTGGAGGGCTCTCTTCAGAAACGCGGCATCGTGGAG
CAGTGTTGCACATCCATTTGCTCACTCTACCAGCTGGAGAACTACTGCAACGGAAGCGG
AGTGAAGCAGACGTTGAATTTTGATTTGTTGAAGTTGGCGGGGGATGTGGAGAGCAATC
CGGGGCCGATGCCCCCTAGTGGCCTCAGACTTTTGTTATTGTTATTACCGCTTTTATGG

CTCTTGGTGCTGACACCGGGCCGTCCGGCTGCTGGCTTGTCGACTTGTAAGACAATTG
ATATGGAATTGGTGAAACGAAAACGGATTGAGGCCATCCGAGGACAGATTTTGAGCAAG
CTGCGGCTTGCCTCGCCACCCTCGCAAGGGGAAGTCCCACCCGGACCTCTACCAGAAG
CAGTCCTAGCGCTGTACAACAGTACAAGAGATAGAGTGGCCGGGAATCCGCAGAACC
AGAGCCTGAGCCTGAAGCCGATTATTATGCAAAGGAAGTGACTAGGGTCCTGATGGTC
GAGACCCATAACGAAATCTACGACAAATTCAAACAAAGTACCCACTCTATCTACATGTTC
TTCAACACCAGTGAGCTAAGAGAAGCCGTGCCCGAACCTGTGCTTCTTTCCCGCGCAG
AACTCCGCCTCTTGAGACTCAAATTGAAAGTTGAACAACACGTAGAGCTTTACCAGAAAT
ACTCTAATAATTCATGGCGATATCTTTCTAATCGTCTCCTCGCCCCATCTGACAGCCCTG
AATGGCTCTCCTTCGACGTTACGGGAGTTGTGCGCCAGTGGCTCAGCAGAGGCGGAGA
GATAGAGGGCTTTCGGCTGAGCGCACATGTATCTGTGGACTCAAGGACAACACATTG
CAAGTGGATATTAACGGTTTTACAACTGGACGGAGAGGGGACCTGGCGACCATCCACG
GCATGAATAGACCTTTCCTGCTGCTGATGGCTACTCCCCTGGAGAGGGCACAGCACTTA
CAGTCTTCCAGACACCGGCGCGCCCTGGATACAAACTACTGCTTCAGCTCCACCGAAAA
GAACTGTTGCGTGCGGCAGCTGTACATTGACTTCAGAAAGGATCTGGGCTGGAAGTGG
ATTCATGAGCCCAAGGGGTATCATGCCAACTTCTGTCTTGGGCCATGCCCATACATCTG
GTCACTGGATACCCAGTACTCCAAAGTTCTGGCCTTGTACAATCAACACAACCCTGGAG
CTTCCGCCGCTCCTTGCTGTGTGCCCCAAGCCCTAGAGCCCCTGCCCATCGTTTATTAT
GTCGGACGCAAGCCCAAAGTAGAACAGCTATCAAATATGATCGTGAGAAGCTGCAAGTG
TAGCTGATAAACGCGTCGAGCATGCATCTAGGGCGGCCAATTCCGCCCCTCTCCCCCC
CACCCCTCTCCCTCCCCCCCCCCTAACGTTACTGGCCGAAGCCGCTTGGAATAAGGCC
GGTGTGCGTTTGTCTATATGTTATTTTCCACCATATTGCCGTCTTTTGGCAATGTGAGGG
CCCGGAAACCTGGCCCTGTCTTCTTGACGAGCATTCCTAGGGGTCTTTCCCCTCTCGCC
AAAGGAATGCAAGGTCTGTTGAATGTCGTGAAGGAAGCAGTTCCTCTGGAAGCTTCTTG
AAGACAAACAACGTCTGTAGCGACCCTTTGTAGACAGCGGAACCCCCCACCTGGCGAT
AGATGCCTCTGCGGCCAAAAGCCACGTGTATAAGATACACCTGCAAAGGCGGCACAAC
CCCAGTGCCACGTTGTGAGTTGGATAGTTGTGGAAAGAGTCAAATGGCTCTCCTCAAGC
GTATTCAACAAGGGGCTGAAGGATGCCCAGAAGGTACCCCATTGTATGGGATCTGATCT
GGGGCCTCGGTGCACATGCTTTACATGTGTTTAGTCGAGGTTAAAAAACGTCTAGGCCC
CCCGAACCACGGGGACGTGGTTTTCCTTTGAAAAACACGATGATAATATGGCTGCCGCT
CATTCTAGTGCCCTTCTTTGCTGCCTGGTCCTGCTCACCGGGGTGCGAGCTAGCCCTG
GACAAGGGACACAATCCGAAAACTCGTGCACCCACTTCCCGGGCAACCTCCCTAACAT
GCTGAGGGACCTCCGTGATGCCTTCAGTAGAGTGAAGACGTTCTTCCAAATGAAAGATC
AGTTAGATAACCTGCTCCTGAAGGAGTCACTCTTAGAAGACTTCAAAGGATACCTCGGC
TGCCAAGCACTTAGCGAGATGATTCAATTCTACTTAGAAGAAGTCATGCCTCAAGCTGA

GAATCAAGACCCCGACATCAAAGCTCATGTGAATTCTTTGGGAGAAAATTTGAAGACTTT
GCGGCTGCGGCTGCGGAGATGTCACCGCTTTCTGCCCTGTGAGAACAAATCAAAAGCG
GTCGAGCAAGTTAAGAATGCCTTCAATAAGCTACAAGAAGGGCATCTACAAAGCAAT
GAGCGAGTTTGATATCTTTATCAATTACATTGAAGCCTACATGACAATGAAGATTAGGAA
TGCCGCGGGGAGCGGCGCTACTAACTTCAGCCTGCTGAAGCAGGCTGGAGACGTGGA
GGAGAACCCTGGACCTATGTACAGAATGCAGCTGCTGAGCTGCATCGCCCTGAGCCTG
GCCCTGGTGACCAACAGCGCACCCACGTCCTCTAGCACCAAGAAGACCCAGTTACAGT
TGGAGCATCTACTTTTAGACCTGCAAATGATTTTGAACGGCATCAACAACTACAAGAATC
CTAAACTTACTCGCATGCTTACCTTCAAATTTTACATGCCCAAGAAGGCCACCGAACTGA
AGCACTTGCAATGTCTGGAGGAAGAACTCAAGCCGCTGGAGGAAGTTCTCAACCTCGC
GCAGTCCAAGAATTTCCACCTCCGGCCAAGAGACCTGATCAGTAACATTAATGTGATAG
TGCTGGAGCTGAAGGGAAGCGAGACTACATTTATGTGCGAGTACGCCGATGAAACCGC
TACAATCGTCGAGTTCCTGAATAGATGGATCACATTTTGCCAGTCAATTATCTCTACTCT
GACATGATAACTCGAGGTCTAGAGGGCCCGTTTAAACCCGCTGATCAGCCTCGACTGT
GCCTTCTAGTTGCCAGCCATCTGTTGTTTGCCCCTCCCCCGTGCCTTCCTTGACCCTGG
AAGGTGCCACTCCCACTGTCCTTTCCTAATAAAATGAGGAAATTGCATCGCATTGTCTGA
GTAGGTGTCATTCTATTCTGGGGGGTGGGGTGGGGCAGGACAGCAAGGGGGAGGATT
GGGAAGACAATAGCAGGCATGCTGGGGATGCGGTGGGCTCTATGGCTTCTACTGGGC
GGTTTTATGGACAGCAAGCGAACCGGAATTGCCAGCTGGGGCGCCCTCTGGTAAGGTT
GGGAAGCCCTGCAAAGTAAACTGGATGGCTTTCTCGCCGCCAAGGATCTGATGGCGCA
GGGGATCAAGCTCTGATCAAGAGACAGGATGAGGATCGTTTCGCATGGCCAAAGAAGA
CAATATTGAAATGCAAGGTACCGTTCTTGAAACGTTGCCTAATACCATGTTCCGCGTAGA
GTTAGAAAACGGTCACGTGGTTACTGCACACATCTCCGGTAAAATGCGCAAAAACTACA
TCCGCATCCTGACGGGCGACAAAGTGACTGTTGAACTGACCCCGTACGACCTGAGCAA
AGGCCGCATTGTCTTCCGTAGTCGCTGATAAATTATTAACGCTTACAATTTCCTGATGCG
GTATTTTCTCCTTACGCATCTGTGCGGTATTTCACACCGCATACAGGTGGCACTTTTCGG
GGAAATGTGCGCGGAACCCCTATTTGTTTATTTTTCTAAATACATTCAAATATGTATCCGC
TCATGAGACAATAACCCTGATAAATGCTTCAATAATAGCACGTGCTAAAACTTCATTTTTA
ATTTAAAAGGATCTAGGTGAAGATCCTTTTTGATAATCTCATGACCAAAATCCCTTAACGT
GAGTTTTCGTTCCACTGAGCGTCAGACCCCGTAGAAAAGATCAAAGGATCTTCTTGAGA
TCCTTTTTTTCTGCGCGTAATCTGCTGCTTGCAAACAAAAAAACCACCGCTACCAGCGGT
GGTTTGTTTGCCGGATCAAGAGCTACCAACTCTTTTTCCGAAGGTAACTGGCTTCAGCA
GAGCGCAGATACCAAATACTGTTCTTCTAGTGTAGCCGTAGTTAGGCCACCACTTCAAG
AACTCTGTAGCACCGCCTACATACCTCGCTCTGCTAATCCTGTTACCAGTGGCTGCTGC
CAGTGGCGATAAGTCGTGTCTTACCGGGTTGGACTCAAGACGATAGTTACCGGATAAG

GCGCAGCGGTCGGGCTGAACGGGGGGTTCGTGCACACAGCCCAGCTTGGAGCGAACG
ACCTACACCGAACTGAGATACCTACAGCGTGAGCTATGAGAAAGCGCCACGCTTCCCG
AAGGGAGAAAGGCGGACAGGTATCCGGTAAGCGGCAGGGTCGGAACAGGAGAGCGCA
CGAGGGAGCTTCCAGGGGGAAACGCCTGGTATCTTTATAGTCCTGTCGGGTTTCGCCA
CCTCTGACTTGAGCGTCGATTTTTGTGATGCTCGTCAGGGGGGCGGAGCCTATGGAAA
AACGCCAGCAACGCGGCCTTTTTACGGTTCCTGGGCTTTTGCTGGCCTTTTGCTCACAT
GTTCTT

### Example 1 - Antigen encoding plasmids compared to antigen + IL-10 encoding plasmids:

It has been suggested in the prior art that depletion of immuno-stimulatory CpG sequences in the plasmid back bone would be required for effective DNA immuno-therapy treatment of T1D. This experiment was thus modelled after previously published experiments (2008 J Immunol. 181(12):8298-307).

NOD mice were given eight once weekly doses of plasmid beginning at week 9 (age): either empty vector (pVAX1, 50 ug) was given, or pVAX1-proinsulin Ag (not endosomally targeted, not preproinsulin), or CpG depleted pVAX1-proinsulin Ag, or a bicistronic construct pVAX1-IL10-IRES-proinsulin Ag in equimolar ratios.

All administrations were intramuscular in the left quadriceps under isoflurane anaesthesia and contained only plasmid in PBS + EDTA. BGVs were assessed in all mice on a weekly basis and incidence of type 1 diabetes was scored based on two BGV readings over 250 mg/dl. Mice were evaluated until 30 weeks of age or a BGV of 600 were reached, followed by sacrifice.

The results from this experiment (table 1) demonstrate that A) CpG depletion is neither necessary nor beneficial for efficacy, B) the inclusion of immuno-modulatory cytokines significantly increases efficacy, and C) the plasmid backbone (empty vector) is equivalent to untreated groups.

**Table 1: T1D incidence in NOD mice at 30 weeks of age.**

| **Plasmid** | **T1 DDisease incidence at 30 weeks of age** |
|---|---|
| Historical untreated colony incidence | 77.8% |
| pVAX1 (empty vector negative control) | 23/29 = 79.3% |
| CpG depleted pVAX1-proinsulin Ag (antigen+modified vector) | 24/29 = 82.7% |
| pVAX1-proinsulin Ag (antigen) | 18/30 = 60% |
| pVAX1-IL10-IRES-proinsulin Ag (antigen+I L-10) | 10/26 = 38.5% |

### Example 2 - Expressed protein products resulting from plasmids encoding antigen, IL-10, IL-2 and TGF-β

Multi-cistronic plasmids were created to co-express TGF-β, IL-10, and optionally IL-2. Freestyle293 cells were transiently transfected and cultured in serum-free media. Supernatants were collected and subjected to ELISA quantification after 72 hours.

The results in table 2 below shows that: A) expression of multiple independent cytokines is achieved from a single vector, B) significant amounts of each cytokine are produced and in the expected ratios, C) minor sequence changes significantly improve IL-10 expression from the first generation IL10/proinsulin plasmid, and D) neither the plasmid backbone (empty vector) or endosomal targeting of antigen (IIAg) induces cytokine production or dysregulation.

**Table 2: ELISA quantification of expressed protein products.**

| **Plasmid** | **Active TGF-b1 (ng/ml)** | **Interleukin-10 (ng/ml)** | **Interleukin-2 (ng/ml)** |
|---|---|---|---|
| pVAX1 (empty vector) | <0.0035 | <0.0027 | <0.0009 |
| pVAX1-IL10/Proinsulin (antigen+IL-10) | <0.0035 | 85.3 | <0.0009 |
| pVAX1-IIAg/TGFβ/IL10/ (antigen+TGFβ+IL-10) | 7.35 | 1,238.8 | <0.0009 |
| pVAX1-IIAg/TGFβ/IL10/IL2 (antigen+TGFβ+IL-10+IL-2) | 2.39 | 1,259.5 | 777.0 |

### Example 3 - Impact of TGF-β and IL-2 on disease suppression

Multi-cistronic plasmids were evaluated for disease prevention in NOD mice as in Example 1, with the exception that dosing was continued once weekly until sacrifice (onset of diabetes) or week 30. One mouse from each group (initial n=24) was sent out for full necropsy after 10 weeks of dosing - including pathology on 10 standard highly perfused tissues, complete blood count, and clinical chemistry. Other than minor muscle disruption and regrowth due to mechanical trauma at the injection site, there were no deviations from un-dosed animals.

The results in table 3 below shows that: A) addition of TGFβ significantly increases efficacy, B) the inclusion of Interleukin-2 may increase efficacy and does not induce pathology, C) chronic dosing with plasmids expressing IL-10 and antigen increases efficacy in disease prevention, and D) chronic dosing with plasmids expressing TGFβ, IL-10 and IL-2 increases efficacy without resulting in any safety signals.

**Table 3: T1D incidence in NOD mice.**

| **Plasmid** | **Disease incidence at 30 weeks of age** |
|---|---|
| Historical untreated colony incidence | 77.8% |
| Untreated (negative control) | 18/21 = 85.7% |
| pVAX1-Ag/IL10 (antigen+IL-10) | 5/23 = 21.7% |
| pVAX1-IIAg/TGFβ/IL 10 (antigen+TGFβ+IL-10) | 2/23 = 8.7% |
| pVAX1-IIAg/TGFβ/IL10/IL2 (antigen+TGFβ+IL-10+IL-2) | 1/23 = 4.3% |

### Example 4 Evaluation of IRES elements, introns as well as subcutaneous administration

Multi-cistronic plasmids were evaluated for disease prevention in NOD mice as in Example 3, except that dosing began earlier (at week 5) in order to better mimic chronic pediatric administration. In addition to validating the pVAX1-IIAg/TGFβ/IL10 and pVAX1-IIAg/TGFβ/IL10/IL2 plasmids containing introns, other control groups were examined. Specifically, a different IRES segment (CrPV [from Cricket Paralysis Virus] as opposed to the EMCV [from EncephaloMyoCarditis Virus]) was evaluated for expected increases in efficacy, as was a deletion of the intron segment to assess its necessity. Due to obvious lack of efficacy compared to the parental plasmid (pVAX1-IIAg/TGFβ/IL10/IL2) the CrPV and intron-free (n.i. = no intron) groups were terminated early. In addition, the cohort of mice utilized in this experiment experienced more rapid progression of disease than previous cohorts, with time from diagnosis to sacrifice averaging 1.25 weeks rather than 2.75 from previous experiments. Finally, a subcutaneous administration group was added. This group was dosed with the triple cytokine plasmid (pVAX1-IIAg/TGFβ/IL10/IL2) with once weekly injection in the s.c. space in the scruff of the neck without anaesthesia.

The results in table 4 show that: A) EMCV IRES elements provide significantly better efficacy than the CrPV IRES, B) the inclusion of an intron (in this plasmid located within the CD74 endosomal targeting region) significantly increases efficacy, C) while the inclusion of IL-2 provides minimal benefit in mild disease settings its presence significantly increases the efficacy and robustness of treatment in aggressive disease settings, and D) subcutaneous dosing, which is ineffective in most DNA vaccine applications, here shows modest efficacy and a significant delay of disease even without optimization.

**Table 4: T1D incidence in NOD mice.**

| Treatment type | Diabetic/total | % diabetic |
|---|---|---|
| Historical control | | 80% @ 30 Weeks |
| Untreated | 15/21 | 71.4% @ 30 Weeks |
| Empty vector control i.m. | 13/21 | 61.9% @ 30 Weeks |
| pVAX1-IIAg/TGFβ/IL10/IL2 (no Intron) i.m. | 10/24 | 41.6% @ 22 Weeks |
| pVAX1-IIAg/TGFβ/IL10/IL2 (CrPv IRES instead of EMCV IRES) i.m. | 7/22 | 31.8% @ 22 Weeks |
| pVAX1-IIAg/TGFβ/IL10 i.m. (no IL-2) | 12/42 | 28.6% @ 30 Weeks |
| pVAX1-IIAg/TGFβ/IL10/IL2 i.m. | 1/42 | 2.4% @ 30 Weeks |
| pVAX1-IIAg/TGFβ/IL10/IL2 s.c. | 12/42 | 28.6% @ 30 Weeks |

### Example 5 Comparison of commercial antibiotic free selection with antibiotic selection systems

An alternate plasmid backbone was evaluated with the object of removing kanamycin resistance to comply with European Medicines Agency guidance. The same insert (IIAg/TGFβ/IL10/IL2, including intron) was cloned into the Nature Technology NTC9385R "nanoplasmid" backbone. The resultant plasmid was evaluated in NOD mice as in Example 3, except that treatment began on week 11 (late start) and terminated early due to failure of the NTC9385R-based plasmid.

The results in table 5 below show that: A) changes to selection system of the plasmid backbone surprisingly induce significant changes to effectiveness of the plasmids, and B) a late start to treatment results in early conversions. Data from other, related experiments indicates that dosing with these tolerogenic DNA vaccine plasmids requires two to four weeks to have efficacy, such that a late start to treatment results in several early cases of diabetes before the treatment becomes efficacious.

**Table 5: T1D disease incidence in NOD mice.**

| **Plasmid** | **Disease incidence at 30 weeks of age** |
|---|---|
| Historical untreated colony incidence | 77.8% |
| Untreated (negative control) | 16/21 = 76.2% |
| pVAX1-IIAg/TGFβ/IL10/IL2 with intron (kanamycin resistant) | 5/21 = 23.8% |
| pNTC9385R-IIAg/TGFβ/IL10/IL2 with intron (commercial antibiotic free selection system) | 13/21 = 61.9% |

### Example 6 Disease suppression efficacy with plasmids with and without antigen

To determine the role of the encoded antigen in the function of the plasmid two experiments were performed (Examples 6 and 7). An alternate plasmid was evaluated with the object of removing the antigen (pre-proinsulin) encoding region while retaining the CD74 targeting domain and all three secreted cytokines. The resultant plasmid was evaluated in NOD mice as in Example 3, except that treatment began on week 11 (late start).

This experiment demonstrates that the antigen portion is required for full efficacy and that it is not merely cytokine production driving the function of the plasmid. This is one of two criteria needed to demonstrate antigen-specificity of the treatment.

**Table 6: T1D incidence in NOD mice.**

| **Plasmid** | **Disease incidence at 30 weeks of age** |
|---|---|
| Historical untreated colony incidence | 77.8% |
| pVAX1-IIAg/TGFβ/IL10/IL2 (antigen+cytokines) | 2/22 = 9.1% |
| pVAX1-II/TGFβ/IL10/IL2 (no antigen+cytokines) | 15/28 = 53.5% |

### Example 7 Impact of the antigen immuno-therapy herein on efficiency of unrelated antigen vaccines

To determine the role of the encoded antigen in the function of the plasmid two experiments were performed (Examples 6 and 7). NOD mice were either sham treated with PBS injection or treated with pVAX1-IIAg/TGFβ/IL10/IL2 plasmid as in Example 3. Following four doses (i.e. at 13 weeks of age) each mouse was immunized i.p. with 50 µg of an irrelevant antigen (Chicken Ovalbumin, OVA) in 100 µl of a 1:1 alum suspension. Sham or plasmid treatments were continued once weekly until sacrifice three weeks (21 days) post-immunization at which time serum was collected. Class-switched (total IgG and IgG2a) antibodies against the ovalbumin antigen were determined via commercial ELISA kits. No significant differences were observed between plasmid and sham treated groups in their total anti-OVA IgG levels, nor did either group produce anti-OVA IgG2a.

The results in table 7 below show that while the plasmid suppresses immune responses related to the targeted disease, it does not suppress immune reactivity toward unrelated antigens (i.e. any antigens not encoded by the plasmid). This is the second of two criteria needed to demonstrate antigen-specificity of the treatment. As treatment of pediatric patients will involve concomitant administration of standard childhood vaccinations this is a significant advantage over systemic / generic immunosuppression via agents such as methotrexate or cyclosporine A.

**Table 7: Response to irrelevant antigen in NOD mice that have received DNA immuno-therapy vaccination against T1D.**

| Treatment | # samples | Mean ug anti-OVA IgG / mL serum | Error |
|---|---|---|---|
| Plasmid treated | 8 | 7.517 | +/- 0.967 |
| PBS (Sham) treated | 5 | 8.954 | +/- 1.227 |

These values result in a non-significant p value of 0.377 and a confidence interval of -1.99 to 4.87. These results indicate that treatment with the immunomodulatory plasmid does not impact immune response to other antigens not encoded by the plasmid, and therefore does not result in broad or systemic immuno suppression.

### Example 8 Individual protein products expressed from the plasmid

The TaV 2A element resulted in unexpected IL-10+IL-2 fusion products herein (data not shown) and other separation strategies were therefore evaluated. Initial separation technologies included upstream extensions of the TaV 2A sequence (leading to rapid degradation and lack of secreted IL-10) and also a carboxypeptidase cleavage site (which induced death of transfected cell lines). Further separation strategies evaluated were GSG-TaV 2A, a furin cleavage site, a furin site followed by TaV 2A, P 2A, and E 2A (equine rhinitis virus A).

Freestyle293 cells were transiently transfected and cultured in serum-free media. Both cell pellets and supernatants were collected and subjected to semi-quantitative multicolor Western blotting after 72 hours.

The results in table 8 below show that: A) unexpectedly, proteolytic cleavage sites fail to function between IL-10 and IL-2 genes, B) GSG tags (decoupler sequences) between IL-10 IL-2 are preferable to extended insulator sequences, C) P 2A is preferable to either TaV 2A or E 2A, and D) 2A sequences may have significant and unexpected effects on the degradation and secretion of expressed upstream proteins such as IL-10.

**Table 8: Separation of expressed IL-10 and IL-2 protein products.**

| **Plasmid** | **Cellular Interleukin -10** | **Secreted Interleukin -10** | **Cellular Interleukin-2** | **Secreted Interleukin-2** | **Cellular Fused product** | **Secreted Fused product** |
|---|---|---|---|---|---|---|
| GSG-TaV 2A | ++++ | ++ | - | +++ | ++ | - |
| Furin cleavage site | + | - | - | - | ++++ | ++ |
| Furin/TaV 2A | +++ | ++ | - | ++ | ++ | + |
| P 2A | ++ | ++++ | - | +++ | + | - |
| E 2A | +++ | ++ | - | ++ | ++ | - |

### Example 9 Comparison of commercial selection system with a heat sensitive selection system provided herein as well as comparison between plasmids encoding IL-2 and plasmids not encoding IL-2 (subcutaneous administration)

Plasmid backbones were created and evaluated with the object of removing kanamycin resistance to comply with European Medicines Agency guidance. The corrected insert (IIAg/GSG-FMDV 2A/TGFβ/EMCV IRES/IL10/GSG-P 2A/IL2, including an intron in the upstream noncoding region) was cloned into either a retrofitted/minimally modified pVAX1 vector containing the Nature Technology "RNA-OUT" selection marker or an equivalent minimally modified pVAX1 vector encoding *wt* infA ("pNN") as backbones. Additionally, plasmids either containing an additional SV40 enhancer element or defiecient in IL-2 were produced. The resultant plasmids were evaluated in NOD mice as in Example 3, except that administration was s.c. either once weekly or three times weekly (preferred).

The results shown in table 9+10 below show that: A) the commercially available exchange of RNA-OUT for the Kanamycin antibiotic resistance in the pVAX1 backbone still unexpectedly underperforms, B) the infA complementation antibiotic-free selection system performs equivalently to the parental pVAX1 vector, C) Interleukin-2 is required for optimal efficacy, D) addition of the SV40 enhancer element does not improve efficacy, and E) the corrected triple cytokine insert retains full functionality.

**Table 9: T1D incidence in NOD mice.**

| **Plasmid, administered 3x weekly (optimal)** | **Disease incidence at 30 weeks of age** |
|---|---|
| Historical untreated colony incidence | 78.9% |
| Untreated (negative control) | 12/15 = 80% |
| pNN empty vector (negative control with heat sensitive selection but no protein encoding sequences) | 12/16 = 75% |
| pVAX1-IIAg/FMDV/TGFβ/IL10/TaV2A/IL2 (kanamycin selection and protein encoding sequences) | 1/16 = 6.3% |
| pNN-IIAg/FMDV/TGFβ/IL10/P2A/IL2 (temperature selective system and protein encoding sequences) | 1/23 = 4.3% |
| pVAX1-RNA-OUT-IIAg/FMDV/TGFβ/IL10/P2A/IL2 (commercial selective system and protein encoding sequences) | 9/23 = 39.1% |

**Table 10: T1D incidence in NOD mice.**

| **Plasmid, administered 1x weekly (sub-optimal)** | **Disease incidence at 27 weeks of age** |
|---|---|
| Historical untreated colony incidence | 78.9% |
| Untreated (negative control) | 12/15 = 80% |
| pNN-IIAg/FMDV/TGFβ/IL10/P2A/IL2 (temperature selective system and protein encoding sequences) | 16/37 = 43.2% |
| pNN-SV40e-IIAg/FMDV/TGFβ/IL10/P2A/IL2 (temperature selective system and protein encoding sequences as well as an enhancer) | 20/37 = 54% |
| pNN-IIAg/FMDV/TGFβ/IL10 (IL-2 deficient) (temperature selective system and protein encoding sequences - except IL-2) | 25/40 = 62.5% |
| pVAX1-RNA-OUT-IIAg/FMDV/TGFβ/IL10/P2A/IL2 (commercial selective system and protein encoding sequences) | 27/38 = 71% |

### Example 10 Examination of durability of tolerance effect following plasmid withdrawal

In the previous experiment (represented in Table 9), the pNN-IIAg/FMDV/TGFβ/IL10/P2A/IL2 group was not sacrificed at 30 weeks of age but ceased dosing with plasmid. Blood glucose values were followed for an additional ten (10) weeks, to a total of 40 weeks of age, to assess whether the plasmid had induced a durable state of tolerance or whether continued dosing was necessary for efficacy.

The results shown in table 11 below indicate that continued dosing is required for durability of tolerance, as a stable disease-free state to 30 weeks of age rapidly deteriorated following discontinuation of dosing. This indicates a beneficial safety profile, as any adverse events that might be encountered with plasmid dosing would also be expected to cease with dosing.

**Table 11: T1D incidence in NOD mice following cessation of plasmid dosing.**

| **Disease incidence to 30 weeks of age** | **Disease incidence to 40 weeks of age** |
|---|---|
| 1/23 = 4.3% | 9/23 = 39.1% |

### Example 11 Examination of plasmid stability and durability on injection

A key issue with plasmid administration is degradation on administration. In the case of injection, shear forces encountered by large and viscous plasmid molecules passing through a thin needle under pressure lead to breakage of the covalently closed circular structure of the plasmid - rendering it linear and both subject to reduced transfection ability and rapid destruction. Most plasmids have 5 to 15% degradation to linear forms on injection through needles of sizes acceptable for clinical use, which leads to either reduced efficacy or a need for larger initial doses to compensate for the loss. Several types of sequence structures which may lead to plasmid unwinding and susceptibility to shear degradation were intentionally minimized in the plasmids disclosed, with the intention of increasing robustness and reliability with injection protocols. In order to assess shear degradation of plasmid, which can vary with viscosity and therefore concentration, the human lead plasmid was resuspended in Tris EDTA buffer to concentrations of 5, 7, and 9 mg/ml and passed three times though a G30 needle (expelled, re-drawn into syringe, then re-expelled) and one (1) microgram samples were run on an agarose gel against reference samples which were not passaged through the injection process.

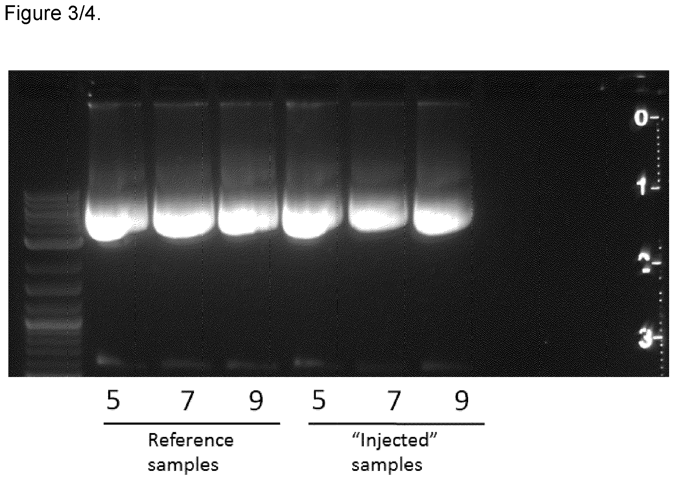

The results shown in Figure 3 indicate, surprisingly, that the plasmid is not noticeably degraded by three injection passages at any tested concentration or viscosity. Plasmid degradation would be visualized as both a smearing of smaller bands (in between the main supercoiled band at 6 Kb and the small process impurity band at the bottom of the gel or roughly 600 bp). Such linearization / degradation smears are not seen for any sample passaged through the injection process. This robust physical stability on dosing is highly desirable and both greater than anticipated or previously reported in literature.

### Example 12 Verification of plasmid retention with infA complementation system

In order to verify that the infA-based plasmid retention selection system functioned as desired, the plasmid transformed bacteria were grown through 100 passages (roughly 36 doublings/generations per passage, for a total of 3,600 generations of potential drift or plasmid loss examined). Passages 1-100 were generated at 11 per week, 2 passages per weekday at 37°C and one each weekend at 30°C. All were performed in liquid animal-free LB media (Teknova soy-tone) supplemented with 15 micrograms/ml naladixic acid (selecting for DH5α base strain, not for plasmid presence). Glycerol stocks were generated from each passage and retained until all 100 passages were obtained for concurrent processing.

Scrapes of the glycerol stocks were used to inoculate 5 ml overnight cultures which were processed via supplier instructions on Qiagen miniprep kits using a vacuum manifold (either 16 or 32 cultures per run, due to gel size constraints). No attempt was made to collect OD600 readings for cell input normalization and all preps were done based on standard volumes. One microliter of each miniprep was subjected to Pstl/Xhol digestion to resolve backbone (approximately 2.4 Kbp) from insert (approximately 4 Kbp), without correction for plasmid concentration resulting from each miniprep. Each gel was run with flanking Tridye 2-Log ladders (NEB https://www.neb.com/products/n3200-2-log-dna-ladder-01-100-kb), a first sample lane of undigested plasmid, and visualized with SybrSafe dye. In the images of the gels, despite lack of control for nucleic acid quantity, all digest lanes show both the presence and expected digestion pattern for plasmid (seen on the images for passages 1-16, 17-48, 49-80, and 81-100).

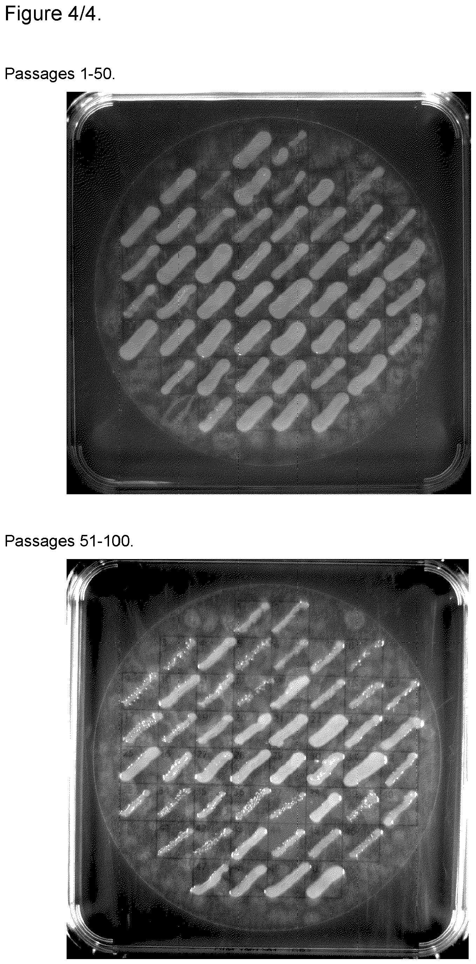

As an additional confirmation, glycerol stocks for passages 1-100 were also streaked onto 50 sector antibiotic-free and animal-free LB agar plates and incubated overnight at 30°C. No attempt to control for streak inoculum was made. As shown in Figure 4, all glycerol stock representative streaks resulted in noticeable growth and thus plasmid retention.

### Example 13 Suitability for scale up with infA complementation system

In order to verify that the infA-based plasmid retention selection system functioned as desired at production scale, the plasmid transformed bacteria were used in a 50 L pilot fed-batch fermentor run with a specific yield enhancing temperature shift step. Minimal medium with the addition of yeast extract was utilized, reducing the doubling rate to 0.88 / hour. The fed-batch was initiated at 17h00 post inoculation and the regulation of dissolved oxygen at 30% was realized by successive increase of the pO2 cascade parameters (stirring at 32h15 , pressure at 40h30, then air flow at 45h40). The biomass increase rate lessened immediately following the shift to 42°C, as anticipated. The amount of plasmid DNA produced was estimated at 1.03 ± 0.17 g/L using a small scale plasmid extraction procedure mimicking immediate post-lysis yield.

## Claims

1. A plasmid which encodes:
i. an insulin antigen;
ii. TGF-β; and
iii. IL-10.

2. The plasmid according to claim 1, wherein said insulin antigen is selected from the group consisting of: proinsulin, pre-proinsulin, and a functional or immuno-dominant peptide fragment thereof.

3. The plasmid according to any one of the preceding claims, wherein said insulin antigen is endosomally targeted insulin.

4. The plasmid according to any one of the preceding claims, wherein said plasmid expresses insulin antigen and TGF-β in an amount of at least 2 fold lower than IL-10.

5. The plasmid according to any one of the preceding claims, wherein said plasmid furthermore co-expresses Interleukin-2 (IL-2).

6. The plasmid according to any one of the preceding claims, wherein said plasmid comprises: (i) an FMDV 2A element separating the insulin antigen encoding sequence and the TGF-β encoding sequence, (ii) an EMCV IRES element separating the TGF-β encoding sequence and the IL-10 encoding sequence, and (iii) a 2A element separating the IL-10 encoding sequence and the IL-2 encoding sequence.

7. The plasmid according to any one of the preceding claims, wherein the TGF-β encoding sequence encodes constitutively active TGF-β.

8. The plasmid according to any one of the preceding claims, wherein said plasmid comprises: (i) an endosomally targeted pre-pro-insulin encoding sequence, (ii) an FMDV 2A element, (iii) a TGF-β encoding sequence, (iv) an EMCV IRES element, (v) an IL-10 encoding sequence, (vi) a P 2A element, (vii) an IL-2 encoding sequence, (viii) a polyadenylation / termination element, (ix) a selection gene, (x) an origin of replication, (xi) a eukaryotic promoter element, (xii) a eukaryotic translational start sequence, (xiii) an endosomal sorting sequence, and (xiv) optionally an intron.

9. A DNA immuno-therapy vaccine comprising a plasmid according to any one of the preceding claims.

10. The DNA immuno-therapy vaccine according to claim 9, or a plasmid according to any one of claims 1-8, for use in delaying or preventing type I diabetes.

11. The DNA immuno-therapy vaccine according to claim 9, or a plasmid according to any one of claims 1-8, for subcutaneous administration.

12. The DNA immuno-therapy vaccine according to claim 9, or a plasmid according to any one of claims 1-8, for intra-muscular administration.

13. A pharmaceutical composition comprising the DNA immuno-therapy vaccine according to claim 9, or a plasmid according to any one of claims 1-8, wherein said pharmaceutical composition comprises a saline solution and/or a buffer and/or a chelator.

14. The pharmaceutical composition according to claim 13, wherein said buffer does not comprise any virus, lipid co-packing agent, or condensation agent.

15. The pharmaceutical composition according to any one of claims 13-14, wherein said composition furthermore comprises a GLP-1R agonist.
